(19) Europäisches Patentamt
European Patent Office
Office européen des brevets

(11) EP 3 590 885 A1

(12) **EUROPEAN PATENT APPLICATION**

(43) Date of publication:
**08.01.2020 Bulletin 2020/02**

(21) Application number: **18181821.2**

(22) Date of filing: **05.07.2018**

(51) Int Cl.:
***B82Y 5/00*** *(2011.01)* ***A61K 47/69*** *(2017.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Karlsruher Institut für Technologie**
**76131 Karlsruhe (DE)**

(72) Inventors:
- **Niemeyer, Christof**
  **28213 Bremen (DE)**
- **Grösche, Maximilian**
  **79219 Staufen (DE)**
- **Rabe, Kersten**
  **76133 Karlsruhe (DE)**
- **Leidner, Arnold**
  **67466 Lambrecht (DE)**
- **Hu, Yong**
  **76344 Eggenstein-Leopoldshafen (DE)**
- **Bauer, Jens**
  **76889 Vorderweidenthal (DE)**

(74) Representative: **Müller-Boré & Partner**
**Patentanwälte PartG mbB**
**Friedenheimer Brücke 21**
**80639 München (DE)**

(54) **COMPOSITE MATERIAL COMPRISING DNA HYDROGEL AND SILICA NANOPARTICLES**

(57) The present invention relates to a composite material comprising DNA hydrogel and silica nanoparticles, a hollow spherical container, a microreactor and microgel employing the composite material and uses thereof.

**Figure 1**

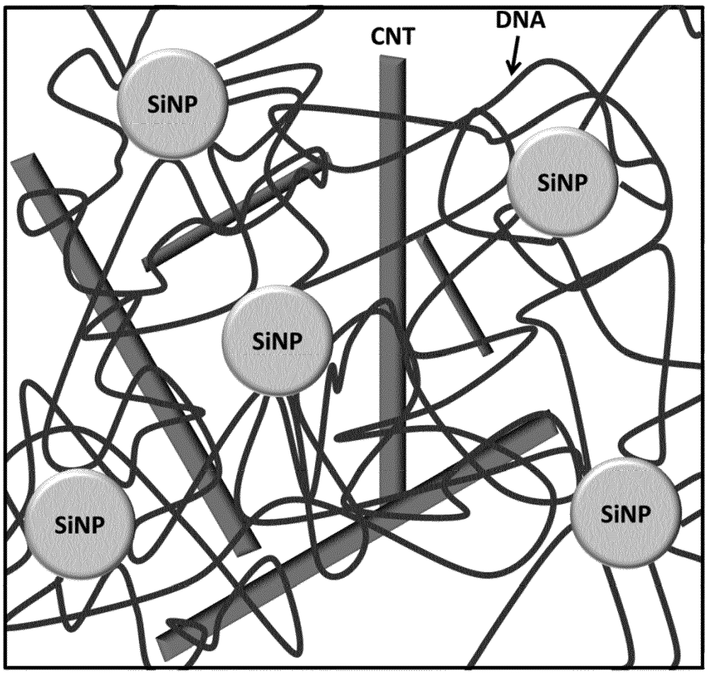


EP 3 590 885 A1

## Description

The present invention relates to a composite material comprising DNA hydrogel and silica nanoparticles, a hollow spherical container, a microreactor and microgel employing the composite material and uses thereof.

The design of novel materials to steer the interaction of eukaryotic cells with technical surfaces is of paramount interest for biomedical applications, such as cancer and stem cell therapies, tissue engineering or drug delivery. Synthetic hydrogels hold great promise as scaffolding materials in biosciences because their 3D porous structure reveals biocompatibility, high water content, and tissue-like elastic properties that allow for effective permeation of oxygen and nutrients, which is crucial for cellular colonization. Hydrogels with defined bulk and surface characteristics can be manufactured with controllable composition from various prepolymers and they are amenable to post synthetic modifications with molecular and colloidal compounds. DNA hydrogels are currently attracting much attention because the inherent properties of the genetic material allow for rational programming of structural and mechanical properties as well as of molecular recognition capabilities of the polymer backbone. This approach has led to an impressive collection of novel biocompatible materials that can display shape memory persistence, stimuli responsiveness and, by integration of genetic elements or proteins, even biochemical activity. However, there is a great demand for novel strategies to create DNA polymer materials with designed properties, such as adhesiveness, stiffness, plasticity, or tensile strength.

Thus, the problem underlying the present invention is to provide a DNA polymer material with designed properties, such as adhesiveness, stiffness, plasticity, or tensile strength.

The solution to the above technical problem is achieved by the embodiments characterized in the claims.

(1) Composite material

The present invention relates to a composite material comprising DNA hydrogel, and silica nanoparticles, wherein the silica nanoparticles are connected with the DNA hydrogel through DNA strands. By incorporating silica nanoparticles into the DNA hydrogel, the mechanical stiffness and elastic properties can be improved.

In an embodiment of the present invention, the content of the silica nanoparticles based on the total amount of the composite material is from 10 to 80 wt%. Preferably, the lower limit of the content of the silica nanoparticles is more than 50 wt%.

According to a further embodiment, the content of the DNA hydrogel based on the total amount of the composite material is from 20 to 90 wt%.

By adjusting the contents of the silica nanoparticles and the DNA hydrogel to the above ranges, the composite material is mechanically stable while having a porous structure such that the composite material can be used for building three-dimensional structures which allow the diffusion of chemical and/or biological substances through the walls of said structures.

In a preferred embodiment, the pore size in the structure is more than 100 nm and less than 700 nm. The preferred pore size can be measured by using two kinds of tracer particles (200 nm tracer particles and 500 nm tracer particles) in multiple particle tracking (MPT) microrheology (see below). Specifically, when the mesh size is estimated to be bigger than 100 nm and smaller than 500 nm, the 200 nm tracer particles freely move inside the hydrogel mesh structure, while 500 nm tracer particles are restrained inside the hydrogel mesh structure.

According to an embodiment, the composite material further comprises carbon nanotubes embedded in the DNA hydrogel. The carbon nanotubes can add an improved mechanical stiffness to the composite material such that a structure being more dimensionally stable can be achieved. The improvement in elastic modulus (storage modulus $G_0$, which is proportional to the number of entanglements in a polymer) by using both silica nanoparticles and carbon nanotubes in the DNA hydrogel is higher than expected from totalling the $G_0$ values of the binary composites silica nanoparticles-DNA hydrogel and carbon nanotubes-DNA hydrogel. Therefore, the ternary composite silica nanoparticles/carbon nanotubes-DNA hydrogel shows a synergistic effect in the mechanical properties, such as elasticity and stiffness.

The carbon nanotubes (CNT) can be mixed with the silica nanoparticles (SiNP) in different weight ratios in the ternary composite material. Preferably, the mass ratio of SiNP:CNT in the ternary composite material ranges from 100:1 to 1:1, preferably from 50:1 to 2.5:1. In an embodiment, the maximum concentration of SiNP in the binary SiNP-DNA hydrogel composite material and in the ternary SiNP/CNT-DNA hydrogel composite material is 20 mg/mL. According to a further embodiment, the maximum concentration of CNT in the ternary SiNP/CNT-DNA hydrogel composite material is 1000 μg/mL.

In an embodiment, the content of the carbon nanotubes based on the total amount of the composite material ranges from 1 to 10 wt%.

The composite material according to the present invention does not only provide mechanical stability while allowing diffusion of chemical and/or biological substances, but does also not show cytotoxicity such that it can be advantageously used for investigations of cells. These properties allow the use of the composite material in various fields of chemical, biological and biomedical applications. For example, the composite material according to the present invention may be advantageously used in cell biology, such as surface coatings for cell culture.

Figure 1 shows an example of the composite material of the present invention comprising DNA hydrogel, silica nanoparticles (SiNP) and carbon nanotubes (CNT).

The expression "embedded in the DNA hydrogel" used herein means that the respective embedded component is fixed in the surrounding DNA hydrogel.

In particular, in the composite material comprising DNA hydrogel, and silica nanoparticles, the DNA strand can be bonded to silica nanoparticles by at least one of its ends. Further, a DNA strand may also be bonded to a silica nanoparticle at other points along its strand. The DNA hydrogel is attached to the silica nanoparticles *via* a covalent bond.

In addition, in the embodiment further comprising carbon nanotubes, the DNA hydrogel contacts a carbon nanotube at least at one position along the outer wall of the carbon nanotube. The carbon nanotube and the DNA hydrogel are initially bound by hydrophobic $\pi$-$\pi$ interaction. In the composite material, the CNT are interwoven with the DNA strands such that they are restrained inside the material.

In an embodiment, the composite material can further comprise at least one of the components selected from gold nanoparticles, metal oxide nanoparticles, such as quantum dots, agarose, alginate, polyacrylamide, polyethylene glycol, proteins (e.g. membrane proteins), peptides, graphene, DNA-protein conjugates, DNA-ligand conjugates and MOSAIC systems (i.e. nanoscaled DNA systems with proteins presented in defined spatial arrangements and stoichiometry).

According to an embodiment of the present invention, individual SiNP/CNT-DNA hydrogel composite materials may be encoded with a distinctive DNA sequence that is introduced to serve as identifier code which allows decoding or deconvolution to identify the material and the substances or compounds being located inside the material after performing cell adhesion or cellular uptake experiments.

The method for producing the composite material according to the present invention is not particularly limited. For example, rolling circle amplification (RCA) or hybridization chain reaction (HCR) may be used. An embodiment of the manufacture of the composite material by using RCA is shown in Figure 2.

Further, the present invention relates to the use of the composite material for cargo delivery to a cell. Cargo delivery includes delivery to the outer membrane of the cell, to the transmembrane compartment of the cell, and the cytosolic compartment of the cell. The cargo to be delivered is not particularly limited and can be selected from pharmacologically active compounds, drugs, metals, metal nanoparticles, metal chelates, proteins, peptides, and DNA molecules that encode genes for RNA and protein production. In an embodiment of the present invention, the composite material is used for drug delivery to a cell. Specifically, the DNA backbone may have an aptamer which specifically binds to a receptor of a cell and, in addition, a drug association site, which can be used for enrichment of intercalating drugs. When incubating a cell with the composite material and the drug which binds to the drug association site, the cell will uptake the composite material bearing the drug at the drug association site. This drug delivery to a cell may be tracked for example by using fluorescent composite material by employing e.g. silica nanoparticles having a fluorescence marker and/or chemically modified deoxynucleotides bearing fluorescent dyes, such as fluorescein (FITC)-modified dUTP.

The present invention further relates to the use of the composite material as surface coating for *in vitro* cell culture. According to an embodiment, the composite material is dried. In another embodiment, the dried composite material has a film form having a thickness of from 0.01 $\mu$m to 10 m. In a preferred embodiment, the film is a thin film having a thickness of from 0.1 $\mu$m to 1000 $\mu$m. In a more preferred embodiment, the film is a thin film having a thickness of from 1 $\mu$m to 100 $\mu$m. The drying process may be carried out for 2 to 6 h under vacuum at room temperature. Cells can be seeded on top of the composite material and cultured for 10 to 30 h. The composite material enables proliferation of cells higher than standard tissue culture (STC) surfaces. This effect can be emphasized by using the composite material comprising DNA hydrogel, silica nanoparticles and carbon nanotubes. The composite material holds the potential as tailorable coating for steering of cell adhesion, proliferation and motility, which is of substantial relevance for biomedical applications, such as medical implants.

Further, the present invention relates to the use of the composite material for enzyme-triggered cell release. In an embodiment, the composite material has a film form having a thickness of from 0.01 $\mu$m to 10 m. In a preferred embodiment, the film is a thin film having a thickness of from 0.1 $\mu$m to 1000 $\mu$m. In a more preferred embodiment, the film is a thin film having a thickness of from 1 $\mu$m to 100 $\mu$m. For the use of the composite material for enzyme-triggered cell release, the composite material can be used directly after synthesis. Specifically, no drying step before application is required. Cells seeded on top of the composite material strongly adhere to the surface thereof. In particular, the cells are entrapped by said composite material in this embodiment. Treatment of the entrapped cells with a restriction enzyme according to an embodiment leads to a reduction of the thickness of the composite material along with the release of the adhered cells. Thus, the composite material allows the selective capture and enzyme-triggered release of surface-bound cells. This effect can be emphasized by using the composite material comprising DNA hydrogel, silica nanoparticles and carbon nanotubes.

According to another embodiment, the composite material of the present invention can be used for cell adhesion and transmigration of cells into the hydrogel layers, thereby the cells being protected from potentially harmful components in the cell medium.

The present invention is not limited to a particular type of cell. The cell used in the present invention is preferably a cell derived from a cell culture or a cell derived from a sample of an individual. The cell culture preferably comprises at least one type of eukaryotic cells or microorganisms. Non-limiting examples of cells are prokaryotic cells including gram-negative or gram-positive bacteria (e.g. *E. coli, Burkholderia spec., Bacillus spec.*) and Archaea as well as eukaryotic cells including yeast cells (such as *Saccharomyces,* e.g. *Saccharomyces cerevisiae*), insect cells (e.g. *Drosophila melanogaster*), plant cells and mammalian cells (e.g. HEK 293, HeLa). Furthermore isolates from natural or technical biofilm communities (e.g. from waste-treatment facilities, biogas producing plants, sewage plants, extreme habitats, sites of chemical spillage) or mixtures of different origin including combinations of a non-limited number of species are suitable for the present invention.

The term "individual" as used herein is not limited to any particular individual and may be a fungus, an animal, a human being or a plant.

The sample taken from an individual may be any sample. In one embodiment of the present invention, the sample may be derived from a naturally occurring system, preferably a sample containing a body fluid or components derived from a body fluid. In one embodiment of the present invention, the sample may be a naturally occurring system such as a solution selected from the group consisting of serum, saliva, urine, bile, lymph, tissue, like e.g. bladder or kidney, cerebrospinal fluid and/or other body fluids in the case of an animal or human individual.

In another embodiment, the sample may be derived from a naturally occurring system, preferably a sample containing a tissue or components derived from a tissue. A tissue or a component derived therefrom may be of various origins, like for example leafs, roots, petals, germs or stems in the case of plant individual, or muscle tissue, heart tissue, liver tissue, kidney tissue, epithelial tissue, connective tissue, or nervous tissue.

The term "cell culture" in its various grammatical forms, refers to cells grown in suspension, roller bottles, flasks and the like. Large scale approaches, such as bioreactors, including adherent cells growing attached to microcarriers in stirred fermenters, also are included. According to an embodiment of the present invention, cells derived from a cell culture may be derived from animals, plants or fungus. Animal cells may be derived for example from insects, fish, birds, or mammals. In a preferred embodiment, cells derived form a cell culture are mammalian cells including those of human origin, which may be primary cells derived from a tissue sample, diploid cell strains, transformed cells or established cell lines. Mammalian cells can include human and non-human cells alike. Mammalian cells of non-human origin can be monkey kidney cells, bovine kidney cells, dog kidney cells, pig kidney cells, rabbit kidney cells, mouse kidney cells, rat kidney cells, sheep kidney cells, hamster kidney cells, Chinese hamster ovarian cells or an animal cell derived from any tissue. In particular, mammalian cells can be BSC-1 cells, LLC-MK cells, CV-1 cells, COS-cells, COS-1 cells, COS-3 cells, COS-7 cells, VERO cells, MDBK cells, MDCK cells, CRFK cells, RAF cells, RK-cells, TCMK-1 cells, LLC-PK cells, PK15 cells, LLC-RK cells, MDOK cells, BHK-21 cells, CHO cells, NS-1 cells MRC-5 cells, WI-38 cells, BHK cells, 293 cells, HeLa, MCF7 and RK-cells.

The sample may be derived from a mammal, preferably a mammal selected from the group consisting of human, mouse, rat, pig, cat, dog, horse, goat, cattle, cow, and monkey and/or others. In a preferred embodiment of the present invention, the sample is derived from a human. In another embodiment of the present invention, the sample contains isolated body fluid compounds or processed body fluids derived from a mammal, preferably a mammal selected from the group consisting of human, mouse, rat, pig, cat, dog, horse, goat, cattle, cow, and monkey and/or others. In a preferred embodiment of the present invention, the sample contains isolated body fluid compounds or processed body fluids derived from a human.

In a preferred embodiment, the sample is pre-purified. In a more preferred embodiment of the present invention, the pre-purification comprises the step of removing impurities that impair significantly or prevent the growth of the cell.

In the following, the DNA hydrogel, the silica nanoparticles and the carbon nanotubes will be described.

(a) DNA hydrogel

A DNA hydrogel is a three-dimensional network of DNA strands held together by association bonds. The DNA hydrogel may contain aqueous components which are added during the gelling process of the DNA strands or added after the DNA hydrogel has been synthesized. DNA hydrogel is able to absorb a large quantity of water. Further, DNA hydrogel may be used as a scaffold material for forming three-dimensional structures having excellent elasticity.

Several methods for synthesizing DNA hydrogel are known in the art.

For example, Liu and co-workers reported the preparation of a fast forming, pH-responsive DNA hydrogel with three-armed DNA nanostructures (Y units) assembled together through the formation of intermolecular i-motif structures (Cheng, E.; Xing, Y.; Chen, P.; Yang, Y.; Sun, Y.; Zhou, D.; Xu, L.; Fan, Q.; Liu, D. A pH-triggered, fast-responding DNA hydrogel. Angew. Chem., Int. Ed. Engl. 2009, 121 (41), 7796-7799). They also reported that DNA hydrogels can be prepared by crosslinking DNA building blocks by sequence-directed hybridization (Xing, Y.; Cheng, E.; Yang, Y.; Chen, P.; Zhang, T.; Sun, Y.; Yang, Z.; Liu, D. Self-assembled DNA hydrogels with designable thermal and enzymatic responsiveness. Adv. Mater. 2011, 23 (9), 1117-1121). Briefly, the Y-scaffold containing three sticky ends and complementary linker DNA (double-stranded DNA, dsDNA) with the two sticky ends were designed. Accumulative hybridization between those two blocks would lead to hydrogel formation.

Nöll et al. reported that three-dimensional DNA hydrogel was generated by self-assembly of short linear double-stranded DNA (dsDNA) building blocks equipped with sticky ends (Nöll, T.; Schönherr, H.; Wesner, D.; Schopferer, M.; Paululat, T.; Nöll, G. Construction of Three-Dimensional DNA Hydrogels from Linear Building Blocks. Angew. Chem., Int. Ed. Engl. 2014, 53 (32), 8328-8332). The resulting DNA hydrogel is thermoresponsive and the length of the supramolecular dsDNA structures varies with temperature.

In 2006, Luo and coworkers first reported the construction of a hydrogel entirely made from branched DNA *via* an enzymatic ligation reaction (Um, S. H.; Lee, J. B.; Park, N.; Kwon, S. Y.; Umbach, C. C.; Luo, D. Enzyme-catalysed assembly of DNA hydrogel. Nat. Mater. 2006, 5 (10), 797-801). In this process, DNA served as both a linker and a substrate. To form the DNA hydrogel, branched DNA motifs, such as X-, T-, or Y-shaped DNA (termed as X-DNA, Y-DNA, and T-DNA), were first prepared through the self-assembly of rationally designed ssDNA strands. They also reported an enzyme-catalyzed assembly of DNA hydrogels through 3D-entanglement of DNA products by combination of the processes of rolling circle amplification (RCA) and multi-primed chain amplification (MCA) (Lee, J. B.; Peng, S.; Yang, D.; Roh, Y. H.; Funabashi, H.; Park, N.; Rice, E. J.; Chen, L.; Long, R.; Wu, M.; Dan, L. A mechanical metamaterial made from a DNA hydrogel. Nat. Nanotechnol. 2012, 7 (12), 816-820). After running RCA and subsequent MCA, a continuous chain reaction was established, thereby creating extremely long DNA molecules which resulted in a physically linked DNA hydrogel.

By introducing G-quadruplex inside the RCA-generated DNA hydrogel, the structure and function of hemin/G-quadruplex moieties were implemented in the hydrogel, which showed a high capability in catalysing the $ABTS^{2-}$ colorimetric reaction (Huang, Y.; Xu, W.; Liu, G.; Tian, L. A pure DNA hydrogel with stable catalytic ability produced by one-step rolling circle amplification. Chem. Commun. 2017, 53 (21), 3038-3041).

Based on RCA, multifunctional DNA nanostructures termed nanoflowers (NFs) were self-assembled from long DNA building blocks for multiplexed cellular imaging/trackable targeted drug delivery (Hu, R.; Zhang, X.; Zhao, Z.; Zhu, G.; Chen, T.; Fu, T.; Tan, W. DNA nanoflowers for multiplexed cellular imaging and traceable targeted drug delivery. Angew. Chem., Int. Ed. Engl. 2014, 126 (23), 5931-5936; Zhu, G.; Hu, R.; Zhao, Z.; Chen, Z.; Zhang, X.; Tan, W. Noncanonical self-assembly of multifunctional DNA nanoflowers for biomedical applications. J. Am. Chem. Soc. 2013, 135 (44), 16438-16445; Lv, Y.; Hu, R.; Zhu, G.; Zhang, X.; Mei, L.; Liu, Q.; Qiu, L.; Wu, C.; Tan, W. Preparation and biomedical applications of programmable and multifunctional DNA nanoflowers. Nat. Protoc. 2015, 10 (10), 1508-1524; Sun, W.; Jiang, T.; Lu, Y.; Reiff, M.; Mo, R.; Gu, Z. Cocoon-like self-degradable DNA nanoclew for anticancer drug delivery. J. Am. Chem. Soc. 2014, 136 (42), 14722-14725) and immobilization of proteins (Kim, E.; Zwi-Dantsis, L.; Reznikov, N.; Hansel, C. S.; Agarwal, S.; Stevens, M. M. One-Pot Synthesis of Multiple Protein - Encapsulated DNA Flowers and Their Application in Intracellular Protein Delivery. Adv. Mater. 2017, 29, 1701086).

Wang *et al.* developed a clamped hybridization chain reaction (HCR)-based strategy, including crosslinked self-assembly and clamped hybridization of hairpin strands and their initiator strand, to guide DNA self-assembly to form hydrogel (Wang, J.; Chao, J.; Liu, H.; Su, S.; Wang, L.; Huang, W.; Willner, I.; Fan, C. Clamped Hybridization Chain Reactions for the Self - Assembly of Patterned DNA Hydrogels. Angew. Chem., Int. Ed. Engl. 2017, 56 (8), 2171-2175).

Preferably, the DNA hydrogel in the composite material of the present invention can be produced by an enzymatic reaction-mediated polymerization or by hybridization chain reaction starting with DNA strands which are covalently coupled to the surface of the silica nanoparticles. Both methods can also be combined to generate the DNA hydrogel by two complementary mechanisms.

According to an embodiment, variations of the DNA backbone in the hydrogel can be implemented. For example, the introduction of restriction sites in conventional DNA polymers enables the design of responsive materials that can be post-synthetically modified with enzymes. To introduce restriction sites into the single-stranded backbone of the composite material according to an embodiment, stem-loop structures of appropriate sequence can be encoded in a rolling circle amplification (RCA) template, thereby enabling efficient enzymatic degradation and concomitant gel-to-sol transition of the composite material. This feature is of great utility, for instance, to achieve controllable release of polymers or polymer-embedded cells, which is required for therapeutic applications and dynamic cell manipulations.

In a further embodiment, the DNA backbone may have an aptamer which specifically binds to a receptor of a cell. According to another embodiment, the DNA backbone may have a drug association site which can be used for enrichment of intercalating drugs.

According to another embodiment, fluorescent properties can be generated by rolling circle amplification (RCA)-based incorporation of chemically modified deoxynucleotides bearing fluorescent dyes, such as fluorescein (FITC)-modified dUTP.

(b) Silica nanoparticles

In the present invention, the average particle size of the silica nanoparticles (SiNP) is not particularly limited. In an embodiment, the average particle size is in the range of from 2 nm to 10 μm. In another embodiment, the average particle size is in the range of from 20 nm to 2 μm. According to a further embodiment, the average particle size is in the range of from 50 nm to 1 μm. In another embodiment, the average particle size is in the range of from 60 nm to 100 nm. The average particle size can be measured by a transmission electron microscope (TEM).

By providing silica nanoparticles having an average particle size in the range of from 5 nm to 10 μm according to an embodiment, a composite material having an advantageous porosity and stability can be achieved.

In an embodiment, the silica nanoparticles are functionalized silica nanoparticles. The functionalization is not particularly limited as long as the chemical group allows the binding to a different molecule. Preferably, the functional group is at least one group selected from amino, thiol, phosphonate, carboxy, alkene, alkyne, halo-alkane, maleimide, succinimide and azide.

According to another embodiment, the silica nanoparticles have a fluorescence marker. This enables fluorescence tracking of the composite material and may be used for e.g. studies of cell uptake and degradation of hydrogels. For example, dye-encoded SiNP can be used. Dye-encoded SiNP can for example be obtained through co-hydrolysis of TEOS and Cy5 dye-conjugated silane as described in the literature (Leidner, A., Weigel, S., Bauer, J., Reiber, J., Angelin, A., Grösche, M., Scharnweber, T., Niemeyer Christof, M. (2018) Biopebbles: DNA-Functionalized Core-Shell Silica Nanospheres for Cellular Uptake and Cell Guidance Studies. Adv. Funct. Mater. DOI 10.1002/adfm.201707572 and references cited therein).

(c) Carbon nanotubes

In an embodiment of the present invention, the carbon nanotubes are single-walled carbon nanotubes. According to another embodiment, the carbon nanotubes are multi-walled carbon nanotubes. Other morphologies of carbon nanotubes, such as peapod and nanobud carbon nanotubes as well as nanotube derivatives containing junctions, metal-doped or metal-coated sites and other allotropes of carbon, such as fullerenes, also represent embodiments.

Preferably, the average diameter of single-walled carbon nanotube is from 0.5 to 1 nm, more preferred from 0.7 to 0.9 nm.

In an embodiment of the present invention, the median length of a carbon nanotube is from 500 nm to 1.5 μm, preferably from 750 nm to 1.25 μm, most preferred from 900 nm to 1.1 μm.

The average diameter and the median length of the carbon nanotube can either be retrieved from the manufacturer (e.g. in the Sigma website) or measured by commonly known techniques. The average diameter can be evaluated by using a Raman microscope and the median length can be measured by an atomic force microscope (AFM).

The carbon nanotubes used in the present invention may be commercially available carbon nanotubes provided by e.g. Sigma.

(2) Hollow spherical container

Further, the present invention relates to a hollow spherical container, wherein the wall of the hollow spherical container comprises at least one layer containing the previously defined composite material.

In an embodiment, the wall of the hollow spherical container further comprises at least one additional layer comprising at least one component selected from DNA-protein conjugate, DNA-ligand conjugate, a DNA origami nanostructure-based ligand assembly system, as well as proteins and ligands such as carbohydrates, hormones, peptides and small-molecule organic ligands. When at least one of such an additional layer is used, the at least one layer containing the previously defined composite material forms the outermost layer of the wall of the hollow spherical container while the at least one additional layer forms an inner layer of the wall of the hollow spherical container.

In another embodiment, additional functional components, such as proteins (e.g. membrane proteins), may be incorporated into the at least one layer of the hollow spherical container comprising the composite material.

The word "spherical" used herewith means that the ratio between the minor axis and the major axis of the container is not less than 0.8, preferably not less than 0.9, most preferably not less than 0.95.

Preferably, the wall of the hollow spherical container is porous. In an embodiment, the pore size of the wall is more than 200 nm and less than 500 nm. The preferred pore size can be measured as outlined above using MPT microrheology. By providing pores in the wall, for example small molecules may enter the inside of the hollow spherical container.

In an embodiment of the present invention, the hollow spherical container has a diameter of 30 μm or more and less than 1 mm. According to a further embodiment, the hollow spherical container has a diameter in the range of from 50 μm to 200 μm, preferably from 80 μm to 160 μm. By setting the diameter in the above range, a container can be provided which allows performing applications on a small scale while being dimensionally stable. The smaller the diameter of the hollow spherical container is, the higher the dimensional stability.

According to an embodiment of the hollow spherical container, each layer has a thickness in the range of from 5 nm to 10 μm. In another embodiment, the thickness is in the range of from 50 nm to 8 μm, preferably from 100 nm to 5 μm. When having a thickness of less than 5 nm, the layer may not provide sufficient dimensional stability.

In an embodiment of the hollow spherical container, the thickness of all layers is in the range of from 5 nm to 100 μm. In an embodiment, the thickness is in the range of from 50 nm to 50 μm, preferably from 100 nm to 10 μm. When having a thickness of less than 5 nm, the total thickness of the layers may not provide sufficient dimensional stability.

The diameter of the hollow spherical container, the thickness of each layer, and the thickness of all layers comprised in the hollow spherical container can be measured by using confocal laser scanning fluorescence microscopy. The thickness of all layers refers to the length of the hydrogel fluorescence shell measured by confocal laser scanning fluorescence microscopy. The thickness (T) of all layers can be calculated according to the formula T = T2 - T1, where T2 and T1 are Cy5 fluorescence (included in the shell of the container) starting point and ending point in shell of the image, respectively. The positions of T1 and T2 are determined from microscopic images by the commercial software Zen Blue and the connecting line between T1 and T2 is perpendicular to the tangent of T1.

According to an embodiment, the wall of the hollow spherical container comprises at least two layers. Further, the wall of the hollow spherical container may comprise at least three layers.

In a preferred embodiment, the at least two layers in the wall of the hollow spherical container differ in their composition. By combining at least two layers, the dimensional stability of the hollow spherical container can be advantageously adjusted. For example, each of the two layers may comprise the composite material, wherein the content of the silica nanoparticles, the carbon nanotubes and the DNA hydrogel are adjusted. In another embodiment, the previously defined additional layer may be used in combination with at least one layer comprising the composite material.

Figure 3 exhibits an example of a hollow spherical container according to the present invention, wherein the container comprises three layers.

In a preferred embodiment, the hollow spherical container is obtained by a process comprising the following steps in the order:

(i) providing a reaction solution comprising the components of the composite material;
(ii) providing a continuous phase which is immiscible with the reaction solution; and
(iii) combining the reaction solution and the continuous phase thereby forming droplets comprising the reaction solution, wherein the droplets are dispersed in the continuous phase and the components of the composite material contained in the reaction solution are absorbed to the inner surface of the droplets.

In an embodiment of the present invention, the components of the composite material provided in the above step (i) comprise the previously defined silica nanoparticles and DNA strands which form the DNA hydrogel during the above process. Preferably, the previously defined carbon nanotubes are also contained in the components of the composite material in step (i). In a further embodiment, the components of the composite material comprise the additional previously defined compounds which may be present in the composite material.

The reaction solution and the continuous phase are not specifically limited as long as they are immiscible, and the reaction solution comprises the components of the composite material.

According to an embodiment of the present invention, the continuous phase is a liquid phase or a gas phase.

In an embodiment, one of the reaction solution and the continuous phase is a hydrophobic phase while the other is a water-based phase. According to a preferred embodiment, the reaction solution is a water-based phase and the continuous phase is a hydrophobic phase.

An oil is preferably used in the hydrophobic phase. The oil may be selected from a mineral oil or a fluorocarbon oil. An example for a mineral oil is bioreagent for molecular biology from Roth (Mineralöl für die Molekularbiologie, Art. HP50.3). Further, as the fluorocarbon oil, at least one of QX200 Droplet Generation Oil for EvaGreen (BioRad), HFE 7500 (3M), Fluoridrop 7500 (Dolomite Microfluidics), Fluoridrop 40 (Dolomite Microfluidics), FC40 (3M) and Novec 7500 (3M) may be used.

In an embodiment of the present invention, at least one of the reaction solution and the continuous phase comprises a surfactant. The surfactant is not particularly limited as long as it changes the interfacial tension between the reaction solution and the continuous phase. In specific, the surfactant can be an ionic or a non-ionic surfactant. Examples of surfactants are PicoSurf 1 (Dolomite Microfluidics), Span 40/80, Tween20/80, SDS, ABIL EM90 (Baret, J.-C. Surfactants in droplet-based microfluidics. Lab Chip 2012, 12 (3), 422-433), 1,2-dioleoyl-3-trimethylammonium-propane (DOTAP), triton X-100, ABIL EM90, oleic acid, monolein, Krytox FSH, 1,2-dioleoyl-sn-glycero-3-phosphocholine (DOPC), 2-oleoyl-1 -palmitoyl-sn-glycero-3-phosphocholine (POPC), 1,2-dipalmitoyl-sn-glycero-3-phosphoethanolamine (DPPE), 1,2-dimyristoyl-sn-glycero-3-phosphocholine (DMPC), 1,2-dipalmitoyl-sn-glycero-3-phosphocholine (DPPC), and hydrophobic particles.

According to a preferred embodiment, the continuous phase comprises an anionic or a cationic surfactant. By using an anionic or a cationic surfactant in the continuous phase, the droplet can be advantageously stabilized. Further, provided that the components of the composite material are oppositely charged compared to the surfactant, said components of the composite material can be easily absorbed to the inner surface of the droplets by electrostatic attraction.

In a preferred embodiment of the present invention, a positively charged surfactant, such as DOTAP, is used as a surfactant in the continuous phase. By using a combination of a positively charged surfactant in combination with negatively charged DNA molecules, carbon nanotubes or silica nanoparticles or negatively charged DNA strand-modified silica nanoparticles or negatively charged DNA strand-modified carbon nanotubes, the components of the composite material can be easily absorbed to the inner surface of the droplet.

According to an embodiment, in the process for obtaining the hollow spherical container, the combining in step (iii) is carried out with a microfluidic droplet generator. In another embodiment, all of the steps of the process for obtaining the hollow spherical container are carried out with a microfluidic droplet generator.

Preferably, in step (iii), the droplets are generated in flow, i.e. the reaction phase passes a narrow point while being combined with the continuous phase upon which the reaction phase forms droplets being dispersed in the continuous phase, since the form of a droplet is energetically favorable. The optionally added surfactant increases the tendency of forming droplets.

The microfluidic droplet generator used in the embodiment of the present invention is not particularly limited as long as it allows the generation of droplets in the sub-millimeter scale.

The use of a microfluidic droplet generator in contrast to commonly used droplet techniques is favorable, since it can provide the generation of highly monodisperse droplets with the advantage of low sample consumption. The droplets are formed, if two immiscible liquids are combined for a defined time.

In an embodiment of the present invention, the volume of a droplet is in a range of from 0.2 nL to 20 nL. Preferably, the volume of a droplet is in the range of from 0.3 nL to 2 nL. The volume of a droplet can be calculated according to the volume formula $4/3*\pi*r^3$, where r is the droplet radius which can be calculated from microscopic images. The monodispersity depends on the specific combination of the components.

In order to generate droplets by using a microfluidic droplet generator, various methods can be used such as the T-junction, Head-on, Double T-junction, Flow-Focusing and Step-Emulsification. These methods are advantageous, since they can be precisely tuned by varying a number of different parameters. According to a preferred embodiment, the Flow-Focusing method is used, since said method can provide a higher monodispersity and a higher amount of droplets being generated in the same amount of time compared to other methods.

In a microfluidic droplet generator, the generation of droplets comprising different compounds and the addition of new compounds at defined time points can be achieved with various unit operations. The simplest approach is to premix everything outside the generator resulting in the encapsulation of all compounds. If the compounds should not react with each other before encapsulation, they can be encapsulated by two laminar flows meeting at the encapsulation junction. A second option is to generate two droplets in different junctions and fuse them in either passive structures or active structures. Passive structures only use geometrical confinements in order to change the droplet stability regarding unwanted coalescences, whereas active structures use electrodes to change the interfacial tension of the droplets and the surrounding oil. Another possible method is the so-called picoinjection. With this procedure, a second fluid is directly injected into the droplet via a side channel. Since microfluidic flows are total laminar, a meander like structure is advantageous to support combining the different compounds.

In an embodiment of the combining step (iii) in the process for obtaining the hollow spherical container, each of the reaction solution and the continuous phase has a flow rate in the range of from 0.1 $\mu$L/min to 1000 $\mu$L/min. By adjusting the flow rates of each of the reaction solution and the continuous phase, the volume of the droplets can be precisely varied. Preferably, the flow rates of each of the reaction solution and the continuous phase is in the range of from 0.5 $\mu$L/min to 100 $\mu$L/min. More preferred is a flow rate for the continuous phase in the range of from 10 to 30 $\mu$L/min while the reaction solution has a flow rate in the range of from 0.5 $\mu$L/min to 5 $\mu$L/min. When using said specific combination of the flow rates for the reaction solution and the continuous phase, droplets with a defined volume in the range of from 0.3 nL to 2 nL can be generated.

In addition, the volume of the droplets can be precisely varied by using different channel geometries, flow rates and components in the reaction solution and the continuous phase.

According to an embodiment, the droplets are collected after step (iii) and incubated. For collecting the droplets, a well plate may be used. In an embodiment of the present invention, the droplets are incubated at a temperature in the range from 20 °C to 40 °C, preferably from 25 °C to 37 °C. In another embodiment, incubation is carried out for 1 to 60 h, preferably from 40 to 50 h.

In the following, an example of a microfluidic droplet generator, which can be used in the present invention, will be described.

The material which can be used for the structure of the microfluidic droplet generator is not particularly limited. For example, one of polydimethylsiloxane (PDMS) and poly(methyl methacrylate) (PMMA) can be used. In order to close the structure to enable fluidic application, the structure can be exposed to thermal plasma oxidation and be bonded onto glass (e.g. microscope slides) or a sealing foil (e.g. polyolefin) can be applied.

In Figure 4, an example of a microfluidic droplet generator which can be used in the present invention is shown which comprises five elements/functional units.

The first unit is the inlet for the continuous phase 1, which comprises two identical arms with any desired profile. In the specific example shown here it is 300 μm x 100 μm (width x height).

The second unit is the inlet for the reaction solution 2, which intersects the two arms of the first unit, resulting in the third unit, i.e. the junction for droplet generation (see detail B in Figure 4).

The junction (i.e. the third unit) has a rectangular canal profile of 100 μm x 100 μm in the specific example shown here.

The fourth canal making up this junction leads to the droplet collection chamber (i.e. the fourth unit). In the specific example shown here, the dimensions of the droplet collection chamber are 21 x 5 mm (length x width) with a depth of 400 μm in the main part of the canal structure. Coming from the entry canal (in the example: 100 μm x 100 μm), the profile is expanded stepwise until the full chamber dimensions are met.

The fifth element is the outlet 3 with a profile of 800 μm x 200 μm (width x height) in the specific example shown here. The inlets/outlets can be realized by using drills with a diameter of 800 μm. The inlets/outlets can be sealed to a connector plate with o-rings made from NBR 70. The connector plate is preferably in turn connected to precision pumps utilizing special fittings and tubings.

In an embodiment, the channels of the microfluidic droplet generator can be treated with substances which make the channels more hydrophobic and/or fluorophobic and/or hydrophilic. Preferably, the channels can be treated with Pico-Glide (Dolomite Microfluidics) or via layer-by-layer deposition of poly(allylamine hydrochloride) and poly-(sodium 4-styrensulfonate) (also see Bauer, W.-A. C.; Fischlechner, M.; Abell, C.; Huck, W. T. Hydrophilic PDMS microchannels for high-throughput formation of oil-in-water microdroplets and water-in-oil-in-water double emulsions. Lab Chip 2010, 10 (14), 1814-1819).

(3) Microreactor

Further, the present invention relates to a microreactor comprising the above-defined hollow spherical container and at least one cell located in the space enclosed by the hollow spherical container. The microreactor according to the present invention can be advantageously used for investigations of cells, since the composite material according to the present invention does not show cytotoxicity.

The at least one cell located in the space enclosed by the hollow spherical container is not particularly limited and corresponds to the cell type defined in the context of the composite material.

The term "microreactor" according to the present invention means a device with a size of less than 1 mm in which chemical or biological reactions can be carried out.

Preferably, the microreactor can be used for the cultivation of cells inside the microreactor. The hollow spherical container provides mechanical stability for the manipulation, but at the same time cells can grow inside. Preferably, small molecules can enter the hollow spherical container due to the walls being porous. The at least one cell located in the space enclosed by the hollow spherical container can be supplied with nutrients and/or treated with pharmaceuticals to analyze the cellular response.

In Figure 5, an example of a microreactor according to the present invention is shown on the right side which comprises several cells in the space enclosed by the hollow spherical container.

Based on the variation of the components contained in the composite material, the wall thickness and the diameter, the hollow spherical container may be easily moved mechanically. Further, the hollow spherical container is compatible with the at least one cell which is enclosed by said hollow spherical container. Due to the encapsulation of the at least one cell in the microreactor, individual experiments can be carried out at a small scale.

In an embodiment of the present invention, the microreactor is semipermeable. The term "semipermeable" used herein means that certain molecules and/or ions can pass through the wall of the microreactor while other substances cannot pass the wall. According to an embodiment, substances that can pass the wall of the microreactor may be chosen from substances that are necessary for the cell viability and/or therapeutic products. The degree of permeability of the microreactor can be adjusted by the porosity of the wall of the hollow spherical container.

Preferably, the microreactor is optically transparent and thereby enables the application of high-throughput screening. Due to the size of the microreactors, pharmacological screens can be carried out at a small scale.

According to an embodiment of the present invention, individual DNA oligomers may be introduced as codes into the wall of the hollow spherical container which allows decoding or deconvolution to identify the microreactor and the substances being located inside the microreactor after performing experiments.

The present invention further relates to the use of the above-defined microreactor for high-throughput screening.

(4) Microgels

Further, the present invention relates to a microgel comprising the composite material according to the present invention.

The term "microgel" according to the present invention refers to a hydrogel of microscale size, wherein the average particle size of the microgel is less than 1 mm.

In an embodiment of the present invention, the microgel has an average particle size of from 0.05 to 50 μm, preferably from 0.1 to 20 μm, more preferably, from 1 to 10 μm, and, even more preferably, from 1 to 5 μm. The average particle size is measured by using fluorescence microscopy.

In the microgel comprising the composite material, the carbon nanotubes (CNT) can be mixed with the silica nanoparticles (SiNP) in different weight ratios when the ternary composite material is employed. Preferably, the mass ratio of SiNP:CNT in the ternary composite material is from 100:1 to 1:1, preferably from 50:1 to 2.5:1. In an embodiment, the maximum concentration of SiNP in the binary SiNP-DNA hydrogel composite material and in the ternary SiNP/CNT-DNA hydrogel composite material is 20 mg/mL, preferably 4000 μg/mL. According to a further embodiment, the maximum concentration of CNT in the ternary SiNP/CNT-DNA hydrogel composite material is 1000 μg/mL, preferably 320 μg/mL.

The method for manufacturing the microgel is not particularly limited. For example, micellar techniques, using, for instance, "inverse micelles" of water-in-oil droplets can be used. Then, after incubation, the particles of the microgel can be collected.

Further, the present invention relates to the use of the microgel for the delivery of cargo to the cytosolic compartment of cells. Owing to the favorable characteristics and biocompatibility, the microgel is readily ingested by cells whereupon they might be transported within the cell. In a preferred embodiment, during this transport, the microgel remains intact and, more preferably, resides in close proximity to the nucleus. Thus, the microgel of the present invention can be used to realize delivery of cargo to cells. The cargo to be delivered is not particularly limited and can be selected from pharmacologically active compounds, drugs, metals, metal and semiconductor nanoparticles, metal chelates, proteins, peptides, and DNA molecules that encode genes for RNA and protein production.

In an embodiment, the microgel of the present invention can be used for gene transfection. Preferably, the microgel can be used as a "synthetic cellular compartment" for the production of endogeneous components, such as messengerRNA or small-interfering RNAs, which has many implications for diagnostics and therapy *in vitro* and *in vivo.*

The cell employed in the above uses of the microgel is not particularly limited and corresponds to the cell type defined in the context of the composite material.

The composite material according to the present invention not only displays cell attractiveness and stiffness, but can also be conveniently designed with inherent functionality of the connecting DNA backbone by incorporation of enzymatic restriction sites and aptamer moieties for specific binding of cellular components, such as proteins, peptides, nucleic acids or glycans located on the inner or outer side of the cell surface or intracellular structures, such as the nucleus, chromosomes, mitochondria, endoplasmatic reticulum or cytosolic vesicles.

Adapting the relative amount of SiNP:CNT leads to gradual changes in the mechanical properties. This feature can be used for controlling cellular adhesion on and transmigration through the hydrogel matrix as well as for controlling cellular uptake and intracellular stability and degradation.

The composite material of the present invention can be employed as a novel substratum for cell adhesion that outperforms standard tissue culture surfaces.

Further, the modularity of the synthesis allows convenient incorporation of fluorescent nanoparticles which renders the composite material of the invention trackable by optical means, which is of utmost importance for biomedical studies, such as materials uptake, cellular degradation or other cell-surface interactions based on adhesion, motility and propagation.

The figures show:

Figure 1 shows an embodiment of the composite material of the present invention comprising DNA hydrogel, silica nanoparticles (SiNP) and carbon nanotubes (CNT).

Figure 2 exemplifies RCA-based synthesis for obtaining binary and ternary SiNP/CNT-DNA hydrogel composite materials. SiNP and/or CNT modified with ssDNA primers are subjected to RCA-based primer extension, which leads to formation of the corresponding pure (S100, binary SiNP-DNA hydrogel or C100, binary CNT-DNA hydrogel) or mixed (SCx (ternary)) composite materials. While S100 and SCx represent embodiments of the present invention, C100 is a general example.

Figure 3 exhibits an example of a hollow spherical container according to the present invention, wherein the container comprises three layers.

Figure 4 shows an example of a microfluidic droplet generator which can be used in the present invention.

Figure 5 displays an embodiment of the microreactor according to the present invention on the right side comprising several cells in the space enclosed by the hollow spherical container.

Figure 6 shows an example of the synthesis of multifunctional oligonucleotide-modified SiNP.

Figure 7 displays SEM images of freeze-dried SiNP-DNA hydrogel synthesized by RCA of P1 primer on SiNP-P at 30 °C for 48 h.

Figure 8 presents DLS measurements of the hydrodynamic size of RCA products produced from RCA of P1 primer on SiNP-P after hybridized with template T in the presence or absence of T4 ligase (insert is the digital photograph of SiNP-DNA hydrogel synthesized by RCA of P1 primer on SiNP-P at 30 °C for 48 h).

Figure 9 shows the variation of dynamic shear-moduli G' (closed symbol) and G" (open symbol) of SiNP-DNA hydrogel synthesized by RCA of P1 primer on SiNP-P at 30 °C for 48 h as a function of frequency.

Figure 10 exhibits the hydrodynamic size of HCR products produced from HCR of H1 and H2 on SiNP-lh or SiNP-I (insert is the digital photograph of SiNP-DNA hydrogel synthesized by HCR of H1 and H2 on SiNP-I under room temperature for 48 h).

Figure 11 displays SEM images of freeze-dried S100 hydrogel products generated from HCR of H1 and H2 on SiNP-I at room temperature for 48 h.

Figure 12 shows the results of a frequency-sweep rheological test (0.05 and 100 rad $S^{-1}$, 1% strain, 25 °C) for SiNP-DNA hydrogel S100 generated *via* HCR of H1 and H2 on SiNP-I at room temperature for 48 h.

Figure 13 exhibits CLSM images of DOTAP droplets with Cy5-doped SiNP as an interior surface after incubation at 30 °C for 48 h. Three images show before photobleaching (a), after photobleaching (b), and after fluorescence recovery for 2 h (c), respectively. The central angels between two arrows represent the bleaching range of the membrane and the scale bar represents 50 $\mu$m. Note that fluorescence recovery after photobleaching occurs on a fast time scale for the DOTAP lipid molecules (top row) while the (middle row) SiNP show a much lower diffusion rate, thereby indicating high cross-linking rates and successful polymerization of the DNA-SiNP hydrogel.

Figure 14 shows the structural features of an embodiment of the SC50 composite material comprising silica nanoparticles, carbon nanotubes and a DNA hydrogel characterized by SEM. The SCx materials of Table 2 show similar morphological structures.

Figure 15 shows the characterization of an embodiment of SiNP/CNT-DNA hydrogel composite materials. Self-assembly of SiNP to S100 during RCA reaction. (a and b) Representative HAADF-STEM imaging (first column), EDS elemental mapping (second to sixth column in Figures a and b) and (c) relative atomic ratios of N and P to Si of SiNP-P and $S100_{48h}$ (shown as "SDH"), respectively. Data represent mean $\pm$ S.D. of the EDS measurements determined over fifty particles in four random areas. (d) Hydrodynamic size of products produced from RCA on SiNP-P constructed in the presence or absence of T4 DNA ligase (inset is the photograph of $S100_{48h}$). (e, f) the trajectory map of tracer particles and (g, h) the slope <MSD> of tracer particles in SiNP-P and $S100_{48h}$, respectively. (i, j) the slope <MSD> of tracer particles in C100 and SC50, respectively. The bright line represents the average MSD.

Figure 16 shows representative TEM images of SiNP-P and S100 obtained after 48 h of RCA reaction.

Figure 17 shows representative SEM images of S100 samples drawn at variable time points during RCA polymerization. Note that the sample drawn at t=0 ($S100_{0h}$) only shows SiNP particles that aggregate to superlattices in the course of dehydration during SEM specimen preparation.

Figure 18 shows examples of the incorporation of fluorescent properties into S100 *via* (a) the use of Cy5 fluorescently-labeled SiNP, (b) enzymatic introduction of chemically modified deoxynucleotides (FITC-dUTP), and (c) through both aforementioned strategies. The resulting hydrogels were analyzed by confocal fluorescence microscopy. The shown images are 3D reconstructions from z-stack analyses (Cy5 dark gray, FITC light gray).

Figure 19 shows representative SEM images of Cy5-encoded S100 materials (Cy5@S100 (Figure 19a), S100-FITC (Figure 19b), and Cy5@S100-FITC (Figure 19c).

Figure 20 illustrates the engineering of the ssDNA backbone by incorporation of enzymatic restriction sites. (a) Predicted secondary structures of RCA product obtained from template T bearing stem-loop structures and double stranded regions with BstEII restriction sites (5'-GAATTC-3') in a repeated junction structure. SEM images of S100 treated for 2 h (37 °C) with restriction enzymes BstEII-HF (b) or, as a control, with SexAI (c). The arrows point at representative DNA linkers between particles. Note that enzymatic digestion leads to particle assemblies (b) similar as obtained from unpolymerized SiNP (Figure 17), whereas treatment with the non-complementary restriction endonuclease SexAI does not change the gel's morphology (c).

Figure 21 shows an example of fluorescently trackable uptake of SC100 by HeLa cells. (a) Predicted secondary structures of the RCA product obtained from template T. Structures were predicted using the Nupack software (J. N. Zadeh, C. D. Steenberg, J. S. Bois, B. R. Wolfe, M. B. Pierce, A. R. Khan, R. M. Dirks, N. A. Pierce, J. Comput. Chem. 2011, 32, 170). Note the presence branched Sgc8 aptamer structures protruding from the backbone. The Sgc8 aptamer specifically binds to PTK7 receptors overexpressed on many cancer cells (D. Shangguan, Y. Li, Z. Tang, Z. C. Cao, H. W. Chen, P. Mallikaratchy, K. Sefah, C. J. Yang, W. Tan, Proc. Natl. Acad. Sci. U. S. A. 2006, 103, 11838; G. Zhu, R. Hu, Z. Zhao, Z. Chen, X. Zhang, W. Tan, J. Am. Chem. Soc. 2013, 135, 16438). According to an embodiment, RCA polymerization was carried out with Cy5-embedded SiNP to yield nanocomposite $S100_{apt}$. (b-f) Confocal fluorescence microscopy images of PTK7-positive HeLa cells treated with 100 μM $S100_{apt}$ that was polymerized by RCA for 0 h (b), 2 h (c), 4 h (d), or 6 h (e). For control, cells in (f) were treated with 100 μM of the free aptamer. Note that increasing polymerization times (and thus increasing amounts of incorporated aptamers) lead to increase in Cy5 fluorescence (third column). Filamentous actin and nuclei were stained with Alexa Fluor 488 Phalloidin (stripe-like structures in the first and fourth column) and DAPI (spots in second and fourth column), respectively. Scale bars are 20 μm.

Figure 22 shows an example of trackable drug delivery to PTK7-positive HeLa cells. (a) Predicted secondary structure of the DNA backbone of fluorescent S100 material produced by RCA according to an embodiment. Note the presence of Sgc8 aptamer and GG/GC-rich stem loops, the latter of which serve as binding sites for the intercalating drug Doxorubicin (DOX). Representative confocal microscopy images of HeLa cells treated with (b) 5 μM free DOX or S100, polymerized for variable times (c, 12 h; d, 24 h; e and f, 48 h) before loading with 5 μM DOX. The cells in (f) were additionally treated with free Sgc8 aptamer. In all cases, cells were incubated with DOX/S100 for 2 h, washed, fixed and subjected to staining of the filamentous actin with Alexa Fluor 488 Phalloidin (stripe-like structures in the first and fourth column). Scale bars are 20 μm. Note that the amount of Cy5-labeled S100 (second column) as well as of DOX (spots in third and fourth column) increases with increasing amounts RCA-synthesized DNA backbone.

Figure 23 exhibits an example of a CCK-8 viability assay of MCF-7 cells after treated with SiNP-DNA hydrogel (S100), CNT-DNA hydrogel (C100), SiNP/CNT-DNA composite hydrogel (SCx, where x gives the mass ratio of SiNP-P:CNT-P).

Figure 24 shows viability, proliferation and migration of $MCF7_{eGFP}$ cells adhered on thin films of the various composite materials according to an embodiment. (a) Schematic illustration of the preparation of fresh and dried composite material films for cultivation of cells. (b,c) Quantification of cells grown on fresh (b) or dried (c) thin films using the CCK-8 assay. The bars indicate relative cell numbers normalized to initially seeded cells (4 h, left column). STC data was obtained from polylysine-coated glass surface lacking hydrogels. (d,e) Representative fluorescence microscopy images of $MCF7_{eGFP}$ cells (spots) after adhesion on STC or SC50 surfaces for 24 h. (f, g) Directional lateral migration of $MCF7_{eGFP}$ cells (spots), which were seeded in a petri dish on STC surface and allowed to migrate to a patch of Cy5-labeled dried SC50. The fluorescence microscopy images of the border region between STC and SC50 surfaces (g) illustrate the gradual migration of the cells from STC to SC50 surfaces.

Figure 25 shows an example of transmitted light inverted microscopy (EVOS™ FL Imaging System) analysis of MCF7 cells adhered for 24 h on the various surfaces of dried hydrogels or, for control, on STC surface. Note that cells on S100 and SCx surfaces reveal a more pronounced elongated fusiform and flattened morphology than cells adhered on STC surface.

Figure 26 shows examples of fluorescence microscopy images of $MCF7_{eGFP}$ cells cultivated on dried SC50 surface prepared from Cy5-labeled SiNP. Note that the cells have not transmigrated through the dried gel matrix. The shown images are 3D reconstructions from z-stack analyses (Cy5: surface, GFP: spots on surface).

Figure 27 shows examples of transmitted light inverted microscopy (EVOS™ FL Imaging System) images of REF52 cells adhered onto the various dried hydrogels and STC surfaces for 4 h and 24 h. Note that cells on S100 and SCx surfaces reveal a more pronounced elongated fusiform and flattened morphology than cells adhered on STC surface.

Figure 28 shows quantification of REF52 cell proliferation grown on dried hydrogel films using the CCK-8 assay. The right bars indicate relative cell numbers normalized to initially seeded cells (4 h). STC data was obtained from polylysine-coated glass surface lacking hydrogels.

Figure 29 shows an example of fluorescence microscopy analysis of $MCF7_{eGFP}$ cells cultivated for 24 h on the dried Cy5-labeled SiNP-P (Cy5: stripes in the background and the role in front, GFP: spots).

Figure 30 shows an example of competitive adhesion of $MCF7_{eGFP}$ cells (spots) to either STC or Cy5-labeled dried SC50 (right-hand site). 1 mL medium containing $1.2 \times 10^4$ $MCF7_{eGFP}$ cells was added to a petri dish (Ibidi) containing a patch of dried SC50 (a) Schematic drawing of the experiment. (b) Fluorescence microscopy image of the border region between STC and SC50 surfaces.

Figure 31 shows an example of the relative cell number of MCF7 cells adhered for 1 h (left column), thoroughly washed to remove loosely attached cells (middle column), followed by culturing for additional 24 h (right column). The numbers indicate percentage increase of proliferated cells. Note that increasing amounts of CNT lead to increased adhesion strength but decreased proliferation.

Figure 32 shows an example of cellular transmigration, adhesion and release. Fluorescence images of $MCF7_{eGFP}$ cells (spots) cultivated on freshly prepared Cy5-labeled SC50 and SC25 (layer) for variable times.

Figure 33 shows an example of a fluorescence image of the SiNP/CNT-DNA composite microgel SC50 according to an embodiment.

Figure 34 shows an example of the fluorescence image of REF52 cells after incubation with SiNP/CNT-DNA microgel SC50 according to an embodiment. Note that the microgel particle (irregular-shaped spot) remained intact and resides in close proximity to the nucleus (round spot in the middle).

Figure 35 shows an example of fluorescence images of REF52 cells after being incubated with SiNP/CNT-DNA microgel SC50 according to an embodiment encoded with mKate gene fragments for different time periods. Note that the REF52 cells show the typical red fluorescence of expressed mKate protein after 5 days (day 6) of culturing.

The present invention is more specifically explained below by examples. However, the present invention is not limited to those examples.

*Materials:* Tetraethyl orthosilicate (TEOS), 3-(trihydroxysilyl)propyl methylphosphonate (THPMP, monosodium salt solution), (3-aminopropyl)trimethoxysilane (APTMS), poly(ethylene glycol) methyl ether maleimide (mPEG-mal, molecular weight ~ 2000), N1 -(3-trimethoxysilylpropyl)diethylenetriamine (DETAPTMS), 5,5'-dithiobis(2-nitrobenzoic acid) (DTNB, Ellman's reagent), tris(2-carboxyethyl)phosphine hydrochloride (TCEP, 0.5 M in water), sodium cyanoborohydride, single-walled carbon nanotubes (CNT, 0.83 nm average diameter, 1 μm median length), oligonucleotides, G418 disulfate salt solution (50 mg/mL in water), doxorubicin (DOX), and Cell Counting Kit-8 (CCK-8) were purchased from Sigma-Aldrich. Cyclohexane, 1-hexanol, hydrogen fluoride (HF), and glutaradehyde (50% in water) were from VWR. Fluorescamine and (3-mercaptopropyl) trimethoxysilane (MPTMS) were purchased from Alfa Aesar. T4 DNA ligase (400,000 units/ml), phi29 DNA polymerase (10,000 units/ml), restriction enzymes (BstEII-HF and SexAI), and deoxynucleotide (dNTP) solution mix (10 mM for each nucleotide) were from New England Biolabs. Sulfo-cyanine 5 NHS-ester was from Lumiprobe. Triton X-100, 4',6-diamidino-2-phenylindole (DAPI), and glycine were from AppliChem. Trypsin/EDTA solution (0.25%/0.02%) and fetal calf serum (FCS) were from Biochrom. Eagle's minimum essential medium (EMEM), Dulbecco's modified Eagle's medium (DMEM), and L-glutamine were from Gibco Laboratories. Fluorescein-12-dUTP solution (FITC-dUTP, 1 mM), penicillin/streptomycin, ethidium bromide (EtBr, 10 mg/mL in water), SYBR™ Gold Nucleic Acid Gel Stain (10,000× Concentrate in water), Alexa Fluor 488 phalloidin, and Dulbecco's phosphate buffered saline (DPBS) for cell experiments were from Thermo Fisher Scientific. Paraformaldehyde (PFA, 16% aqueous solution) was from Polysciences. 1,2-dioleoyl-3-trimethylammonium-propane (DOTAP) (chloride salt) and 1,2-dimyristoyl-sn-glycero-3-phosphoethanolamine-N-(lissamine rhodamine B sulfonyl) (Rh-PE) (ammonium salt) were from Avanti Polar Lipids. Mineral oil was from Carl Roth. All chemicals were used as received without further purification.

*Absorbance and fluorescence spectroscopy:* Absorption spectra were measured by an Agilent Cary 100 UV-Vis spectrophotometer or a BioTek Synergy microplate reader. The latter was used for the measurement of fluorescence spectra as well.

*Transmission electron microscopy (TEM), scanning transmission electron microscopy (STEM), energy dispersive X-ray spectroscopy (EDS) analysis:* Particle size and morphology of SiNP were investigated by FEI Titan$^3$ 80-300 electron microscope (FEI Company) at an accelerating voltage of 200 kV and the obtained images were analyzed by ImageJ (http://www.rsb.info.nih.gov/ij/download.html). Further, STEM and EDS analysis were performed at a voltage of 200 kV, equipped with a high-angle annular dark-field (HAADF) detector (Fischione Company) and a high solid-angle silicon drift detector system (FEI Company). The samples were prepared by placing a drop of the sample solution on 200-mesh carbon-coated Cu grids (Plano GmbH) and air-dried at room temperature.

*Dynamic light scattering (DLS) measurements:* The kinetic hydrodynamic size of SiNP during RCA reaction was studied by DLS (Malvern Zetasizer Nano ZSP) coupled with a standard 633 nm laser at 30 °C.

*Scanning electron microscopy (SEM) analysis:* After lyophilization, the hydrogels were coated with 4 nm platinum using ion beam deposition and their morphology was characterized by using a QUANTA 650-FEG scanning electron microscope from FEI Company, with an accelerating voltage of 5-10 kV.

*Raman analysis:* Raman analysis was performed using a Senterra Raman microscope (Bruker Optics, Ettlingen, Germany) equipped with a 532 nm laser operated at 5 mW output power. An Olympus MPLAN 100x objective, NA 0.9 (Olympus, Tokyo, Japan) was used for visualization of the samples, focusing of the excitation beam, and collimation of backscattered light as well. The spot size on the sample was 5 μm ∅. The measurement time was 15 s with three coadditions (3 × 5 s) for each spot. Samples were prepared by transferring a piece of hydrogel (~ 5 μL) onto a flat gold-coated Si wafer and air-drying before measurements.

*Multiple particle tracking (MPT) method:* The mechanical property of hydrogels was investigated *via* Multiple Particle Tracking (MPT) method. MPT experiments were performed using an inverted fluorescence microscope (Axio Observer D1, Zeiss), equipped with a Fluar 100x, N.A. 1.3, oil-immersion lens combined with a 1× optovar magnification changer. The Brownian motion of green fluorescent polystyrene microspheres of 0.2, 0.5, and 1.0 μm diameter (Bangs Laboratories) used as tracer particles were tracked. Before measurement, the tracer particles were trapped inside hydrogels by mixing of tracer particles with initial RCA reaction mixture. Images of these fluorescent beads were recorded onto a personal computer *via* a sCMOS camera Zyla X (Andor Technology: 21.8 mm diagonal sCMOS Sensor size, 2160 × 2160 square pixels). Displacements of particle centers were monitored in an 127 × 127 μm field of view, at a rate of 50 frames/s. Movies of the fluctuating microspheres were analyzed by a custom MPT routine incorporated into the software Image Processing System (Visiometrics iPS) and a self-written Matlab program (A. Kowalczyk, C. Oelschlaeger, N. Willenbacher, Polymer 2015, 58, 170) based on the widely used Crocker and Grier tracking algorithm.

*Atomic force microscopy (AFM) imaging:* The morphology of DNA-dispersed CNT (CNT-P) was observed by atomic force microscopy (AFM). Briefly, the sample was diluted up to 25 fold in TEMg (20 mM Tris base, 1 mM EDTA, 12.5 mM MgCl2, pH 7.4). Then, 5 μl solution was deposited on a cleaved mica surface (Plano GmbH) and adsorbed for 3 min at room temperature. After addition of 10 μl TAEMg (40 mM Tris, 20 mM acetic acid, 2 mM EDTA, 12.5 mM Mg acetate, pH 8.0), the sample was scanned with sharpened pyramidal tips (SNL-10 tips 0.35 N/m, Bruker) in Tapping Mode with a MultiModeTM 8 microscope (Bruker) equipped with a Nanoscope V controller.

*Electrophoresis:* For both polyacrylamide gel electrophoresis (PAGE) and agarose gel electrophoresis tests, the samples were prepared at required temperature for certain duration to complete the reaction. The raw products without purification were loaded onto a 6% native polyacrylamide gel (1× TAE-$Mg^{2+}$) and run under a voltage of 120 V for 45 min then stained with SYBR Gold or EtBr. Likewise, the raw products without purification were loaded onto a 2.5% agarose gel (1× TAE-$Mg^{2+}$) and run under a voltage of 120 V for 45 min then stained with SYBR Gold or 20 min then stained with EtBr.

DNA sequences:

| | |
|---|---|
| H1 | 5'-GAT CGC GAT CCT GGC TCC TGT GAT TGT GCT CTA GAC ATC GCT AGA GCA CAA TCA CAG G-3' |
| H2 | 5'-CTA GAG CAC AAT CAC AGG AGC CAG TTT TCC TGT GAT TGT GCT CTA GCG ATG T-3' |
| alh | 5'-[AmC12]-TTT TTT TTT TTT TTT TTT TTC ATC TCA GTC TAG GAT TCG GCG TG-3' |
| h1 | 5'-AGT CTA GGA TTC GGC GTG ACG ACT TTC ACG CCG AAT CCT AGA CTG AGA TG-3' |

(continued)

| | |
|---|---|
| h2-I | 5'-ACA TCG CTA GAG CAC AAT CAC AGG TTA GTC GTC ACG CCG AAT CCT AGA CTT TCA TCT CAG TCT AGG ATT CGG CGT G-3' |
| I | ACA TCG CTA GAG CAC AAT CAC AGG |
| aP1 (primer for SiNP modification) | 5'-[AmC12]-TTT TTT TTT TTT TCT AAC TGC TGC GCC GCC GGG AAA ATA CTG TAC GGT TAG A-3' |
| P2 (primer for CNT modification) | 5'- TTT TTT TTT TTT TTT TTT TTT TTT TTT TTT TTT TTT TTT TTC TAA CTG CTG CGC CGC CGG GAA AAT ACT GTA CGG TTA GA-3' |
| Sgc8 | 5'-ATC TAA CTG CTG CGC CGC CGG GAA AAT ACT GTA CGG TTA GA-3' |
| T (template for RCA) | 5'-[Phos]TTC CCG GCG GCG CAG CAG TTA GAT GCT GCT GCA GCG ATA CGC GTA TCG CTA TGG GTA ACC GTA CGG TTA CCC GCA GCA GCA TCT AAC CGT ACA GTA TT-3' |
| Th (template for simultaneous RCA and HCR) | 5'-[Phos]TTCCCGGCGGCGCAGCAGTTAGATGCTGCTGCAGC GAACATCGCTAGAGCACAATCACAGGTACGATATGCCGCAG CAGCATCTAACCGTACAGTATT-3' |
| Forward primer (primer for PCR) | 5'-CGT ACG ATA TGC CAT AGC GAT ACG CGG CGG TTT GCG TAT TGG GCG CTC TTC C-3' |
| Reverse primer (primer for PCR) | 5'-CTC TCC CCG CGC GTT GGC CG-3' |
| pcDNA-EXP40-mKate (plasmid for PCR) | 5'-GACGGATCGGGAGATCTCCCGATCCCCTATGGTGCACTCT CAGTACAATCTGCTCTGATGCCGCATAGTTAAGCCAGTATCT GCTCCCTGCTTGTGTGTTGGAGGTCGCTGAGTAGTGCGCGA GCAAAATTTAAGCTACAACAAGGCAAGGCTTGACCGACAATT GCATGAAGAATCTGCTTAGGGTTAGGCGTTTTGCGCTGCTTC GCGATGTACGGGCCAGATATACGCGTTGACATTGATTATTGA CTAGTTATTAATAGTAATCAATTACGGGGTCATTAGTTCATAG CCCATATATGGAGTTCCGCGTTACATAACTTACGGTAAATGG CCCGCCTGGCTGACCGCCCAACGACCCCCGCCCATTGACGT CAATAATGACGTATGTTCCCATAGTAACGCCAATAGGGACTT |

(continued)

| | |
|---|---|
| | TCCATTGACGTCAATGGGTGGAGTATTTACGGTAAACTGCCC ACTTGGCAGTACATCAAGTGTATCATATGCCAAGTACGCCCC CTATTGACGTCAATACGGTAAATGGCCCGCCTGGCATTATGC CCAGTACATGACCTTATGGGACTTTCCTACTTGGCAGTACAT CTACGTATTAGTCATCGCTATTACCATGGTGATGCGGTTTTG GCAGTACATCAATGGGCGTGGATAGCGGTTTGACTCACGGG GATTTCCAAGTCTCCACCCCATTGACGTCAATGGGAGTTTGT TTTGGCACCAAAATCACGGGACTTTCCAAAATGTCGTAACAA CTCCGCCCCATTGACGCAAATGGGCGGTAGGCGTGTACGGT GGGAGGTCTATATAAGCAGAGCTCTCTGGCTAACTAGAGAAC CCACTGCTTACTGGCTTATCGAAATTAATACGACTCACTATAG GGAGACCCAAGCTGGCTAGTTAAGCTATCAACAAGTTTGTAC AAAAAGCAGGCTCTAAGGAGGATAGAACCATGGTGTCTAAG GGCGAAGAGCTGATTAAGGAGAACATGCACATGAAGCTGTA CATGGAGGGCACCGTGAACAACCACCACTTCAAGTGCACAT CCGAGGGCGAAGGCAAGCCCTACGAGGGCACCCAGACCAT GAGAATCAAGGTGGTCGAGGGCGGCCCTCTCCCCTTCGCCT TCGACATCCTGGCTACCAGCTTCATGTACGGCAGCAAAACCT TCATCAACCACACCCAGGCATCCCCGACTTCTTTAAGCAGTC CTTCCCTGAGGGCTTCACATGGGAGAGAGTCACCACATACG AAGACGGGGGCGTGCTGACCGCTACCCAGGACACCAGCCT CCAGGACGGCTGCCTCTCTACAACGTCAAGATCAGAGGGGT GAACTTCCCATCCAACGGCCCTGTGATGCAGAAGAAAACACT CGGCTGGGAGGCCTCCACCGAGATGCTGTACCCCGCTGAC GGCGGCCTGGAAGGCAGAAGCGACATGGCCCTGAAGCTCG TGGGCGGGGGCCACCTGATCTGCAACTTGAAGACCACATAC AGATCCAAGAAACCCGCTAAGAACCTCAAGATGCCCGGCGT CTACTATGTGGACAGAAGACTGGAAAGAATCAAGGAGGCCG ACAAAGAGACCTACGTCGAGCAGCACGAGGTGGCTGTGGCC AGATACTGCGACCTCCCTAGCAAACTGGGGCACAAACTTAAT TACCCAGCTTTCTTGTACAAAGTGGTTGATCTAGAGGGCCCG CGGTTCGAAGGTAAGCCTATCCCTAACCCTCTCCTCGGTCTC GATTCTACGCGTACCGGTCATCATCACCATCACCATTGAGTT TAAACCCGCTGATCAGCCTCGACTGTGCCTTCTAGTTGCCAG CCATCTGTTGTTTGCCCCTCCCCCGTGCCTTCCTTGACCCTG |

(continued)

| | |
|---|---|
| | GAAGGTGCCACTCCCACTGTCCTTCCTAATAAAATGAGGAAA<br>TTGCATCGCATTGTCTGAGTAGGTGTCATTCTATTCTGGGGG<br>GTGGGGTGGGGCAGGACAGCAAGGGGGAGGATTGGGAAGA<br>CAATAGCAGGCATGCTGGGGATGCGGTGGGCTCTATGGCTT<br>CTGAGGCGGAAAGAACCAGCTGGGGCTCTAGGGGGTATCC<br>CCACGCGCCCTGTAGCGGCGCATTAAGCGCGGCGGGTGTG<br>GTGGTTACGCGCAGCGTGACCGCTACACTTGCCAGCGCCCT<br>AGCGCCCGCTCCTTTCGCTTTCTTCCCTTCCTTTCTCGCCAC<br>GTTCGCCGGCTTTCCCCGTCAAGCTCTAAATCGGGGGCTCC<br>CTTTAGGGTTCCGATTTAGTGCTTTACGGCACCTCGACCCCA<br>AAAAACTTGATTAGGGTGATGGTTCACGTAGTGGGCCATCGC<br>CCTGATAGACGGTTTTTCGCCCTTTGACGTTGGAGTCCACGT<br>TCTTTAATAGTGGACTCTTGTTCCAAACTGGAACAACACTCAA<br>CCCTATCTCGGTCTATTCTTTTGATTTATAAGGGATTTTGCCG<br>ATTTCGGCCTATTGGTTAAAAAATGAGCTGATTTAACAAAAAT<br>TTAACGCGAATTAATTCTGTGGAATGTGTGTCAGTTAGGGTG<br>TGGAAAGTCCCCAGGCTCCCCAGCAGGCAGAAGTATGCAAA<br>GCATGCATCTCAATTAGTCAGCAACCAGGTGTGGAAAGTCCC<br>CAGGCTCCCCAGCAGGCAGAAGTATGCAAAGCATGCATCTC<br>AATTAGTCAGCAACCATAGTCCCGCCCCTAACTCCGCCCATC<br>CCGCCCCTAACTCCGCCCAGTTCCGCCCATTCTCCGCCCCA<br>TGGCTGACTAATTTTTTTTATTTATGCAGAGGCCGAGGCCGC<br>CTCTGCCTCTGAGCTATTCCAGAAGTAGTGAGGAGGCTTTTT<br>TGGAGGCCTAGGCTTTTGCAAAAAGCTCCCGGGAGCTTGTA<br>TATCCATTTTCGGATCTGATCAAGAGACAGGATGAGGATCGT<br>TTCGCATGATTGAACAAGATGGATTGCACGCAGGTTCTCCGG<br>CCGCTTGGGTGGAGAGGCTATTCGGCTATGACTGGGCACAA<br>CAGACAATCGGCTGCTCTGATGCCGCCGTGTTCCGGCTGTC<br>AGCGCAGGGGCGCCCGGTTCTTTTTGTCAAGACCGACCTGT<br>CCGGTGCCCTGAATGAACTGCAGGACGAGGCAGCGCGGCT<br>ATCGTGGCTGGCCACGACGGGCGTTCCTTGCGCAGCTGTGC<br>TCGACGTTGTCACTGAAGCGGGAAGGGACTGGCTGCTATTG<br>GGCGAAGTGCCGGGGCAGGATCTCCTGTCATCTCACCTTGC<br>TCCTGCCGAGAAAGTATCCATCATGGCTGATGCAATGCGGC<br>GGCTGCATACGCTTGATCCGGCTACCTGCCCATTCGACCAC |

(continued)

| | |
|---|---|
| | CAAGCGAAACATCGCATCGAGCGAGCACGTACTCGGATGGA AGCCGGTCTTGTCGATCAGGATGATCTGGACGAAGAGCATC AGGGGCTCGCGCCAGCCGAACTGTTCGCCAGGCTCAAGGC GCGCATGCCCGACGGCGAGGATCTCGTCGTGACCCATGGC GATGCCTGCTTGCCGAATATCATGGTGGAAAATGGCCGCTTT TCTGGATTCATCGACTGTGGCCGGCTGGGTGTGGCGGACCG CTATCAGGACATAGCGTTGGCTACCCGTGATATTGCTGAAGA GCTTGGCGGCGAATGGGCTGACCGCTTCCTCGTGCTTTACG GTATCGCCGCTCCCGATTCGCAGCGCATCGCCTTCTATCGC CTTCTTGACGAGTTCTTCTGAGCGGGCTCTGGGGTTCGCGA AATGACCGACCAAGCGACGCCCAACCTGCCATCACGAGATT TCGATTCCACCGCCGCCTTCTATGAAAGGTTGGGCTTCGGAA TCGTTTTCCGGGACGCCGGCTGGATGATCCTCCAGCGCGGG GATCTCATGCTGGAGTTCTTCGCCCACCCCAACTTGTTTATT GCAGCTTATAATGGTTACAAATAAAGCAATAGCATCACAAATT TCACAAATAAAGCATTTTTTTCACTGCATTCTAGTTGTGGTTT GTCCAAACTCATCAATGTATCTTATCATGTCTGTATACCGTCG ACCTCTAGCTAGAGCTTGGCGTAATCATGGTCATAGCTGTTT CCTGTGTGAAATTGTTATCCGCTCACAATTCCACACAACATAC GAGCCGGAAGCATAAAGTGTAAAGCCTGGGGTGCCTAATGA GTGAGCTAACTCACATTAATTGCGTTGCGCTCACTGCCCGCT TTCCAGTCGGGAAACCTGTCGTGCCAGCTGCATTAATGAATC GGCCAACGCGCGGGGAGAGGCGGTTTGCGTATTGGGCGCT CTTCCGCTTCCTCGCTCACTGACTCGCTGCGCTCGGTCGTTC GGCTGCGGCGAGCGGTATCAGCTCACTCAAAGGCGGTAATA CGGTTATCCACAGAATCAGGGGATAACGCAGGAAAGAACAT GTGAGCAAAAGGCCAGCAAAAGGCCAGGAACCGTAAAAAGG CCGCGTTGCTGGCGTTTTTCCATAGGCTCCGCCCCCCTGAC GAGCATCACAAAAATCGACGCTCAAGTCAGAGGTGGCGAAA CCCGACAGGACTATAAAGATACCAGGCGTTTCCCCCTGGAA GCTCCCTCGTGCGCTCTCCTGTTCCGACCCTGCCGCTTACC GGATACCTGTCCGCCTTTCTCCCTTCGGGAAGCGTGGCGCT TTCTCATAGCTCACGCTGTAGGTATCTCAGTTCGGTGTAGGT CGTTCGCTCCAAGCTGGGCTGTGTGCACGAACCCCCCGTTC AGCCCGACCGCTGCGCCTTATCCGGTAACTATCGTCTTGAGT |

(continued)

| | |
|---|---|
| | CCAACCCGGTAAGACACGACTTATCGCCACTGGCAGCAGCC ACTGGTAACAGGATTAGCAGAGCGAGGTATGTAGGCGGTGC TACAGAGTTCTTGAAGTGGTGGCCTAACTACGGCTACACTAG AAGAACAGTATTTGGTATCTGCGCTCTGCTGAAGCCAGTTAC CTTCGGAAAAAGAGTTGGTAGCTCTTGATCCGGCAAACAAAC CACCGCTGGTAGCGGTGGTTTTTTTGTTTGCAAGCAGCAGAT TACGCGCAGAAAAAAAGGATCTCAAGAAGATCCTTTGATCTT TTCTACGGGGTCTGACGCTCAGTGGAACGAAAACTCACGTTA AGGGATTTTGGTCATGAGATTATCAAAAAGGATCTTCACCTA GATCCTTTTAAATTAAAAATGAAGTTTTAAATCAATCTAAAGTA TATATGAGTAAACTTGGTCTGACAGTTACCAATGCTTAATCAG TGAGGCACCTATCTCAGCGATCTGTCTATTTCGTTCATCCATA GTTGCCTGACTCCCCGTCGTGTAGATAACTACGATACGGGA GGGCTTACCATCTGGCCCCAGTGCTGCAATGATACCGCGAG ACCCACGCTCACCGGCTCCAGATTTATCAGCAATAAACCAGC CAGCCGGAAGGGCCGAGCGCAGAAGTGGTCCTGCAACTTTA TCCGCCTCCATCCAGTCTATTAATTGTTGCCGGGAAGCTAGA GTAAGTAGTTCGCCAGTTAATAGTTTGCGCAACGTTGTTGCC ATTGCTACAGGCATCGTGGTGTCACGCTCGTCGTTTGGTATG GCTTCATTCAGCTCCGGTTCCCAACGATCAAGGCGAGTTACA TGATCCCCCATGTTGTGCAAAAAAGCGGTTAGCTCCTTCGGT CCTCCGATCGTTGTCAGAAGTAAGTTGGCCGCAGTGTTATCA CTCATGGTTATGGCAGCACTGCATAATTCTCTTACTGTCATG CCATCCGTAAGATGCTTTTCTGTGACTGGTGAGTACTCAACC AAGTCATTCTGAGAATAGTGTATGCGGCGACCGAGTTGCTCT TGCCCGGCGTCAATACGGGATAATACCGCGCCACATAGCAG AACTTTAAAAGTGCTCATCATTGGAAAACGTTCTTCGGGGCG AAAACTCTCAAGGATCTTACCGCTGTTGAGATCCAGTTCGAT GTAACCCACTCGTGCACCCAACTGATCTTCAGCATCTTTTAC TTTCACCAGCGTTTCTGGGTGAGCAAAAACAGGAAGGCAAAA TGCCGCAAAAAAGGGAATAAGGGCGACACGGAAATGTTGAA TACTCATACTCTTCCTTTTTCAATATTATTGAAGCATTTATCAG GGTTATTGTCTCATGAGCGGATACATATTTGAATGTATTTAGA AAAATAAACAAATAGGGGTTCCGCGCACATTTCCCCGAAAAG TGCCACCTGACGTC-3’ |
| Note: "[AmC12]" and [Phos] represent "Amino-Modifier C12" and "Phosphate", respectively. | |

1. Rolling circle amplification (RCA)-mediated synthesis of silica nanoparticles (SiNP)-DNA hydrogel

1.1 Synthesis of ssDNA (P1)-modified SiNP

Multifunctional SiNP having an average particle size of 80 nm with amino, thiol and phosphonate groups were firstly synthesized. An embodiment thereof is shown in Figure 6. In particular, cyclohexane (38 mL), 1-hexanol (9 mL) and triton X-100 (9 mL) was mixed rigorously in a 250 mL round-bottom glass bottles. Then, double distilled water (2 mL) was added to the mixture to form stable reverse micelles. TEOS (500 μL) was introduced in the mixture after 10 min, and the hydrolysis and condensation of TEOS was catalyzed by adding ammonia solution (28-30 %, 500 μL). The mixture was stirred at room temperature for 24 h. Then an additional TEOS (250 μL) was added to the mixture and stirred for another 30 min, and subsequently THPMP (200 μL) and DETAPTMS (50 μL) were added to modify the surface of the nanoparticles with negatively charged phosphonate and amino groups. The mixture was allowed to react for 24 h, and MPTMS (30 μL) was added to modify the nanoparticle's surface with thiol groups. The mixture was stirred at room temperature for an additional 3 h. The micelles were broken with acetone, and the obtained nanoparticles were centrifuged and washed at least 5 times with absolute ethanol, and finally dispersed in PBS buffer (pH 7.4, 10 mM) with a concentration of 10 mg/mL. Then, TCEP (0.5 M solution, 8.0 μL) was added to 1 mL of SiNP PBS solution to cleave any disulfide bonds on the particle surface, followed by reaction with mPEG-mal (50 mg/mL, 10 μL) to fabricate PEGlyated SiNP. Afterwards, amino-modified ssDNA P1 (aP1) was chemically immobilized on the particle surface *via* glutaraldehyde chemistry. Specifically, PEGlyated SiNP (10 mg/mL, 1.0 mL) in PBS buffer were mixed with glutaraldehyde (50 % in water, 250 μL), and the mixture was stirred at room temperature for 1 h. The resulting nanoparticles were washed 3 times with PBS buffer to remove unreacted glutaraldehyde. The obtained nanoparticles were dispersed in PBS buffer (1.0 mL), and then aP1 (100 μM, 50 μL) was added to the particle suspension and incubated for 2 h at room temperature. Following, glycine (0.4 M, 1.0 mL) was added in the mixture to block unreacted aldehyde groups, followed by adding sodium cyanoborohydride (60 mM, 400 μL) to reduce the imide bond into a stable C-N bond.

1.2 Synthesis particle-primer-template complexes

After obtaining (P1)-modified SiNP (SiNP-P), ssDNA (T) was phosphorylated at its 5' end and used to hybridize with P1 primer on the particle. In brief, the template T (10 μM, 30 μL) and 10x T4 DNA ligation buffer (10 μL) were added into 60 μL (10 mg/mL) SiNP suspension and the mixture was incubated for 3 h at 25 °C. After adding 2.5 μL T4 DNA ligase, the mixture was further incubated for more than 3 h at 25 °C to ligate the nicked ends of the template, leading to the formation of particle-primer-template complexes.

1.3 Rolling circle amplification of primer on particle

The rolling circular amplification solution was firstly prepared through mixing dNTPs (10 mM, 10 μL), 10× BSA (5 μL), 10× phi29 DNA polymerase buffer (5 μL), and phi29 DNA polymerase (10,000 U/ml, 5 μL), then 51.25 μL of the above-prepared particle-primer-template complexes was subjected to RCA reaction solution for 48 h at 30 °C. After purification of the raw product by carefully replacing surrounding solution of hydrogel with distilled water for 5-7 times, SiNP-DNA hydrogel was collected and stored at 4 °C.

2. Hybridization chain reaction (HCR)-mediated synthesis of SiNP-DNA hydrogel

2.1 Synthesis of ssDNA (Ih)-modified SiNP

ssDNA (Ih)-modified SiNP (SiNP-Ih) were synthesized with the similar protocol in part 1.1 using amine-modified Ih (aIh) instead of using aP1.

2.2 Annealing procedure for constructing hairpin structures

To prepare DNA hairpins for HCR, H1, H2, h1 and h2-I were purchased, dissolved and annealed. To prepare DNA hairpins for the linear HCR process, the purchased strands (H1, H2, h1 or h2-I) were kept at 95 °C for five minutes and then quickly quenched with ice to 4 °C. A 1X TAE-$Mg^{2+}$ buffer (40 mM Tris, 20 mM acetic acid, 2 mM EDTA, 12.5 mM $Mg^{2+}$, pH=8.0) was used to dissolve H1 and H2 strands. A 5X SSC buffer (750 mM sodium chloride, 75 mM sodium citrate, pH=7.0) was used for h1 and h2-I strands.

2.3 HCR on SiNP-Ih

h1 (500 μM, 20 μL) and h2-I (500 μM, 20 μL) were successively added into 20 μL SiNP-Ih suspension (10 mg/mL) and the reaction mixture was incubated overnight under room temperature. After purification by centrifugation/resuspension in distilled water for 3-5 times, the excess h1 and h2-I were removed and the obtained particle (SiNP-I) suspension was concentrated to 10 μL. After the first hybridization chain reaction (HCR) process, the initiator on particle was changed from Ih to I and the number of initiator on particle was amplified. The formed SiNP-I can further trigger the second HCR process of H1 and H2. Thus, 10 μL SiNP-I suspension was thoroughly mixed with 10 μL H1 (2.25 mM) and 10 μL H2 (2.25 mM) solutions in 250-μL tube and the mixture was exposed to air and evaporated overnight under room temperature. Afterwards, 10 μL distilled water was replenished and the mixture solution was incubated for at least 36 h to obtain a SiNP-DNA hydrogel.

3. Formation of SiNP-DNA hydrogel in DOTAP droplet

3.1 Preparation of RCA reaction solution and DOTAP-containing mineral oil

The protocol for preparation of the RCA reaction solution can be found in part 1.1 as described above. The only difference is that SiNP used here were doped with Cy5 by co-condensation and co-hydrolysis of tetraethoxysilane (TEOS) and Cy5-modified (3-aminopropyl)trimethoxysilane (APTMS-Cy5) during particle synthesis. Notably, APTES-Cy5 was synthesized by co-incubation of sulfo-Cy5-NHS ester (1.28 μmol, in 1 mL DMSO) with APTMS at a molar ratio of 1:10 at room temperature overnight.

1,2-dioleoyl-3-trimethylammonium-propane (DOTAP)-containing mineral oil was prepared by ultrasonication of DOTAP in mineral oil in an ice bath for 90 min. For visualization of the droplet membrane, 1,2-dimyristoyl-sn-glycero-3-phosphoethanolamine-N-(lissamine rhodamine B sulfonyl) was also used during the preparation of DOTAP-containing mineral oil. The concentration of DOTAP and Rh-PE in mineral oil are 4 mM and 4 μM, respectively.

3.2 RCA reaction inside the droplet

Mineral oil containing DOTAP and Rh-PE (continuous phase) and RCA reaction solution were pumped into a microfluidic droplet generator made of poly(methyl methacrylate) (PMMA) which uses a flow-focusing junction at the speeds of 20 μL/min (continuous phase) and 1 μL/min (reaction solution), respectively. The formed droplets were collected into 96-well plate and incubated at 30 °C.

To monitor the gelation process in droplets over time, fluorescence recovery after photobleaching (FRAP) experiment was performed using confocal laser scanning fluorescence microscopy (CLSM) following the protocol with slight modification. The excitation wavelengths are 561 nm for rhodamine B and 633 nm for Cy5, respectively. When bleaching both dyes, the parameters were set as: bleach radius 25 μm to 35 μm, bleach time 1 min, and laser power 100%. Then, the images of droplets were acquired before and after bleaching at defined time points.

4. Hybridization chain reaction (HCR)-reinforced SiNP-DNA hydrogel preparation

First of all, hairpin structures of H1 and H2 were formed by the annealing procedure, described in part 2.2. Herein, a 1X phi29 DNA polymerase buffer (5 mM Tris-HCI, 1 mM MgCI2, 1 mM, $(NH_4)_2SO_4$, 0.4 mM DTT (pH 7.5 @ 25°C) was used to dissolve the DNA strands. To trigger the HCR during RCA reaction on SiNP-P, ssDNA Th was used as the template and 4 μL H1 (2.25 mM) and 4 μL H2 (2.25 mM) were mixed with RCA reaction mixture.

5. Formation of a composite material comprising SiNP, CNT and DNA hydrogel

CNT-P was prepared by adding CNT (0.6 mg) and P2 (100 μM, 172 μL) into a NaCl solution (0.14 μM, 428 μL), and ultrasonication of the mixture in an ice bath for 90 min. After that, the unsuspended CNT were precipitated by centrifugation (40 min, 16000g). The suspension of CNT-P was collected and further purified by filtration until no free DNA could be detected.

The processes of synthesis of SiNP/CNT-DNA composite hydrogels SCx are similar to those of RCA-mediated synthesis of SiNP-DNA hydrogel S100. The difference is that both CNT-P and SiNP-P at different SiNP/CNT mass ratios were used in the one-pot reaction. Following by the same processes, a mixture of P1 primer-modified SiNP and P2 primer-modified CNT was used to synthesize nanoparticle/nanotube-primer-template complexes, which was used for the synthesis of the composite material SiNP/CNT-DNA hydrogel. Various composite materials that contained only SiNP or CNT (denoted as S100 or C100, respectively) or ternary composite materials containing both SiNP and CNT, denoted as SCx, where x gives the mass ratio of SiNP-P:CNT-P, were prepared as indicated in Table 2 below.

6. Cell culture

Human $MCF7_{eGFP}$ breast cancer cells stably transfected to express the EGF receptor fused to enhanced green fluorescent protein (eGFP-EGFR) were obtained from Max-Planck Institute for Molecular Physiology (Dortmund). The cells were cultured in $MCF7_{eGFP}$ medium, composed by EMEM, with addition of 1% penicillin/streptomycin, 10% FCS and 0.6% G418 disulfate salt solution at 37 °C in a 5% $CO_2$ environment. The cells were washed twice with DPBS (-/-) (without calcium and magnesium) and treated with trypsin by adding 500 μL 0.25% Trypsin solution in PBS-EDTA (PBS with 0.02% EDTA) for 3 minutes. The trypsin activity was blocked by adding 9.5 mL of fresh $MCF7_{eGFP}$ medium. The cell concentration of the resulting suspension was determined by hemocytometer analysis. MCF7 cells without transfection and HeLa cervical cancer cells from ATCC were also used in this research and cultivated without G418 disulfate salt solution in EMEM with 1% penicillin/streptomycin, 10% FCS. REF52 cells (a rat fibroblast line) were maintained in DMEM supplemented with 1% penicillin/streptomycin, 10% FCS, and 2 mM L-glutamine.

7. Bioimaging and drug delivery

Aptamers were inserted into the elongated DNA after RCA reaction by using the rationally designed DNA templates and primers. To this end, the long DNA chain-capped SiNP (S100apt) with aptamers were formed with periodic DNA containing multiple copies of aptamers. To evaluate the feasibility of S100apt for bioimaging application, HeLa cells which express PTK7 receptors were seeded with an initial density of $1.2x10^4$ cells/well into a 4-well μ-slide (Ibidi) and allowed to adhere for 12 h, followed by the addition of fresh medium that contained S100apt (400 μg/mL SiNP-P) for 6 h of incubation. Prior to fluorescence microscope imaging, the cells were washed with DPBS for three times, fixed by addition of a PFA solution (4%, in DPBS) for 10 min, permeabilized with a triton X-100 solution (0.1%, in DPBS) for 2 min, treated with CAS-Block for 20 min to suppress unspecific binding of the cell staining reagents, stained with Alexa Fluor 488 phalloidin solution (160 nM, in DPBS) for 20 min to visualize the actin filament, and ultimately counterstained with DAPI-solution (1.4 μM, in DPBS) for 10 min to locate the cell nucleus. DPBS was used to wash the cells for 2-3 times during each step.

The capability of $S100_{48h}$ for loading anthracycline drug doxorubicine (DOX) was investigated by stoichiometric quenching of DOX in DNA. Briefly, DOX (50 μM) was incubated with $S100_{48h}$ (0-75 μL) dispersed in 200 μL DPBS at room temperature for 24 h, followed by centrifugation at 10,000 rpm for 15 min. The free DOX in the supernatant was isolated and quantified by measuring the emission of DOX at an excitation of 480 nm. The DOX loading amount into $S100_{48h}$ was calculated by subtracting DOX amount determined in supernatant from initially used DOX amount. After that, $1.2\times10^4$ HeLa cells/well were seeded into a 4-well μ-slide (Ibidi) and allowed to adhere for 12 h, followed by the addition of fresh medium that contained free DOX or $S100_{48h}$-DOX (5 μM DOX equivalent) for 2 h of incubation.

8. Controlled cell migration inside the SCx

Cell behavior inside the various SCx materials synthesized in a 25 μL RCA reaction was analyzed by confocal microscope (LSM 880, Carl Zeiss) after seeding cells on the SCx. To this end, SCx with different SiNP-P/CNT-P concentration ratios were prepared in 96-well plates with standard tissue culture (STC) cover glass as bottom (MoBiTec GmbH). After careful washing of the formed hydrogels with DPBS (3-5 times) and medium (3-5 times), $6\times10^3$ $MCF7_{eGFP}$ cells per well with 200 μL medium were seeded on the top of hydrogels and allowed to settle down for 2 h. The cell behavior was monitored by confocal fluorescence microscopy using both Z stack and time lapse modes. After cultivation of cells for 24 h, a cell proliferation assay was carried using the CCK-8 assay as described above.

Since the cells were restrained from vertical migration inside the hydrogel matrix within the SCx when the CNT concentration was higher than 160 μg/mL (e.g., as in SC25), it is possible to release them from the hydrogels. To this end, template T was firstly used to prepare the mixture of nanoparticle/nanotube-primer-template complexes. Then, SC25 containing BstEII restriction sites was synthesized by RCA reaction and used for restraining and controlled release of cells after digestion of hydrogel using BstEII-HF with a final concentration of 2 U/mL. The cell behavior was monitored by confocal fluorescence microscopy using both Z stack and time lapse modes.

9. Cell proliferation on the top of the condensed SCx

The various DNA-crosslinked SiNP and CNT composite materials were investigated for their ability to serve as coating that support cell spreading. To this end, various hydrogels of different SiNP/CNT concentration ratios were synthesized in 75 μL RCA reaction mixture in 96-plate wells with cover glass as bottom, purified by replacing the initial buffer with distilled water, and condensed by drying in vacuum (Eppendorf Concentrator plus, 20 mbar) to form the coatings. Afterwards, $6\times10^3$ $MCF7_{eGFP}$ or REF52 cells per well with 200 μL medium were seeded on the top of hydrogels and visualized by fluorescence microscopy. After cultivation of the cells for 48 h, a cell proliferation assay was carried using the aforementioned CCK-8 assay. To compare the adhesion strength of cells to different hydrogel surfaces, MCF7 or REF52 cells were incubated on hydrogel surfaces for 1 h and then washed with DPBS. The number of the remained cells was quantified by CCK-8 assay (see Figure 31).

To compare the attraction of cells towards either the hydrogel surface or the STC surface, a sample of SC50 synthesized in 75 μL RCA reaction mixture was deposited and dried on petri dish with STC surface (Ibidi), thereby generating a patch of SC50 on the petri dish surface. Following, 1 mL medium containing $1.2\times10^4$ $MCF7_{eGFP}$ cells was transferred into a petri dish so that the hydrogel was fully covered by medium. After another 24 h of cultivation, adhered $MCF7_{eGFP}$ cells were visualized by fluorescence microscopy. Alternatively, for cell migration studies, 20 μL medium containing $1.2\times10^4$ $MCF7_{eGFP}$ cells were transferred into the petri dish to form a droplet that was about 0.8 cm apart from the hydrogel. After 4 h incubation, the suspension cells were carefully removed by replacing the remaining medium with 1 mL fresh medium such that the entire surface of the petri dish was covered with medium. This enabled the adherent cells to migrate from the STC-surface to the SC50 surface where they proliferated (see Figure 30).

10. Data

10.1 RCA-based SiNP-DNA hydrogel

To monitor the gelation process, dynamic light scattering (DLS) was employed to record the hydrodynamic size of SiNP in the RCA reaction mixture (Figure 8). To simplify the description, we abbreviate SiNP-DNA hydrogel as S100xh, where x represents the RCA time. The particle-primer-template without T4 DNA ligase treatment displayed no obvious change in the hydrodynamic size. In sharp contrast, the hydrodynamic size of SiNP increased over time when the nicked end of template T on particle surface was ligated. The SiNP-DNA hydrogel (S100) formed after 48-hour incubation at 30 °C leading to a hydrogel that could be stretched like a rubber band (inset in Figure 8).

The structural feature of this hydrogel was characterized by scanning electron microscopy (SEM) (Figure 7). The dehydrated sample from RCA of primer on SiNP for 48 h showed a 3D crosslinked morphology with DNA linker filaments between particles. The DNA polymer which serves as a linker between two particles could be visualized in the magnified SEM image.

Multiple particle tracking (MPT) microrheology was then applied to quantitatively characterize the mechanical strength by introducing and tracking 0.5 μm or 1.0 μm polystyrene (PS) tracer particles in RCA reaction solution. In MPT experiments, the thermally driven motion of inert tracers that are distributed within a sample is monitored. The resulting particle trajectories are transformed into mean square displacement (MSD) traces and the viscoelastic moduli G' and G" can be extracted from the average MSD. The rheological data confirm that the $S100_{48h}$ (over the entire frequency range) reveals typical characteristics of hydrogels, as G' is constantly higher than G" and G' at the frequency of 10 rad $s^{-1}$ is termed as $G_0$ that was further calculated to be 3.2 Pa (Figure 9).

10.2 HCR-based SiNP-DNA hydrogel

To monitor the gelation process, DLS was employed to record the hydrodynamic size of SiNP in the HCR reaction mixture (Figure 10). It is evident that SiNP-Ih without initiator I displays no obvious change in the hydrodynamic size. In sharp contrast, the hydrodynamic size of SiNP-I increases over time when H1 and H2 were triggered by I on the particle. Notably, the SiNP-DNA hydrogel is solid after 48-hour incubation at room temperature (inset in Figure 10).

The structural feature of this HCR-reinforced hydrogel was characterized by scanning electron microscopy (SEM) (Figure 11). The dehydrated sample from HCR of H1 and H2 on SiNP-I for 48 h showed a 3D crosslinked morphology. The magnified SEM image shows DNA polymer covering on particle surface and crosslinked particles.

Bulk rheological tests were then applied to quantitatively characterize the mechanical strength of this hydrogel generated by HCR of H1 and H2 on SiNP-I. Rheology datum confirms that the SiNP-DNA hydrogel is a true hydrogel, as G' is constantly higher than G" in the frequency range of 0.05 and 100 rad $s^{-1}$ (see Figure 12).

10.3 RCA-mediated polymerization of SiNP-DNA hydrogel inside DOTAP-stabilized droplets

As shown in Figure 13a, Cy5-doped SiNP accumulated in the interior surface of the droplet like a shell. This occurs due to electrostatic interactions between the positively charged DOTAP membrane and negatively charged DNA-modified SiNP. After incubation of the RCA reaction mixture inside the droplet at 30 °C for 48 h, FRAP experiments were performed. It was found that the bleached central angel of Cy5-doped SiNP only slowly recovered (from 33.65° to 30.40°) within 2 h after photobleaching (Figure 13b,c), indicating that the fluidity of the SiNP shell was low. This indicates that the SiNP shell was in the gel phase due to RCA-induced polymerization.

10.4 Composite material comprising SiNP, CNT and DNA hydrogel

The structural feature of the composite material comprising SiNP/CNT-DNA hydrogel was characterized by SEM (Figure 14). The dehydrated sample obtained by RCA of primer-modified SiNP and CNT for 48 h showed the typical 3D crosslinked morphology.

10.5 Mechanical properties of the hydrogels

**Table 1:** MPT microrheology characterization of SiNP/CNT-DNA hydrogels synthesized *via* RCA reaction as compared to HCR-reinforced SiNP-DNA hydrogels.

| Entry | Name[a] | [SINP-P][b,e] | [CNT-P][b,e] | [H1] or [H2] | $G_0$ (Pa)[c] |
|---|---|---|---|---|---|
| 1 | S100 | 4000 | 0 | 0 | 3.2 ± 0.4 |
| 2 | SC50 | 4000 | 80 | 0 | 4.8 ± 0.2 |
| 3 | SC25 | 4000 | 160 | 0 | 8.5 ± 0.7 |
| 4 | SC12.5 | 4000 | 320 | 0 | 14.1 ± 1.0 |
| 5 | S100-HCR[d)] | 4000 | 0 | 120 μM H1, 120 μM H2 | 7.3 ± 0.7 |

[a)] Acronyms in Entry 1-4 represent the mass ratio of SiNP-P:CNT-P subjected to RCA polymerization
[b)] Given in μg/mL
[c)] Determined by multiple particle tracking (MPT) after 48 h RCA reaction time. Note that the storage modulus (elastic modulus $G_0$) is substantially higher for ternary composite materials (e.g., SC12.5) than for binary composite material S100.
[d)] S100-HCR represents the S100 reinforced by HCR method.
[e)] DNA concentration may vary depending from the concentration and amount of nucleotides and the duration of RCA. This is well known for conventional DNA polymers. The maximum DNA concentration corresponds to the amount of nucleotides used in RCA or primers used in HCR.

Indicated in Table 2 are various composite materials that contained only SiNP or CNT (in the following denoted as S100 or C100, respectively) or ternary composite materials containing both SiNP and CNT, denoted as SCx, where x gives the mass ratio of SiNP-P:CNT-P. S100 hydrogels containing 4 mg/mL SiNP had excellent properties for cell experiments after 48 h of polymerization, whereas a maximum of 320 μg/mL CNT could be incorporated into pure C100 hydrogels. Higher amounts of CNT led to precipitation from the reaction mixture and hardly reproducible formation of brittle materials with no apparent viscosity. Hence, all ternary composites contained relative fractions of these maximal amounts. In the following, short term acronyms of the ternary materials SCx are as indicated in Table 2.

**Table 2:** Overview on binary and ternary SiNP/CNT-DNA hydrogel composite materials.

| Entry | Name[a,d] | [SiNP-P][b,d] | [CNT-P][b,d] | $G_0$ (Pa)[c] |
|---|---|---|---|---|
| 1 | S100 | 4000 | - | 3.2 ± 0.4 |
| 2 | SC50 | 4000 | 80 | 4.8 ± 0.2 |
| 3 | SC25 | 4000 | 160 | 8.5 ± 0.7 |
| 4 | SC12.5 | 4000 | 320 | 14.1 ± 1.0 |
| 5 | SC6.25 | 2000 | 320 | 10.3 ± 1.0 |
| 6 | SC2.5 | 800 | 320 | 3.1 ± 0.2 |

(continued)

| Entry | Name[a,d] | [SiNP-P][b,d] | [CNT-P][b,d] | $G_0$ (Pa)[c] |
|---|---|---|---|---|
| 7 | C100 | - | 320 | 2.8 ± 0.2 |

a) Acronyms represent the mass ratio of SiNP-P:CNT-P subjected to RCA polymerization
b) Given in μg/mL
c) Determined by multiple particle tracking (MPT) after 48 h RCA reaction time. Note that the storage modulus (elastic modulus $G_0$) is substantially higher for ternary composite materials (e.g., SC12.5) than for binary composite materials S100 or C100.
d) DNA concentration may vary depending from the concentration and amount of nucleotides and the duration of RCA. This is well known for conventional DNA polymers. The maximum DNA concentration corresponds to the amount of nucleotides used in RCA.

Dynamic light scattering (DLS) was used to monitor the RCA-induced gelation process of S100 (SiNP-DNA hydrogel) (Figure 15d). It is clearly evident that the hydrodynamic size of SiNP-P (SiNP modified with ssDNA primers) increased over time when the linear template T was circularized and ligated on the particle surface. For control, SiNP-P without ligase treatment displayed negligible changes in the hydrodynamic size. Naked-eye-inspection clearly indicated that the materials revealed typical viscoelastic properties of hydrogels after reaction times of >12 h (Figure 2, right side).

To quantitatively characterize the mechanical properties of the composite materials, multiple particle tracking microrheology (MPT) is used. In this technique, the Brownian motion of polymer-embedded fluorescent tracer particles is quantitatively tracked by time-resolved microscopy. The computed trajectories are directly correlated with the rheological properties through the Generalized Stokes-Einstein Relation (Oelschlaeger, C., Bossler, F. & Willenbacher, N. Synthesis, structural and micromechanical properties of 3D hyaluronic acid-based cryogel scaffolds. Biomacromolecules 17, 580-589 (2016)). The particle trajectories are transformed into mean square displacement (MSD) traces that allow for calculation of the linear viscoelastic moduli, the shear-storage modulus (G') and the shear-loss modulus (G"). It is clearly evident from the trajectory maps of tracer particles in Figure 15e,f that the particles can freely diffuse in solutions of SiNP-P but are restrained by the network of polymerized S100. Calculation of the viscosity $\eta_{MPT}$ and plateau moduli of G' ($G_0$) yielded 1.0 mPa·s and 3.2 Pa, respectively, for S100 after 48 h of polymerization, and these values increased with increasing RCA time. Likewise, the viscoelastic properties of C100 gels depended on the CNT-P (CNT modified with P2 primers) concentration as indicated from MPT analyses. Notably, the binary nanocomposites differ in mesh size (0.2-0.5 and 0.5-1.0 μm, for S100 and C100, respectively), as estimated from MPT measurements using differently sized tracer particles experiments. The mechanical properties of the various ternary SCx materials were significantly changed in dependency of the mass ratio of SiNP-P:CNT-P (Tables 1 and 2). In fact, $G_0$ constantly increased from 4.8 Pa to 14.1 Pa with increasing amounts of CNT (SC50, SC25, and SC12.5), indicating that, indeed, the CNT content increases the composite material's mechanical stiffness. Likewise, increased concentrations of SiNP enhanced the mechanical stiffness of the ternary SC hydrogels (3.1 Pa for SC2.5, 10.3 Pa for SC6.25, and 14.1 Pa for SC12.5) when the concentration of CNT was constant. Importantly, the $G_0$ values of some SCx materials were substantially higher than those expected from totaling the values of pure S100 (3.2 Pa) and C100 (2.8 Pa). This result clearly demonstrates that the two differently shaped nanoparticles are synergistically determining the mechanical stiffness and viscoelastic properties of the composite materials.

The structural features of the hydrogels were characterized by transmission electron microscopy (TEM, Figure 16) and scanning electron microscopy (SEM, Figure 7). S100 gel has an amorphous morphology with a distinctive hierarchical ultrastructure, which clearly reveals the DNA-coated particles that are connected by DNA filaments. Direct comparison of TEM/SEM images of SiNP-P and S100 (Figures 16 and 17) and analysis by scanning transmission electron microscopy (STEM) coupled with energy dispersive X-ray spectroscopy (EDS, Figure 15) confirmed the presence of a dense layer of polymerized DNA polymer that coats and crosslinks the particles in the composite material. Further, HCR-reinforced SiNP-DNA hydrogel displays a larger $G_0$ when compared with SiNP-DNA hydrogel S100 prepared by RCA.

Altogether, these results indicate that the mechanical properties of SiNP-DNA hydrogels can be enhanced by the formation of composite hydrogels or combination of HCR method.

10.6 Modular variations of the design

Several studies were carried out to exemplify the modularity of composite material design: Firstly, dye-modified SiNP can be conveniently introduced to enable fluorescence tracking of the materials. This feature is important, for instance, for studies of cell uptake and degradation of hydrogels. It is demonstrated in Figure 18 that fluorescent properties can be incorporated into the composite material by either using dye-encoded SiNP, obtained through co-hydrolysis of TEOS and Cy5 dye-conjugated silane (Leidner, A. et al. Biopebbles: DNA - Functionalized Core-Shell Silica Nanospheres for Cellular Uptake and Cell Guidance Studies. Adv. Funct. Mater. 0, DOI 10.1002/adfm.201707572 (2018)). Else, fluorescent properties can be generated by RCA-based incorporation of chemically modified deoxynucleotides bearing fluorescent dyes, such as fluorescein (FITC)-modified dUTP. The resulting materials revealed the expected optical properties (Figure 18) while their morphology remained unchanged (Figure 19).

Secondly, variations of the DNA backbone can be implemented. For example, the introduction of restriction sites in conventional DNA polymers enables the design of responsive materials that can be post-synthetically modified with enzymes (Lilienthal, S., Shpilt, Z., Wang, F., Orbach, R. & Willner, I. Programmed DNAzyme-Triggered Dissolution of DNA-Based Hydrogels: Means for Controlled Release of Biocatalysts and for the Activation of Enzyme Cascades. ACS Appl. Mater. Interfaces 7, 8923-8931 (2015)). To introduce restriction sites into the single-stranded backbone of the composite materials, stem-loop structures of appropriate sequence were encoded in the RCA template (T), thereby enabling efficient enzymatic degradation and concomitant gel-to-sol transition of the composite material (Figure 20). This feature is of great utility, for instance, to achieve controllable release of polymers or polymer-embedded cells, which is required for therapeutic applications and dynamic cell manipulations. To illustrate the applicability of the aforementioned strategies for studies on the trackable drug delivery to cells, a fluorescent S100 material was engineered that contained a structured backbone bearing the Sgc8 aptamer, which specifically binds to the PTK7 receptor (Shangguan, D. et al. Aptamers evolved from live cells as effective molecular probes for cancer study. Proc. Natl. Acad. Sci. U. S. A. 103, 11838-11843 (2006)), as well as double-stranded GG- or GC-rich drug association sites, which can be used for enrichment of intercalating drugs, such as anthracycline doxorubicin (DOX) (Zhu, G. et al. Self-Assembled Aptamer - Based Drug Carriers for Bispecific Cytotoxicity to Cancer Cells. Chem. - Asian J. 7, 1630-1636 (2012)). Studies with PTK7-positive HeLa cells showed that uptake of fluorescent S100 was dependent on the amount of aptamer units incorporated into the material (Figure 21). After loading these materials with DOX, their uptake was also clearly dependent on the amounts of aptamer and drug loading moieties incorporated into the DNA backbone (Figure 22). These findings indicate that the composite materials hold the potential for applications as vehicles for the cell-specific delivery of DNA-binding drugs into cancer cells.

10.7 Cytotoxicity Assay

Cell Count Kit-8 (CCK-8, sigma) cell viability assay was employed to evaluate the *in vitro* cytotoxicity of the SiNP/CNT-DNA hydrogels under standard manufacturer's instructions. In brief, MCF-7 cells at a density of $8\times10^3$ cells per well in 200 μL MCF7 medium were seeded into wells of a 96-well plate. After incubation at 37 °C and 5% $CO_2$ for 12 h, the adherent cells were incubated with 200 μL fresh medium containing PBS (control) and 75 μL SiNP-DNA hydrogel (S100), CNT-DNA hydrogel (C100), or SiNP/CNT-DNA hydrogel (SCx) at different SiNP/CNT mass ratios. After another 24 h incubation, 20 μL CCK-8 was added into each well, and the MCF7 cells were then incubated for another 4 h at 37 °C and 5% $CO_2$. Following, the absorbance of each well at 450 nm was recorded using a BioTek Synergy microplate reader. Cell viability was calculated according to equation (1):

$$\text{Cell viability} = \frac{A1-B}{A0-B} \quad (1)$$

where A0 and A1 represent the OD values of CCK-8 in medium containing MCF-7 cells after treatment with PBS, S100, C100, and SCx, respectively. B represents the OD value of CCK-8 in medium. Average numbers and standard deviations (SD) of 5 parallels for each sample were reported.

As shown in Figure 23, all types of hydrogels exhibit negligible cytotoxicity on cells as compared to PBS control. These results indicate that SiNP/CNT-DNA hydrogels possess excellent biocompatibility.

10.8 Cell adhesion, migration and proliferation on ternary SiNP/CNT DNA hydrogel composite materials

Further, the utility of ternary SCx composite materials for *in vitro* cell culture was determined. Using MCF7 breast cancer cells, as described above, it was established that none of the various hydrogels revealed any cytotoxicity (Figure 23). For an in-depth investigation of the composite materials, arrays of the various materials were prepared in 96 well glass bottom plates by adding 75 μL reaction mixtures for RCA synthesis of the materials directly inside the wells (Figure 24a). Polymerization for 48 h led to formation of a hydrogel layer of about 350 μm thickness. These freshly prepared materials were either used directly for cell culture experiments (route A in Figure 24a) or else subjected to drying for 4 h under low pressure at room temperature (route B in Figure 24a), which led to thin films of about 7 μm thickness (corresponding to an about 98% shrinkage upon drying). After seeding of MCF7 cells that constitutively express an eGFP-tagged epidermal growth factor receptor on their cell membrane ($MCF7_{eGFP}$) (Angelin, A. et al. Multiscale Origami Structures as Interface for Cells. Angew Chem Int Ed Engl 54, 15813-15817 (2015)) on top of these freshly prepared or dried hydrogels, cells were cultured for 24 h. Quantitative assessment of cell proliferation with the CCK-8 assay revealed distinctive material-dependent differences in the adhesion and proliferation of living cells (Figure 24b,c).

For the fresh hydrogels, proliferation of cells on S100 and SC50 was slightly lower than on the standard tissue culture (STC) surface lacking the hydrogel whereas the other composite thin films decreased proliferation upon increasing amounts of CNT (Figure 24b). The composition of dried hydrogels induced a similar trend, however, on a substantially different level: Proliferation of cells on S100 and SC50 was significantly higher than on STC, while the other composite thin films still induced proliferation, although with decreasing effectiveness at increasing amounts of CNT (Figure 24c). Microscopy inspection revealed that cells on dried films showed a more pronounced elongated fusiform and flattened morphology than cells adhered on STC surface (Figures 24e, Figure 25, Figure 26). The same, however, even more pronounced results were obtained with rat embryo fibroblast cells REF52 (Figures 27 and 28). Life cell imaging of $MCF7_{eGFP}$ also indicated that cells on SC50 are highly agile in terms of spreading, communication, motility, and proliferation. Control experiments with SiNP-P that were not subjected to RCA polymerization showed that the particles dried into thin layers of cracked particle blocks to which $MCF7_{eGFP}$ cells predominantly adhered at the blocks' edges in a round morphology (Figure 29). The high attractiveness of dried SC50 was also emphasized in experiments, where cells were allowed to adhere to either STC or SC50 surfaces (Figure 30) or to specifically migrate from STC to SC50 surfaces (Figure 24f,g).

To shed light on cellular adhesion strength, MCF7 cells were allowed to adhere for 1 h on the various surfaces, and the samples were then washed thoroughly with buffer to remove weakly adhered cells. There, the adhesion strength steadily increased with increasing amounts of CNT in the composite materials (Figure 31). The observed preferences for distinct composite surfaces is attributed to a combination of nano-roughness, mechanical properties and anionic nature of the surfaces. Tumor cells are known for their preference to grow on rough surfaces. It is known from studies with standard cell culture substrates that highest cell motility and replication occurs at an intermediate adhesion strength, whereas strong adhesion impairs cell motility and proliferation. Therefore, it is concluded that the dried composite material films hold potential as tailorable coating for steering of cell adhesion, proliferation and motility, which is of substantial relevance for biomedical applications, such as medical implants.

In addition, in-depth studies on the interaction of cells with the fresh composite material hydrogels were conducted, that led to a lower proliferation activity as compared to STC substratum (Figure 24b). It was observed that $MCF7_{eGFP}$ cells rapidly transmigrate into the matrix of hydrogels S100 and SC50 with an apparent velocity of about 2 μm/h (Figure 32). In contrast, the cells could not transmigrate but rather dug themselves into the upper layer of materials with higher contents of CNT (e.g., SC25, Figure 32). These observations are in line with the higher CNT concentration and mechanical stiffness of SC25, as compared to SC50 (Table 2).

Since washing did not remove the cells from SC25, it was concluded that this material should be suited for selective capture and enzyme-triggered release of surface-bound cells. Indeed, treatment of the SC25-entrapped cells with a restriction enzyme for 2 h led to a noticeable reduction of the gel's thickness from 45 μm to 15 μm along with the release of adhered cells (Figure 32). Importantly, the released cells were still alive and migrated deeper into the gel matrix to adhere to the underlying STC where they propagated to form small cell populations 10 h after of enzymatic treatment. These results confirm the utility of the composite materials of the present invention for biomedical applications.

10.9 Cell adhesion on SiNP/CNT-DNA hydrogel

SiNP/CNT-DNA hydrogel (75 μL) was deposited on a μ-dish (∅ = 35 mm, standard tissue culture (STC) surface) and dried in vacuum overnight. Afterwards, $1.2\times10^4$ MCF7 cells stably transfected to express the EGF receptor fused to enhanced green fluorescent protein ($MCF7_{eGFP}$ cells) were seeded on the top of the dried hydrogel and incubated for another 24 h. The cell morphology was observed by confocal fluorescence microscope.

It was found that less cells settled down on the untreated μ-dish surface while more cells chose to stay on the hydrogel surface and spread there (right part of Figure 30). These results indicate that the hydrogel of the present invention is more attractive for cells than commercially available culture dish surfaces and can support cell spreading.

11. Microgels

11.1 Preparation of microscale SiNP/CNT-DNA hydrogel composite material

RCA reaction mixture (75 μL) for synthesis of SiNP/CNT-DNA composite microgel was prepared in the same way as described for the synthesis of SiNP/CNT-DNA hydrogel composite material. Herein, both SiNP-P and CNT-P were used with 4 mg/mL SiNP and 80 μg/mL CNT as the final concentrations, respectively. This mass ratio is the same as used for the SC50 composite material described above (Table 2). Subsequently, RCA reaction mixture was transferred into 600 µL mineral oil-surfactant mixture (4.5% (vol/vol) Span 80, 0.4% (vol/vol) Tween 80, 0.05% (vol/vol) Triton X-100), at the same time, stirred at a speed of 1,000 rpm for 30 min using a mechanical stirrer. The formed water-in-oil emulsion was incubated at 30 °C and stirred at a speed of 500 rpm. After another 48 h, microgels were isolated by precipitation in a large excess of isopropanol followed by centrifugation to remove the oil phase. The resultant microgels were thoroughly washed with isopropanol before being dispersed into 75 µL distilled water.

The morphology, size, and fluorescence property of the SiNP/CNT-DNA composite microgel were characterized by fluorescence microscopy (Figure 33). The microgels formed in an irregular shape and displayed strong Cy5 fluorescence. The typical diameter of the microgels was calculated to be about 10 µm in average.

11.2 Cellular uptake of microgels

200 µL REF52 cell suspension ($6\times10^3$ cells in REF52 medium) were placed into a 96-well plate. After incubation at 37 °C and 5% $CO_2$ for 12 h, the adherent cells were incubated with 200 µL fresh medium containing 10 µL of the aforementioned stock suspension of SiNP/CNT-DNA composite microgel. After an additional 6 h incubation, REF52 cells were washed with PBS, counterstained with Alexa Fluor 488 phalloidin and 4',6-diamidino-2-phenylindole (DAPI), and imaged by fluorescence microscope.

It was observed that SiNP/CNT-DNA composite microgels locate in the cytosolic compartment close to the cell nucleus, indicating the efficient cellular internalization of the microgels (Figure 34). Thus, the SiNP/CNT-DNA composite microgels can be used to realize delivery of cargo, such as drugs, proteins, or genes, to the nuclear compartment of eukaryotic cells.

11.3 Cellular uptake of microgel encoded with mKate gene fragments

A gene fragment encoding for the red fluorescent protein "mKate" was incorporated in the microgels. To prepare SiNP/CNT-DNA microgels containing mKate gene fragments, the mKate gene was amplified from plasmid (pcDNA-EXP40-mKate, 6217 base pairs) via PCR and then was mixed with RCA reaction mixture at the concentration of 15 ng/µL. After preparation and purification of the microgels as described above, REF52 cells ($6\times10^3$ cells per well with 200 µL REF52 medium) were seeded into a 96-well plate. After incubation at 37 °C and 5% $CO_2$ for 12 h, the adherent cells were incubated with 200 µL fresh medium containing 10 µL stock suspension of microgel. After an additional 12 h incubation (Day 1) or 6 days, REF52 cells were washed with PBS, counterstained with Alexa Fluor 488 phalloidin and 4',6-diamidino-2-phenylindole (DAPI), and imaged by fluorescence microscope.

While REF52 cells did not express mKate protein after incubation for 12 h, the red fluorescence of mKate was clearly evident in the cytoplasma, indicating the capability of microgel for gene transfection (Figure 35). Thus, the SiNP/CNT-DNA microgels can be used as a carrier to deliver functional cargo into eukaryotic cells.

Applicant: Karlsruher Institut für Technologie
"Composite material comprising DNA hydrogel and silica nanoparticles" Our Ref.: K 6049EU - kl / cwe

**List of Reference Numerals**

1 continuous phase inlet
2 reaction solution inlet
3 outlet

## Claims

1. A composite material, comprising
DNA hydrogel, and
silica nanoparticles, wherein the silica nanoparticles are connected with the DNA hydrogel through DNA strands.

2. The composite material according to claim 1, wherein the silica nanoparticles have an average particle size in the range of from 5 nm to 10 μm.

3. The composite material according to claim 2, wherein the silica nanoparticles have an average particle size in the range of from 60 nm to 100 nm.

4. The composite material according to any one of claims 1 to 3,
further comprising carbon nanotubes embedded in the DNA hydrogel.

5. Use of the composite material according to any one of claims 1 to 4 for cargo delivery to a cell.

6. Use of the composite material according to any one of claims 1 to 4 as surface coating for *in vitro* cell culture.

7. Use of the composite material according to any one of claims 1 to 4 for enzyme-triggered cell release.

8. A microgel comprising the composite material according to any one of claims 1 to 4.

9. The microgel according to claim 8 having an average particle size of from 0.05 to 50 μm.

10. Use of the microgel according to claim 8 or 9 for the delivery of cargo to the cytosolic compartment of cells.

11. Use of the microgel according to claim 10 for gene transfection.

12. A hollow spherical container, wherein the wall of the hollow spherical container comprises at least one layer containing the composite material according to any one of claims 1 to 4.

13. A microreactor comprising
the hollow spherical container according to claim 12 and at least one cell in the space enclosed by the hollow spherical container.

14. Use of the microreactor according to claim 13 for high-throughput screening.

Figure 1

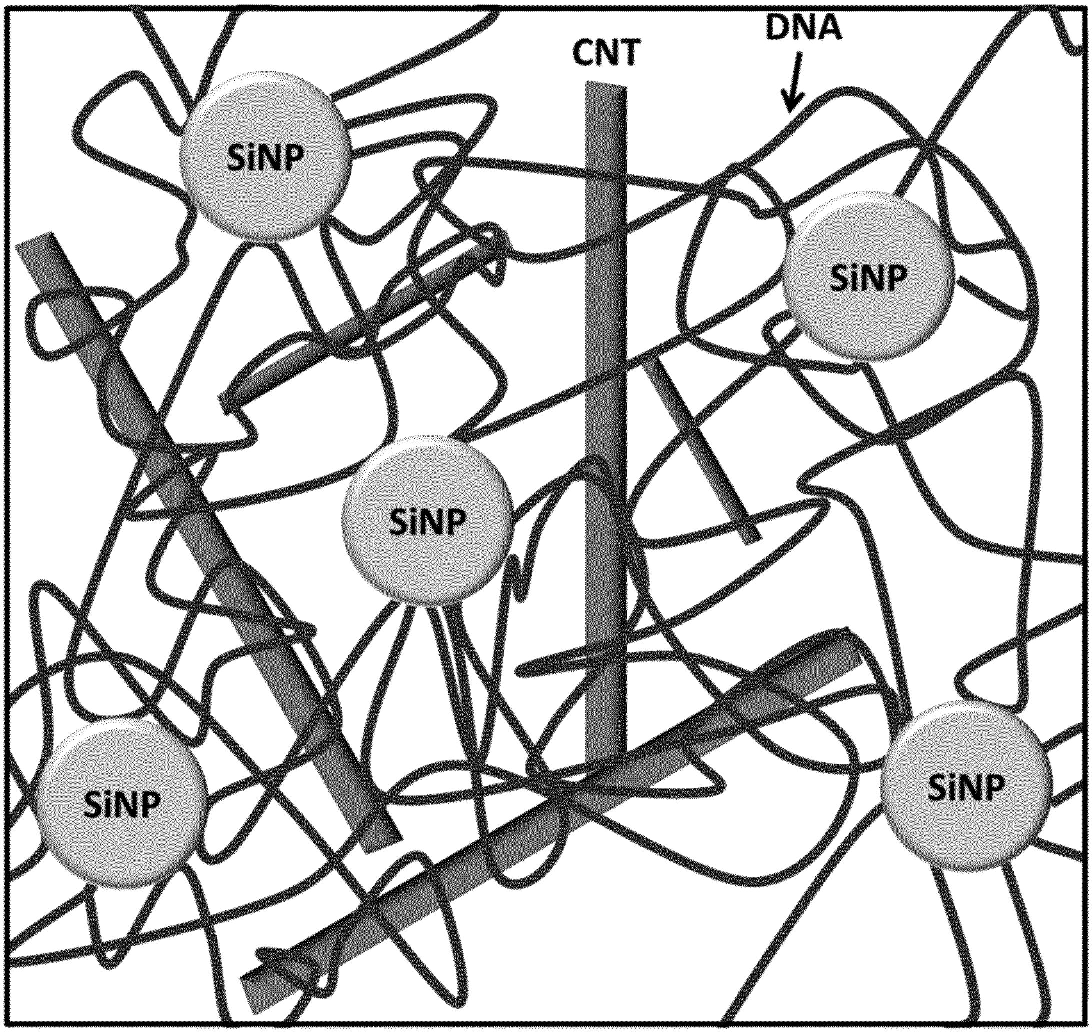

Figure 2

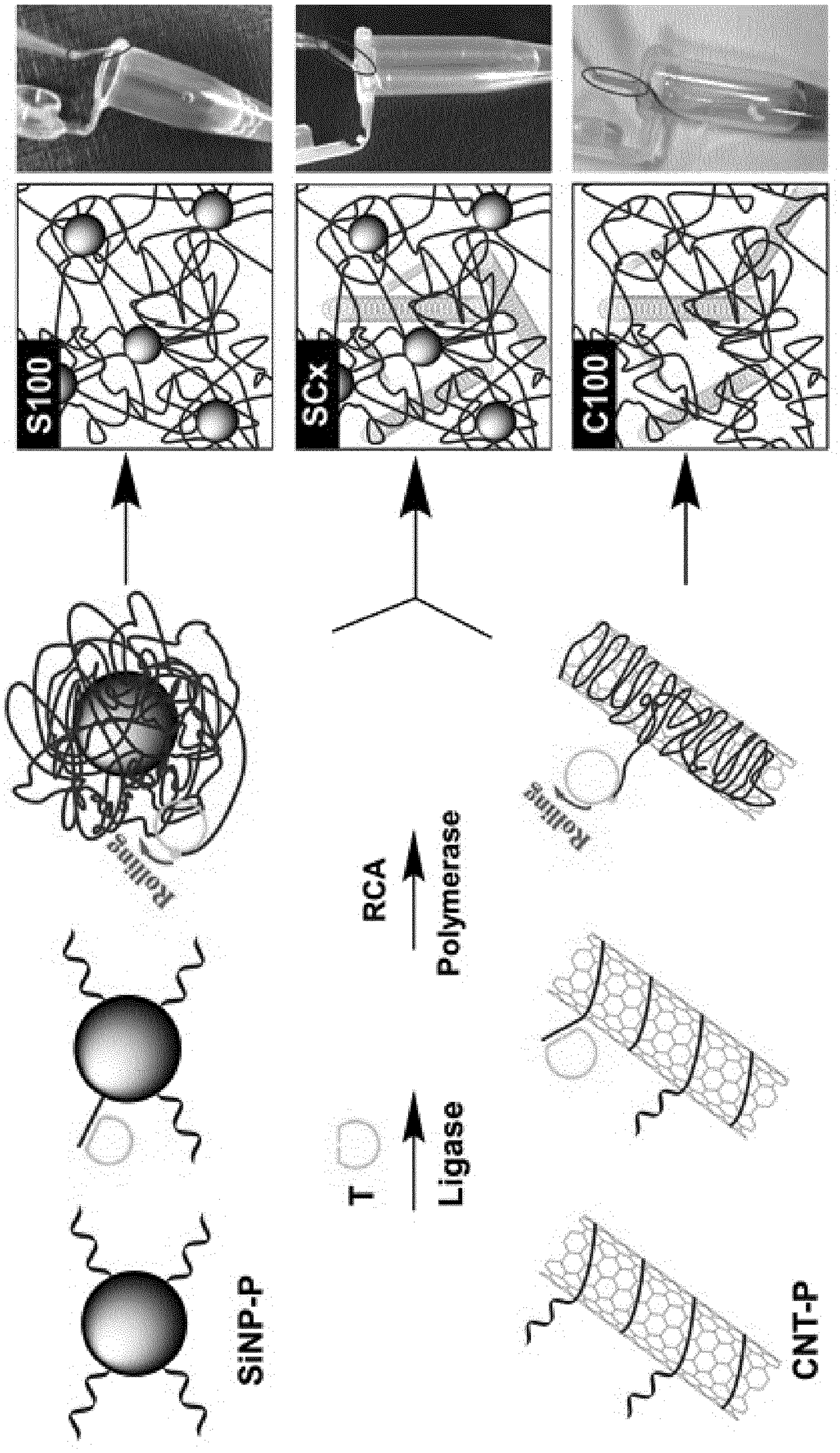
S100
SCx
C100
SiNP-P
CNT-P
T
Ligase
RCA
Polymerase
Rolling
Rolling

Figure 3

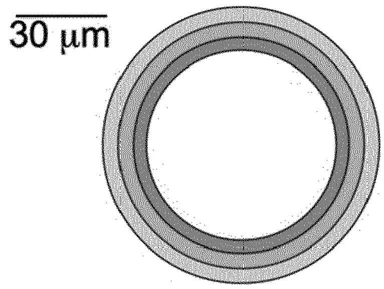


Figure 4

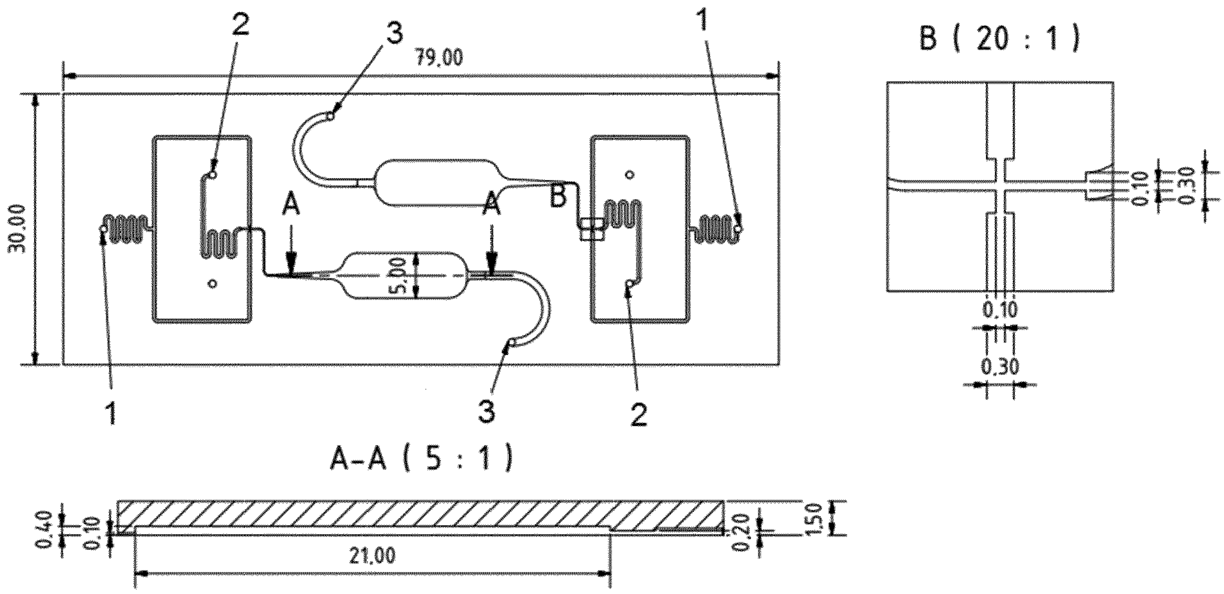

Figure 5

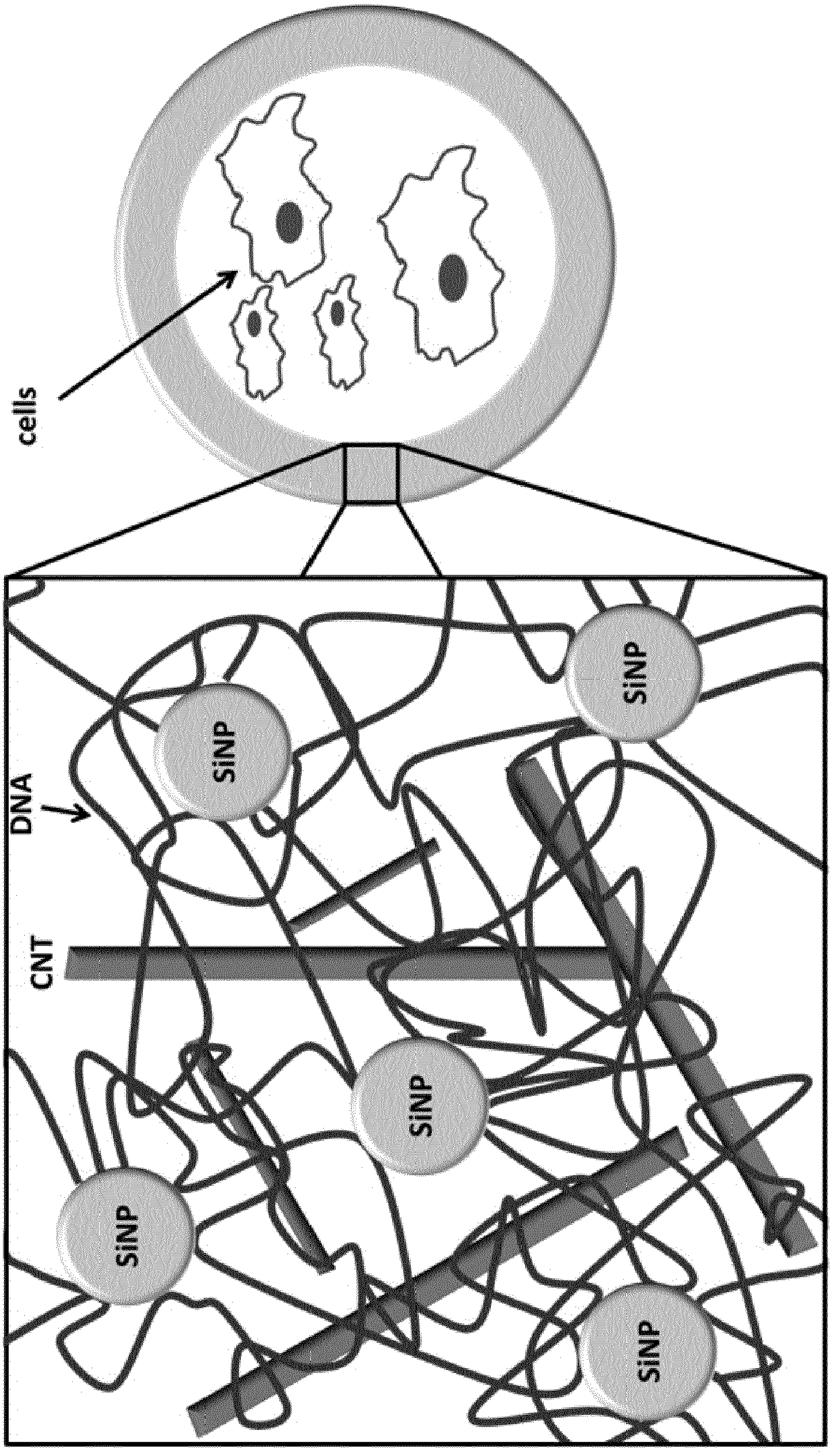

# Figure 6

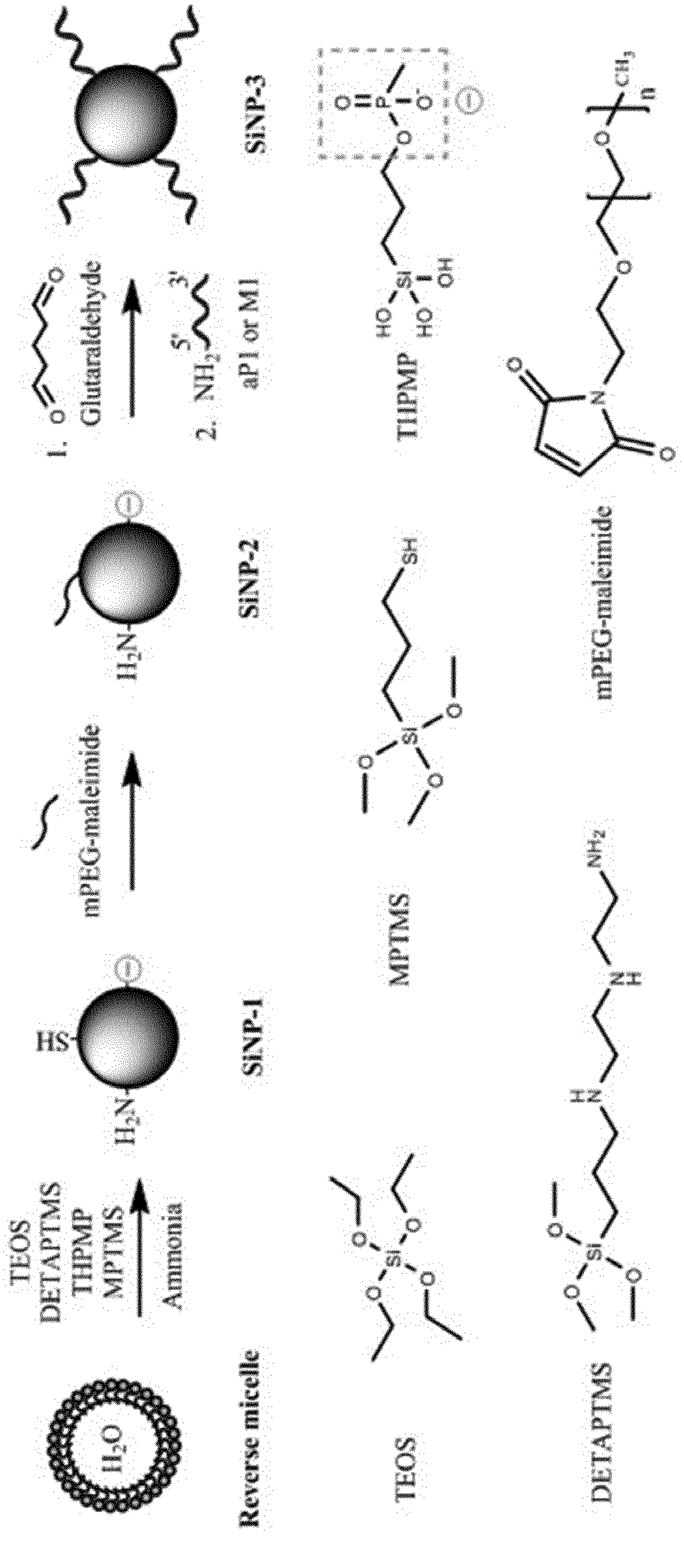
Reverse micelle
TEOS
DETAPTMS
THPMP
MPTMS
Ammonia
SiNP-1
mPEG-maleimide
SiNP-2
1. Glutaraldehyde
2. aP1 or M1
SiNP-3
TEOS
DETAPTMS
MPTMS
THPMP
mPEG-maleimide

## Figure 7

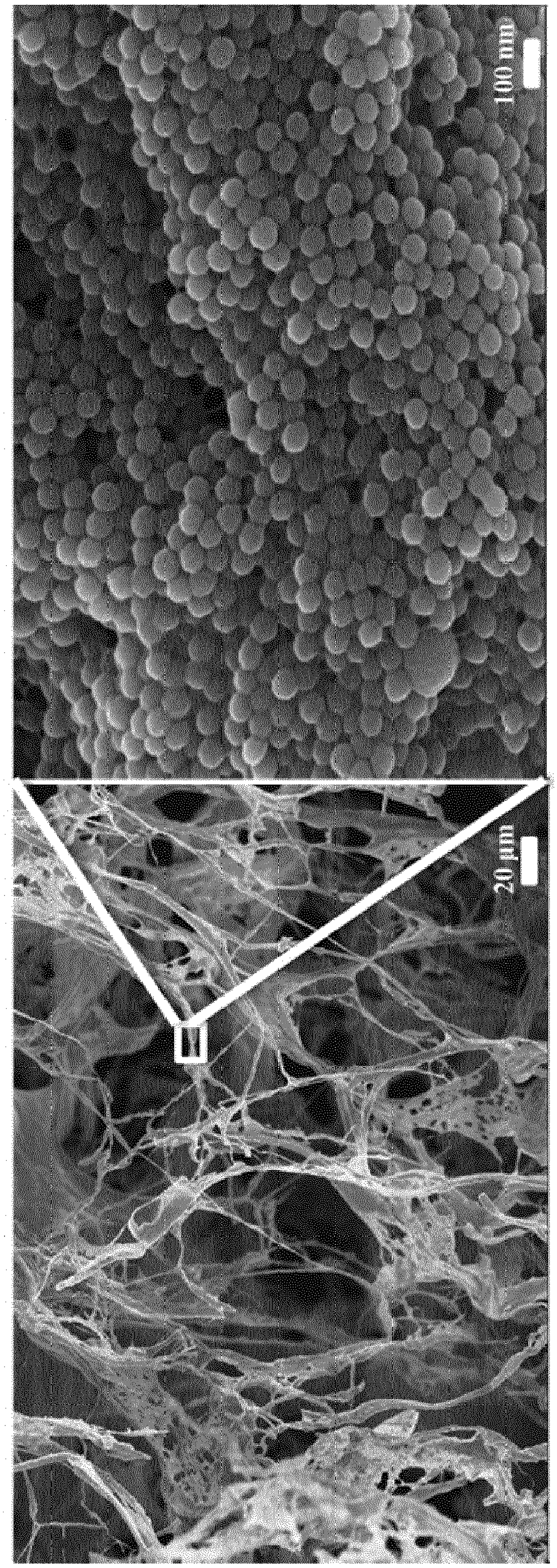

Figure 8

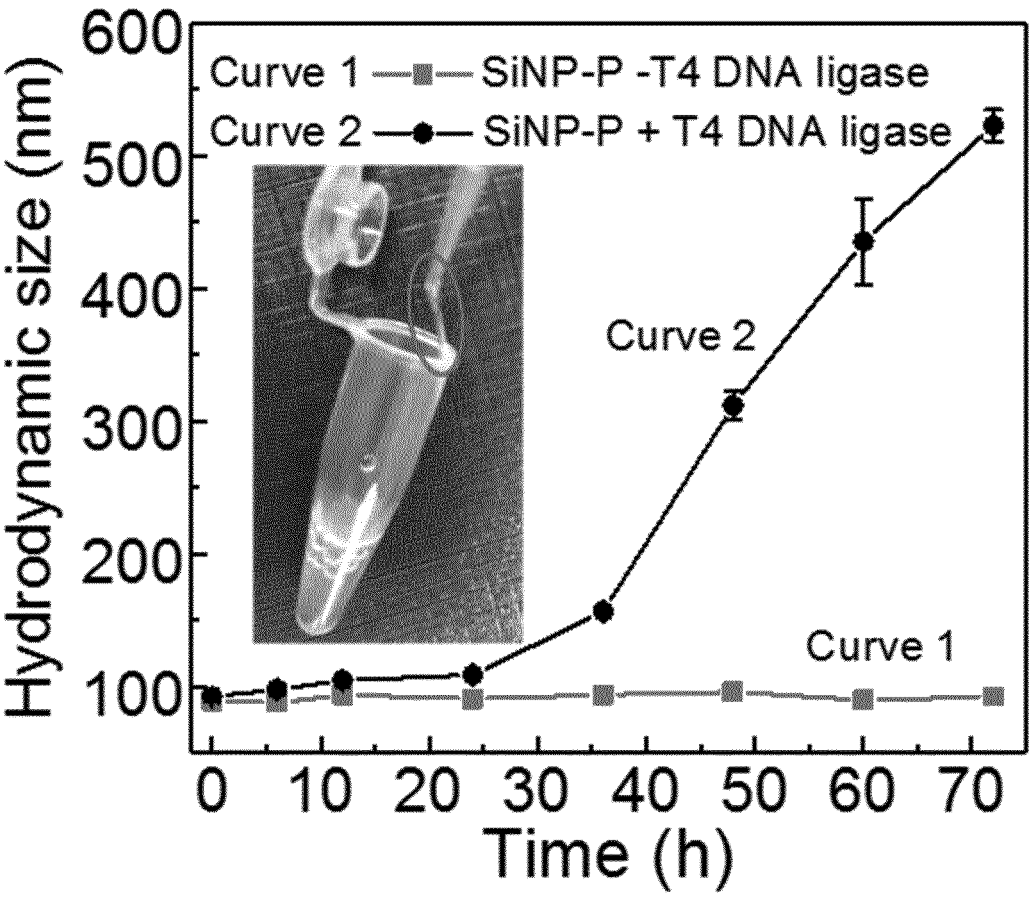
600
500
400
300
200
100
Hydrodynamic size (nm)
Curve 1 SiNP-P -T4 DNA ligase
Curve 2 SiNP-P + T4 DNA ligase
Curve 2
Curve 1
0 10 20 30 40 50 60 70
Time (h)

Figure 9

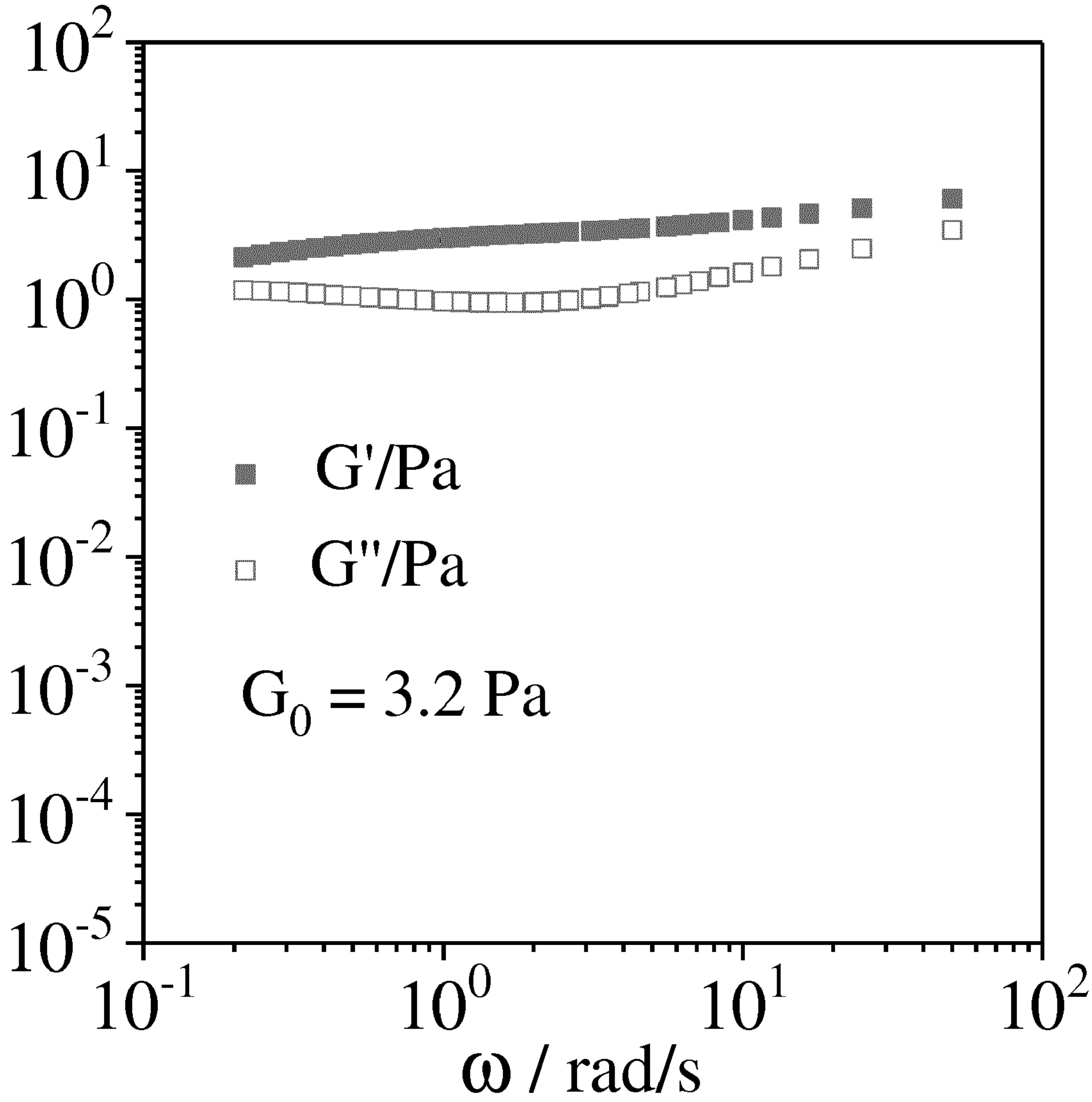
10^2
10^1
10^0
10^-1
10^-2
10^-3
10^-4
10^-5
10^-1
10^0
10^1
10^2
ω / rad/s
G'/Pa
G''/Pa
G0 = 3.2 Pa

Figure 10

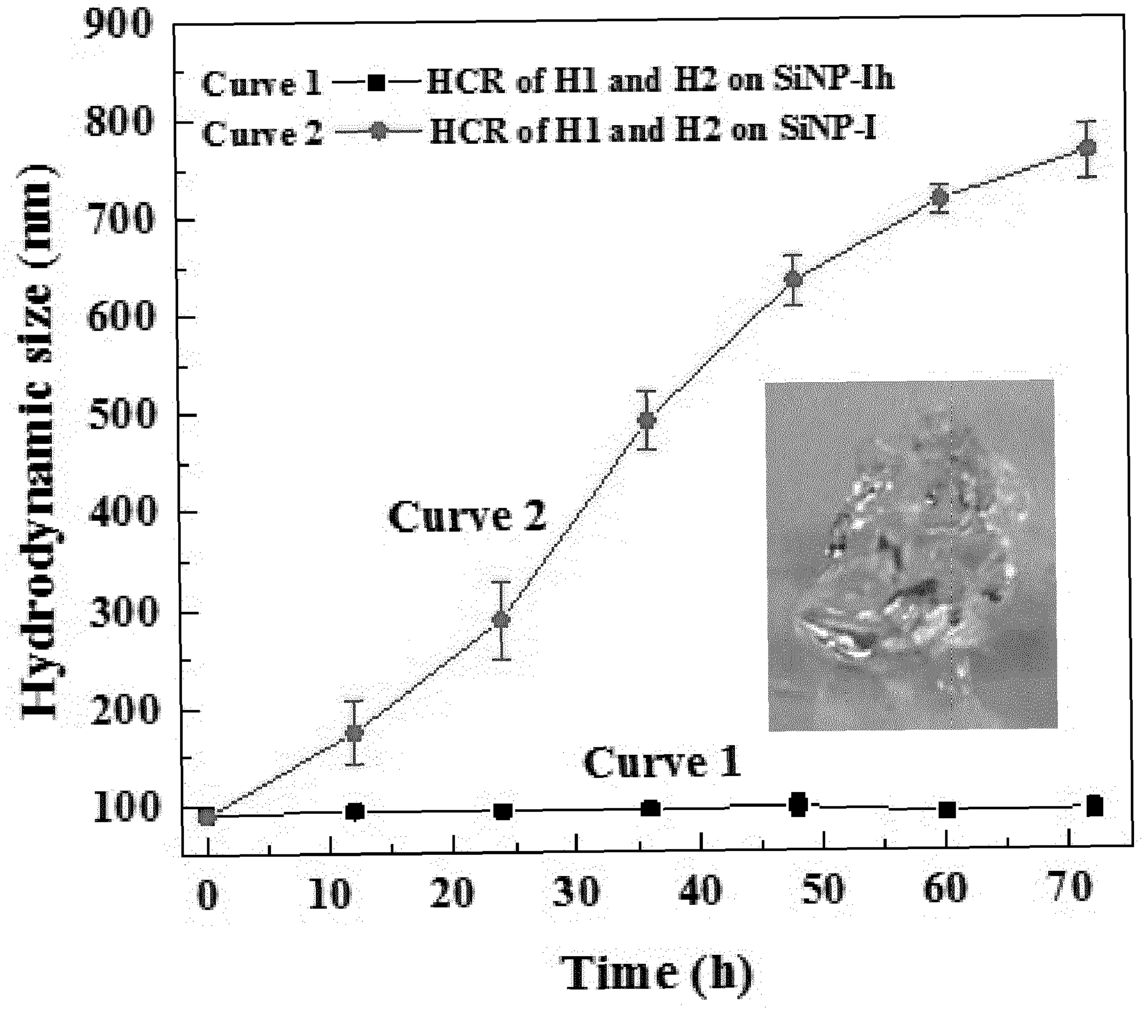

900
800
700
600
500
400
300
200
100
Hydrodynamic size (nm)
Curve 1 —■— HCR of H1 and H2 on SiNP-Ih
Curve 2 —●— HCR of H1 and H2 on SiNP-I
Curve 2
Curve 1
0
10
20
30
40
50
60
70
Time (h)

## Figure 11

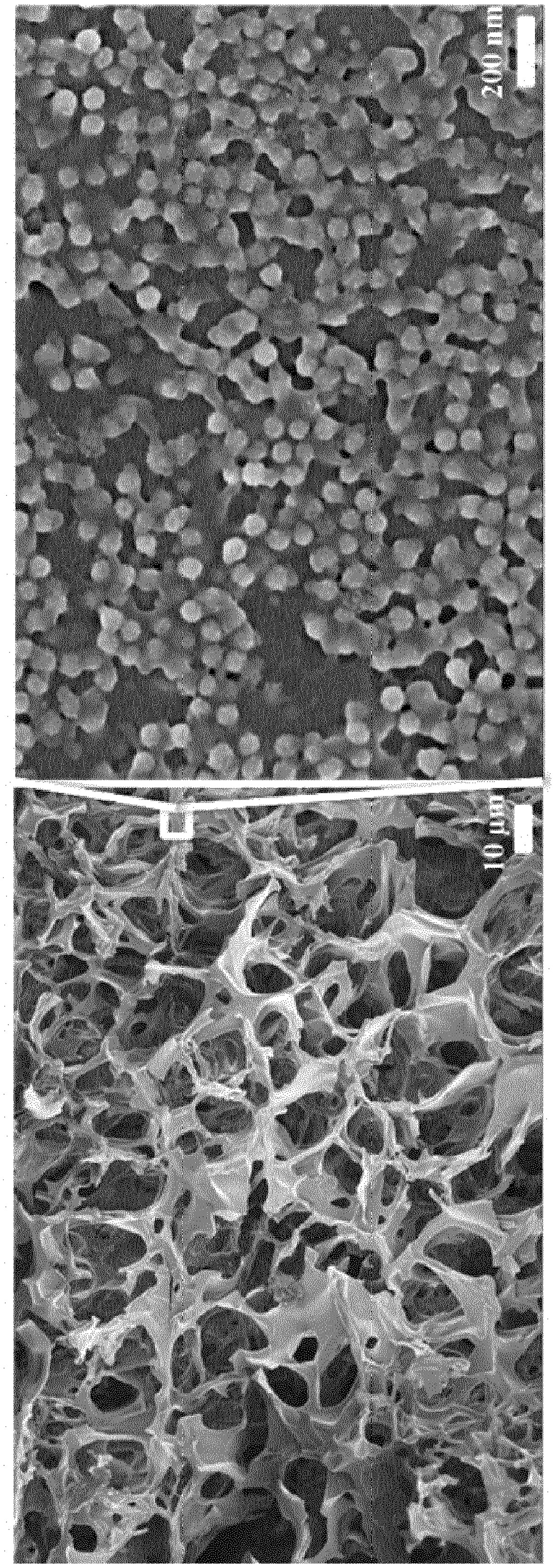

Figure 12

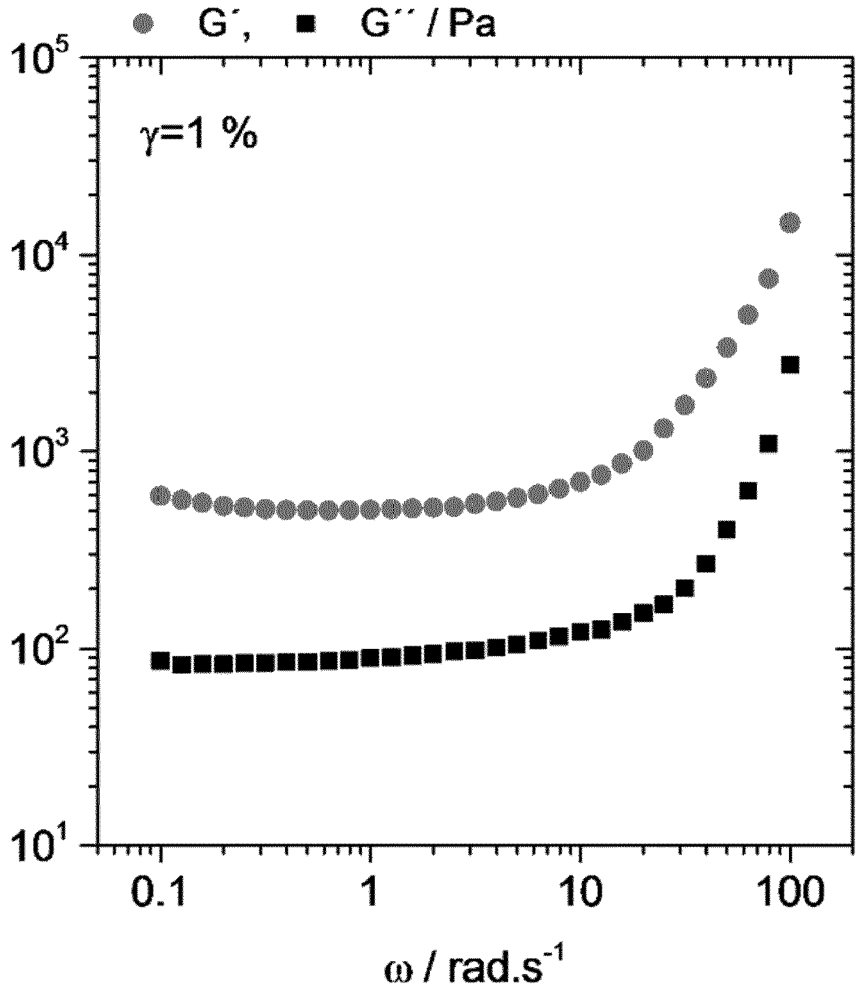

## Figure 13

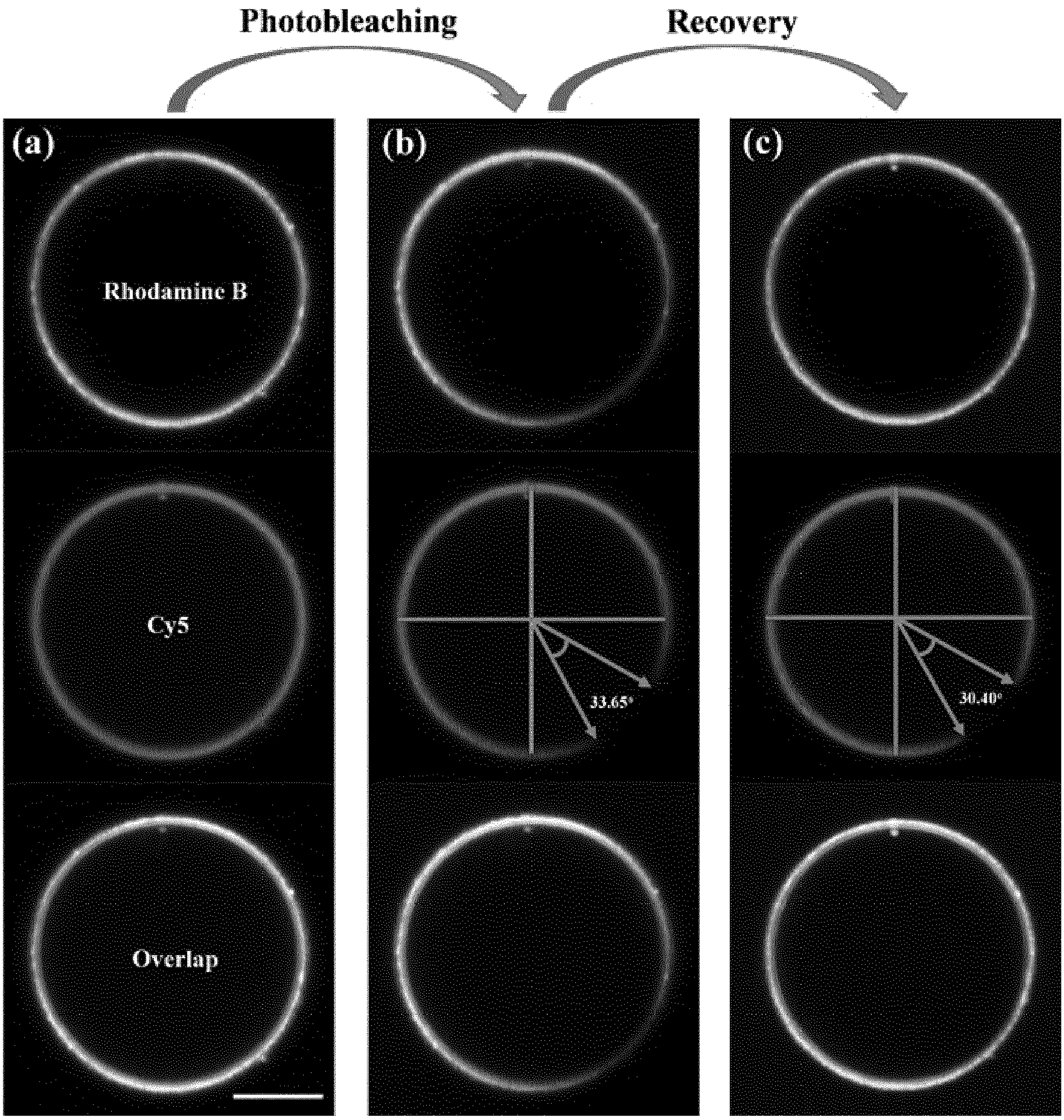
Photobleaching
Recovery
(a)
(b)
(c)
Rhodamine B
Cy5
Overlap
33.65°
30.40°

Figure 14

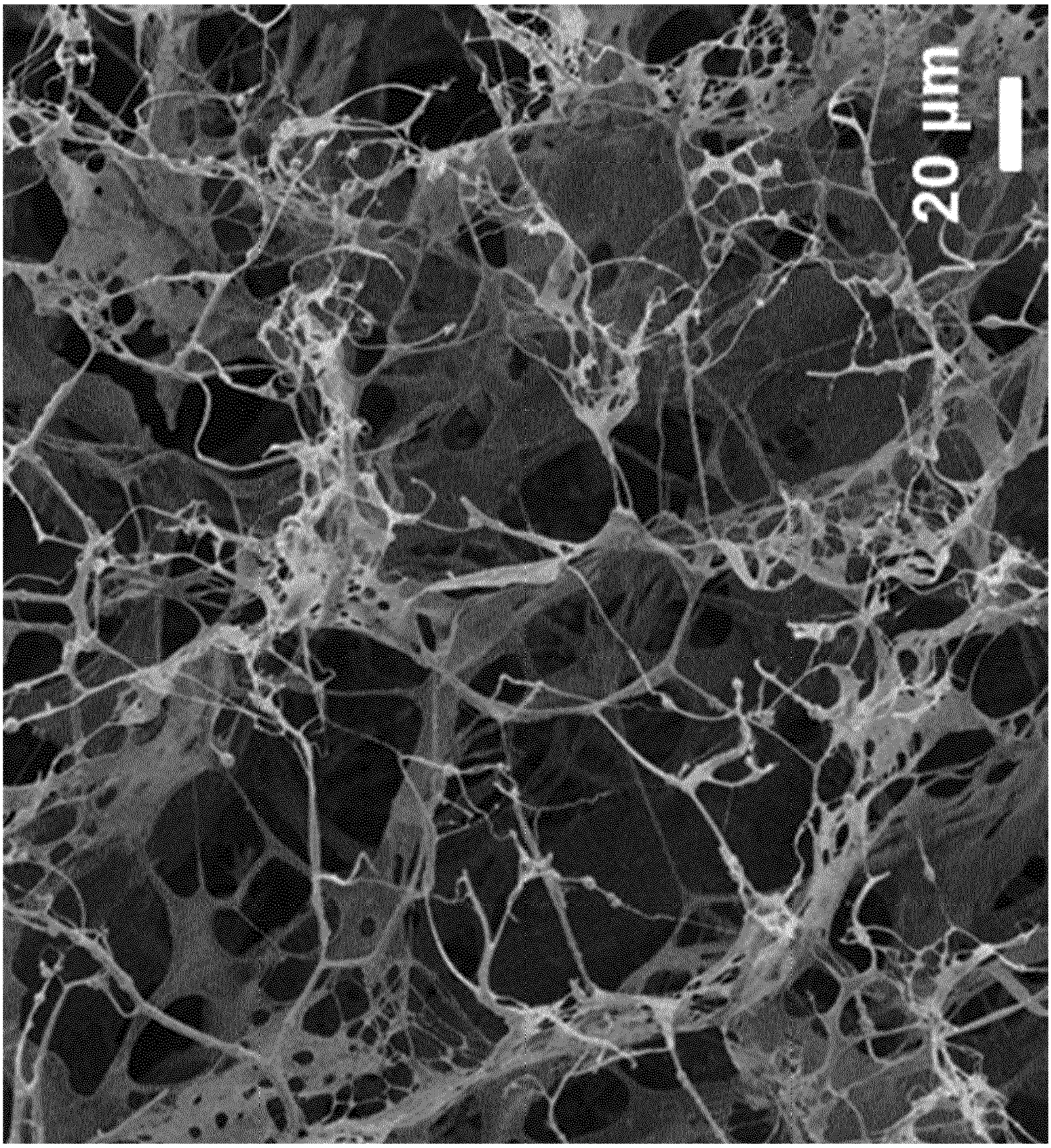

# Figure 15

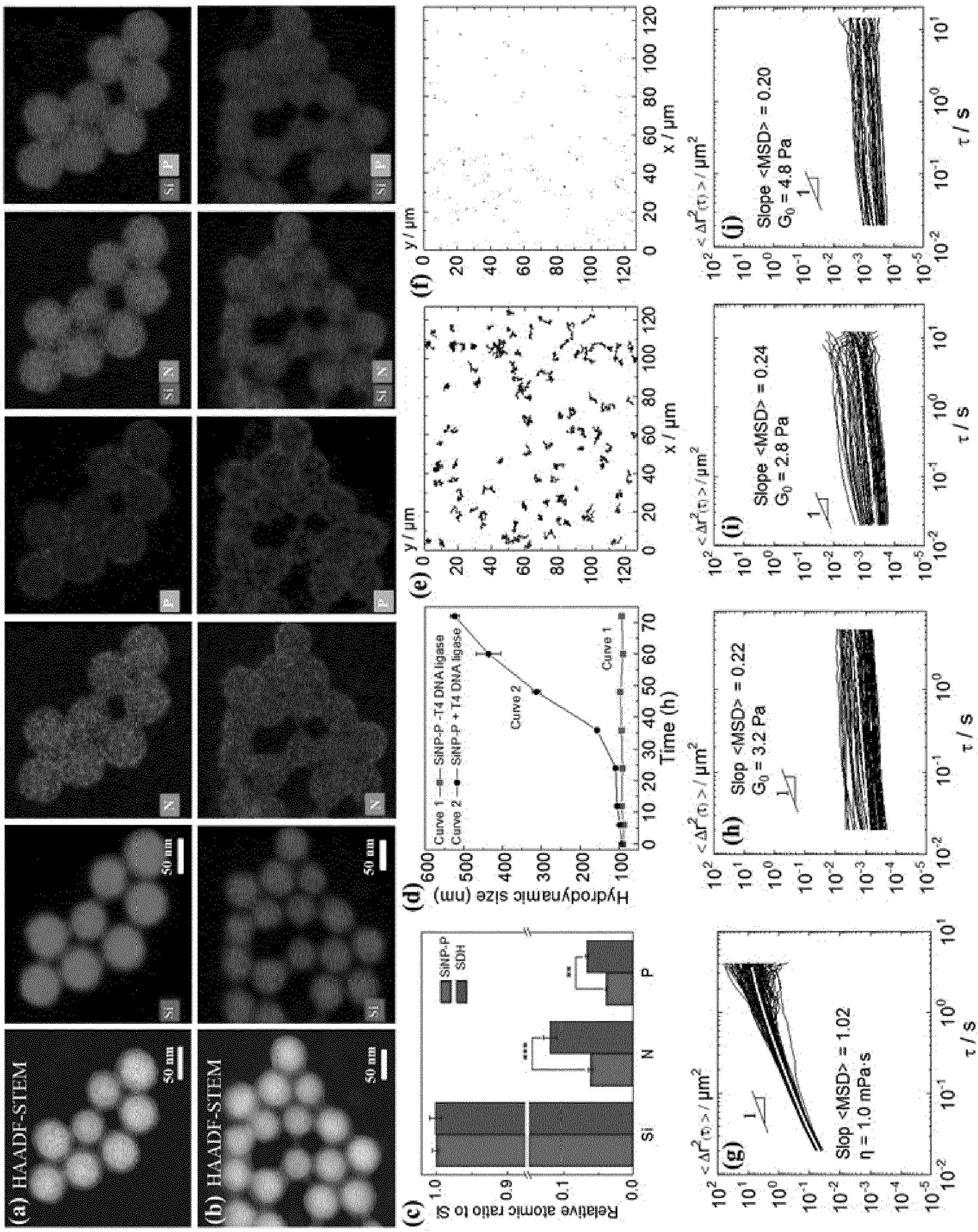
(a) HAADF-STEM
(b) HAADF-STEM
50 nm
Si
N
P
(c)
Relative atomic ratio to Si
SiNP-P
SDH
(d)
Hydrodynamic size (nm)
Time (h)
Curve 1 —■— SiNP-P -T4 DNA ligase
Curve 2 —●— SiNP-P + T4 DNA ligase
Curve 1
Curve 2
(e)
(f)
y / µm
x / µm
(g)
Slop <MSD> = 1.02
η = 1.0 mPa·s
(h)
Slop <MSD> = 0.22
$G_0$ = 3.2 Pa
(i)
Slope <MSD> = 0.24
$G_0$ = 2.8 Pa
(j)
Slope <MSD> = 0.20
$G_0$ = 4.8 Pa
τ / s

Figure 16

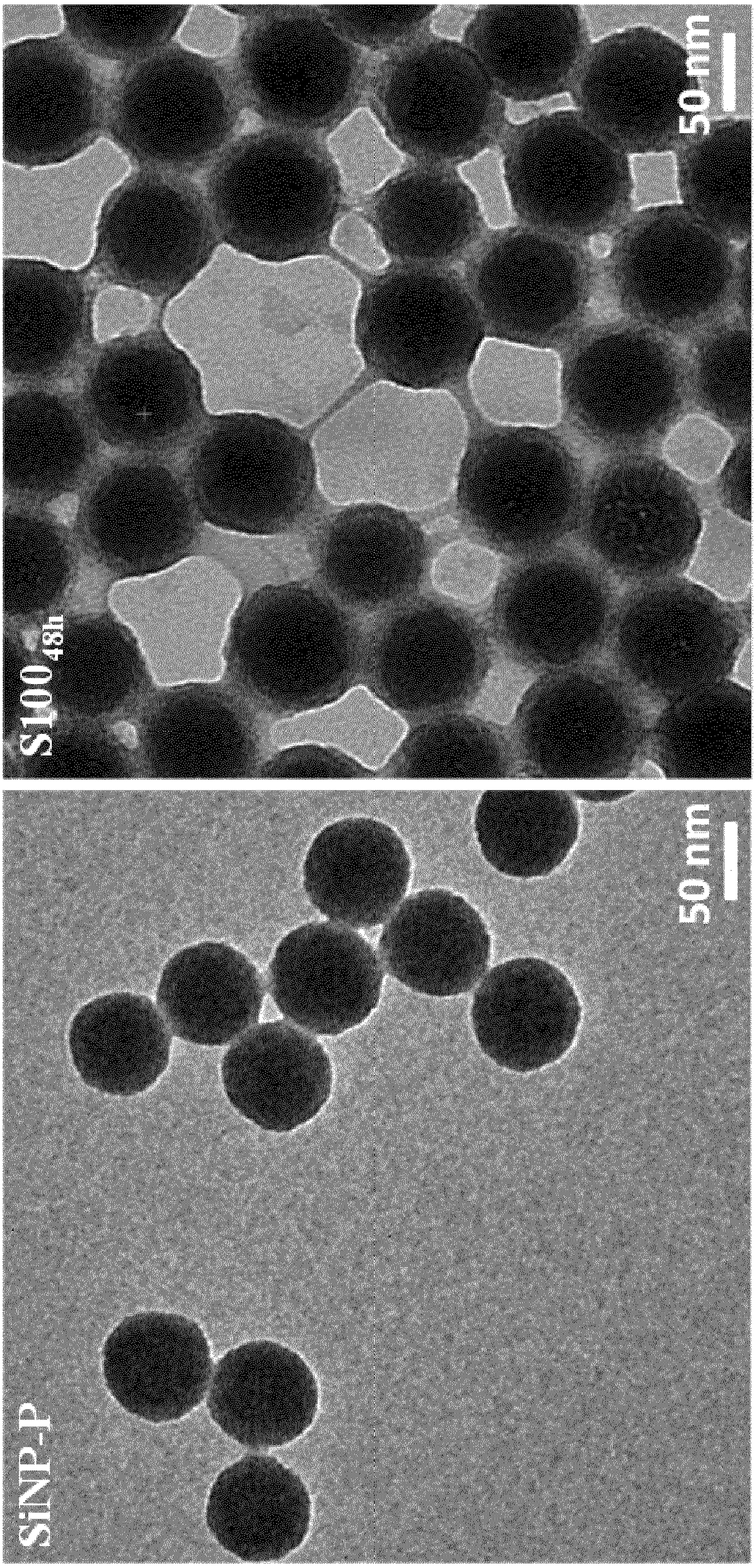

Figure 17

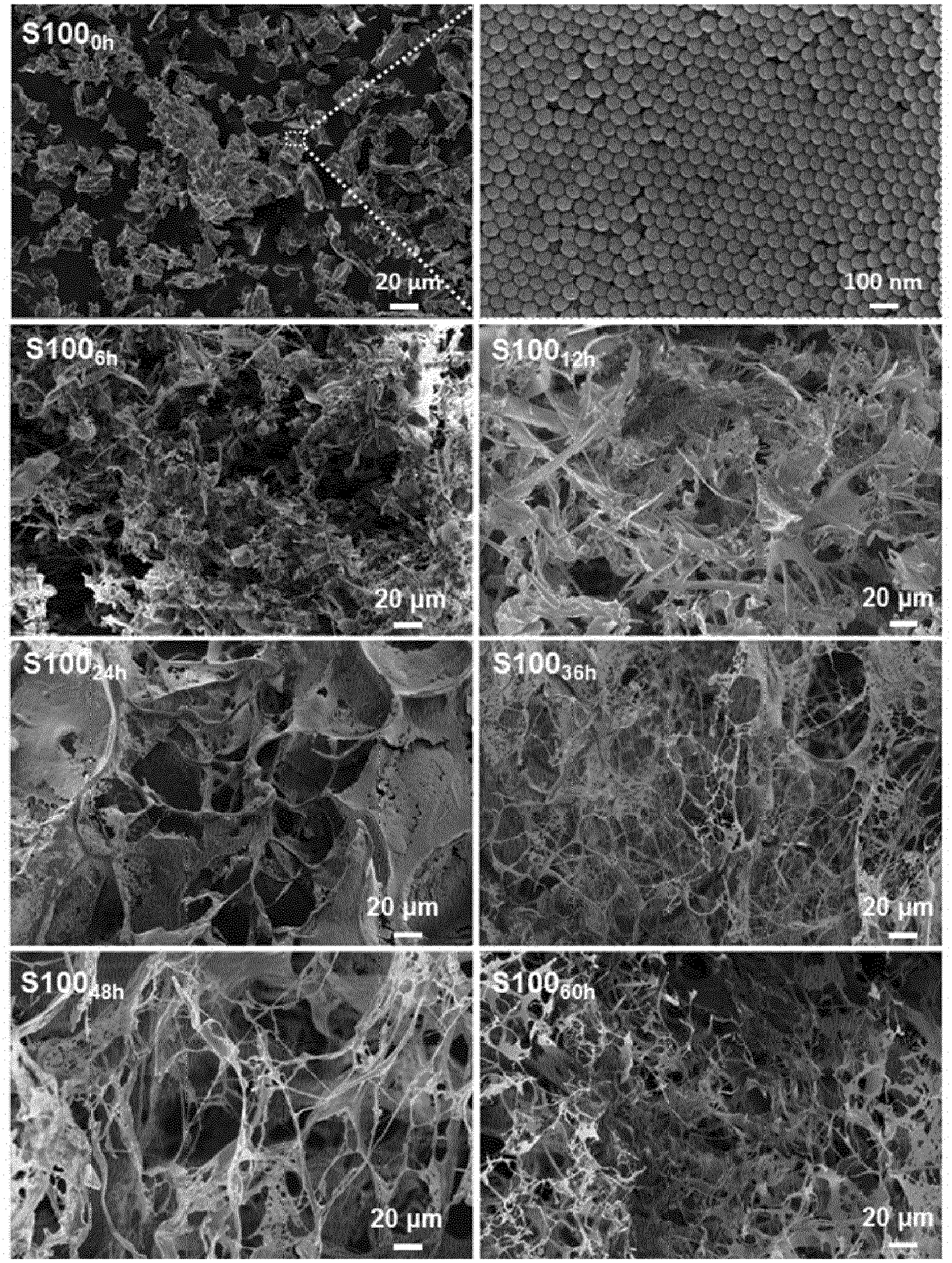
S100$_{0h}$
20 µm
100 nm
S100$_{6h}$
20 µm
S100$_{12h}$
20 µm
S100$_{24h}$
20 µm
S100$_{36h}$
20 µm
S100$_{48h}$
20 µm
S100$_{60h}$
20 µm

# Figure 18

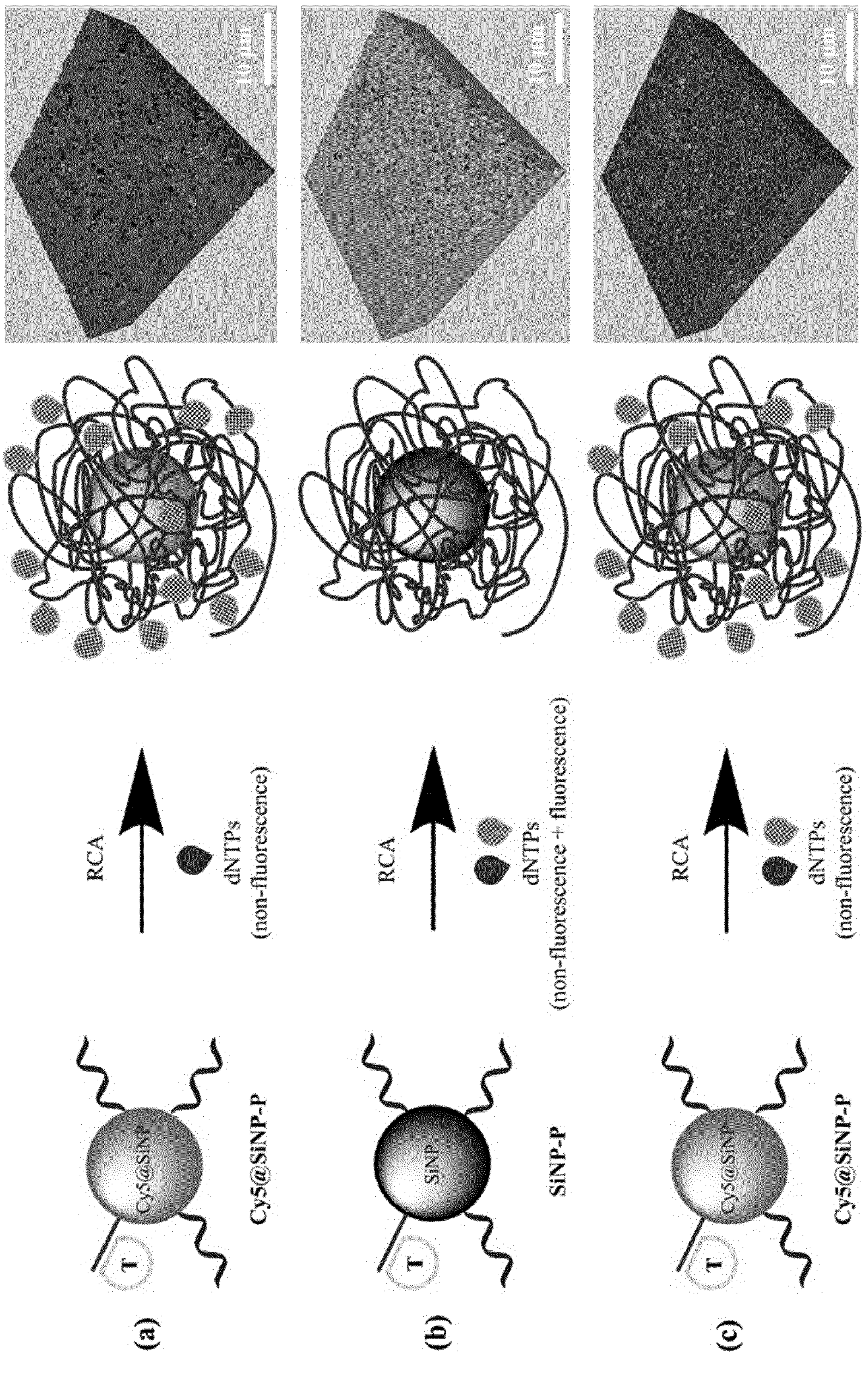
(a)
T
Cy5@SiNP
Cy5@SiNP-P
RCA
dNTPs
(non-fluorescence)
10 μm
(b)
T
SiNP
SiNP-P
RCA
dNTPs
(non-fluorescence + fluorescence)
10 μm
(c)
T
Cy5@SiNP
Cy5@SiNP-P
RCA
dNTPs
(non-fluorescence)
10 μm

# Figure 19

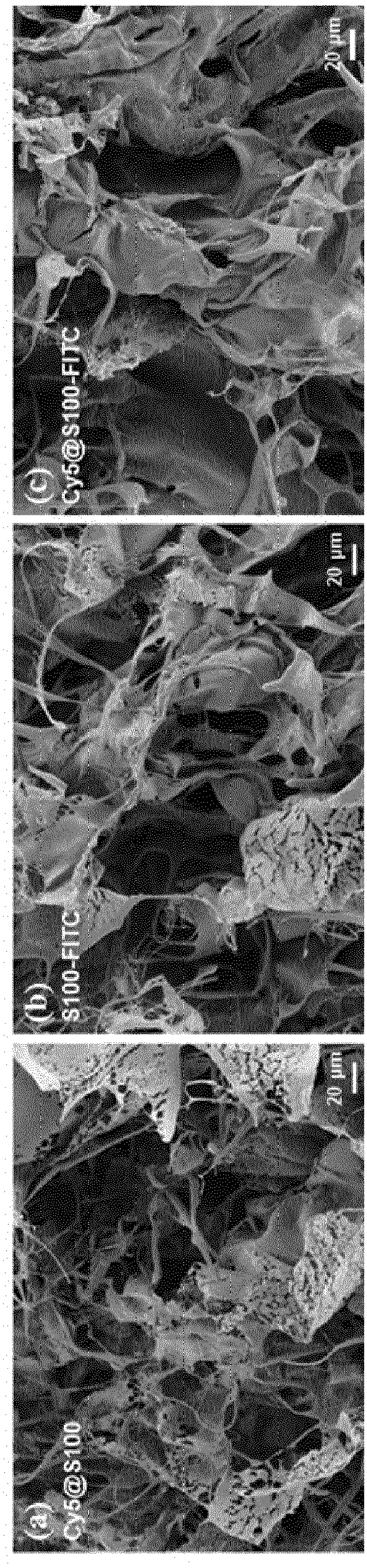

Figure 20

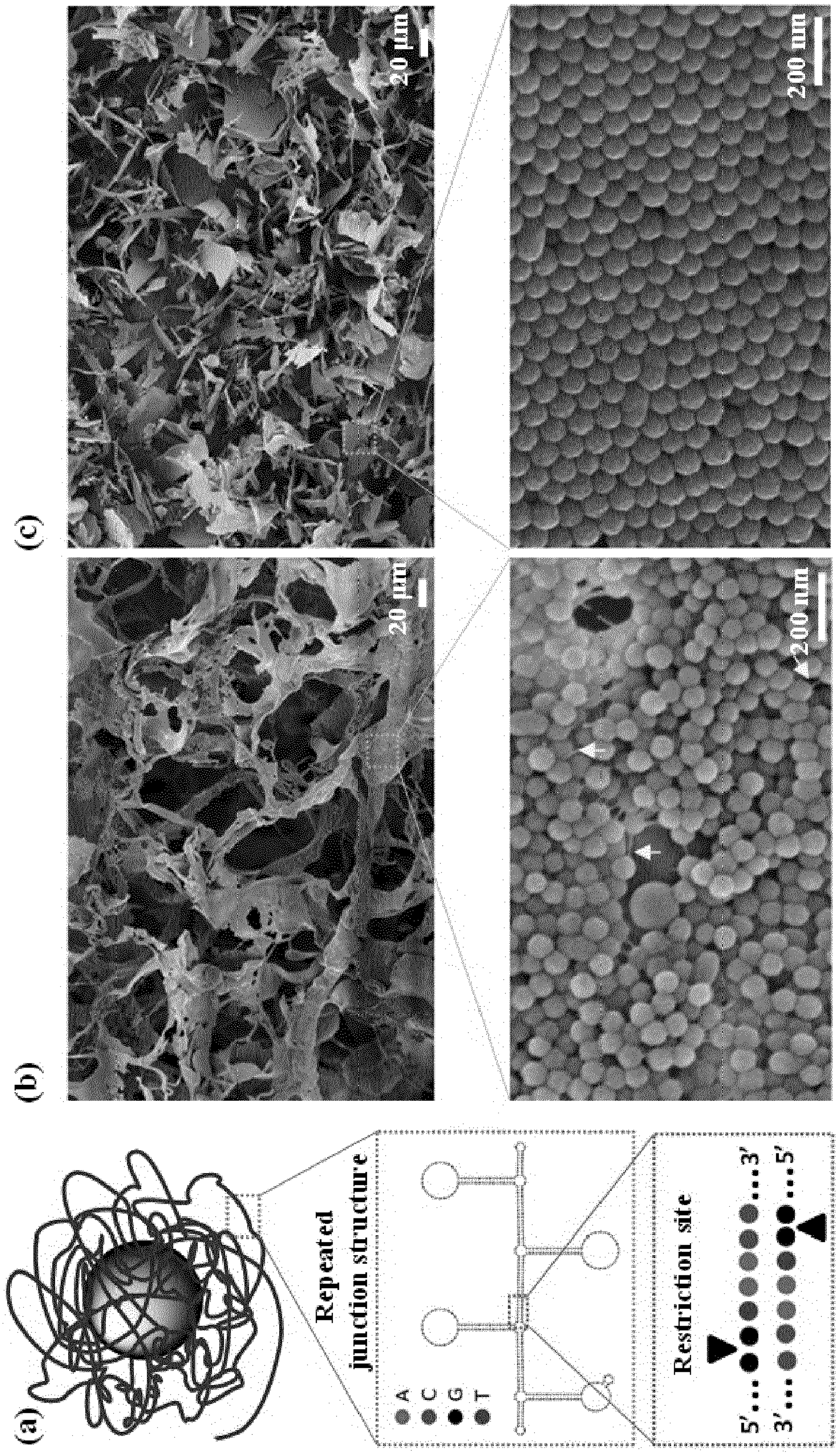
(a)
(b)
(c)
Repeated junction structure
A
C
G
T
Restriction site
5'....3'
3'....5'
20 μm
20 μm
200 nm
200 nm

Figure 21

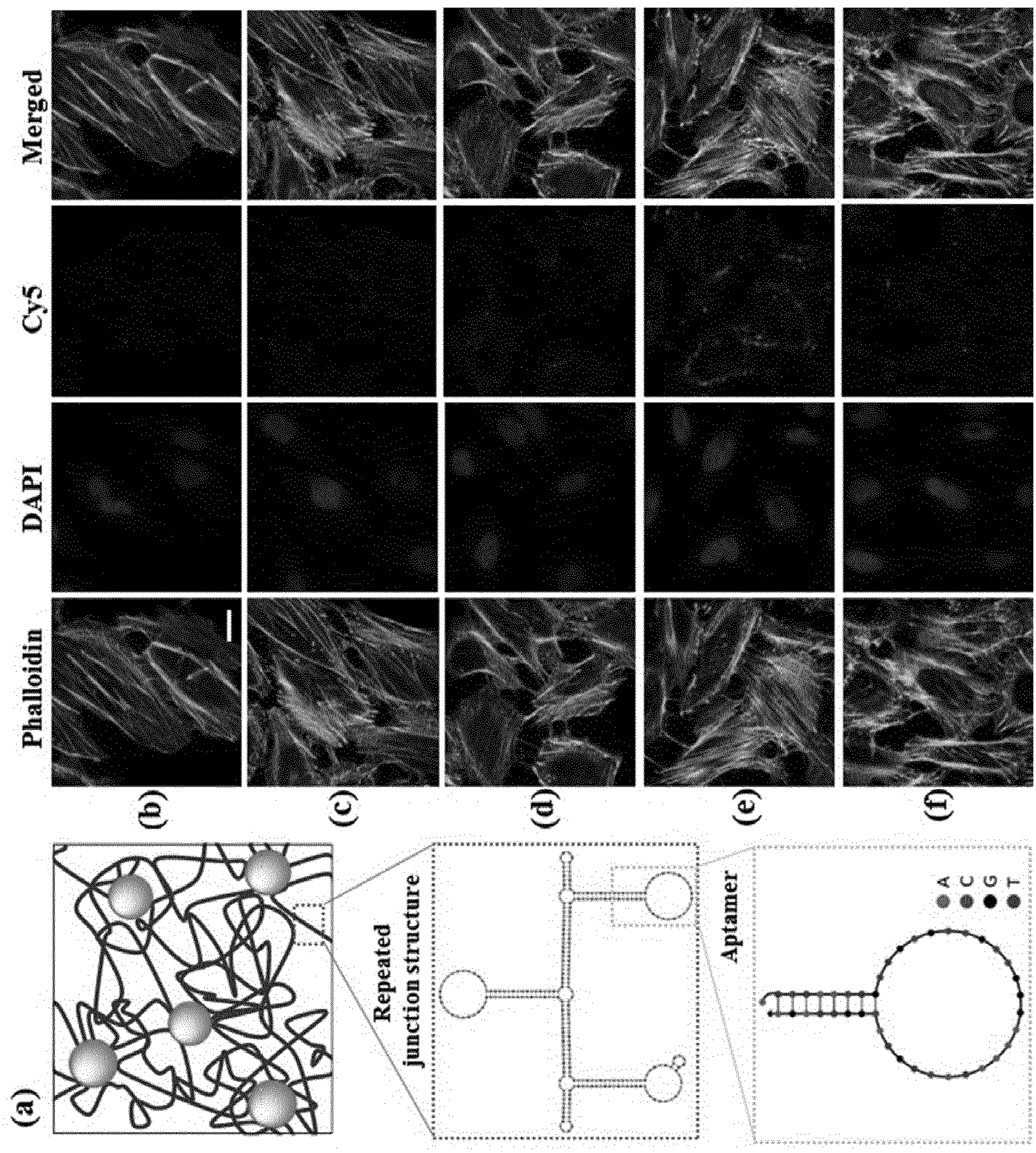
(a)
Repeated junction structure
Aptamer
A
C
G
T
(b)
(c)
(d)
(e)
(f)
Phalloidin
DAPI
Cy5
Merged

## Figure 22

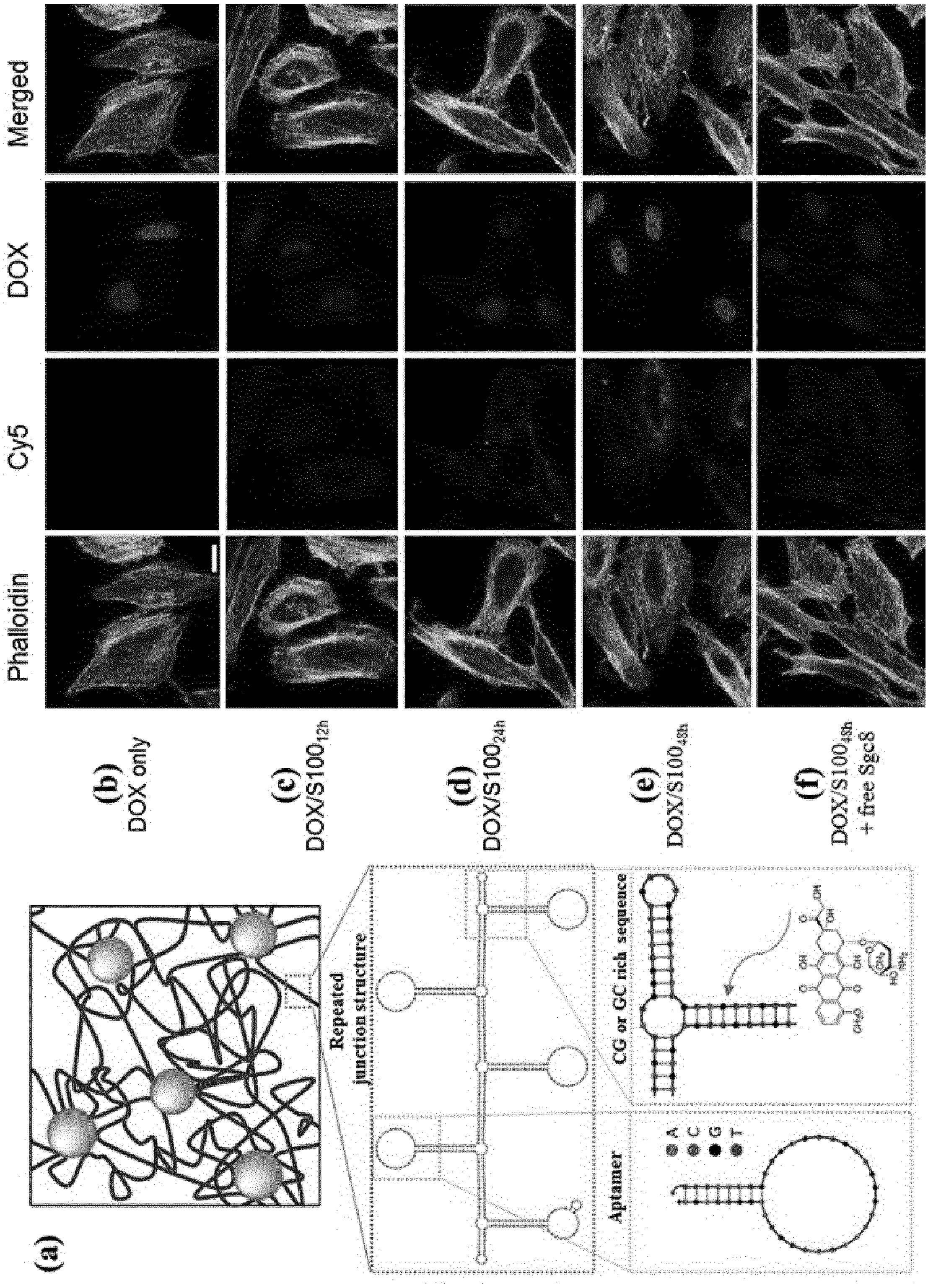
Phalloidin
Cy5
DOX
Merged
(a)
(b) DOX only
(c) DOX/S100 12h
(d) DOX/S100 24h
(e) DOX/S100 48h
(f) DOX/S100 48h + free Sgc8
Repeated junction structure
Aptamer
CG or GC rich sequence
A
C
G
T

Figure 23

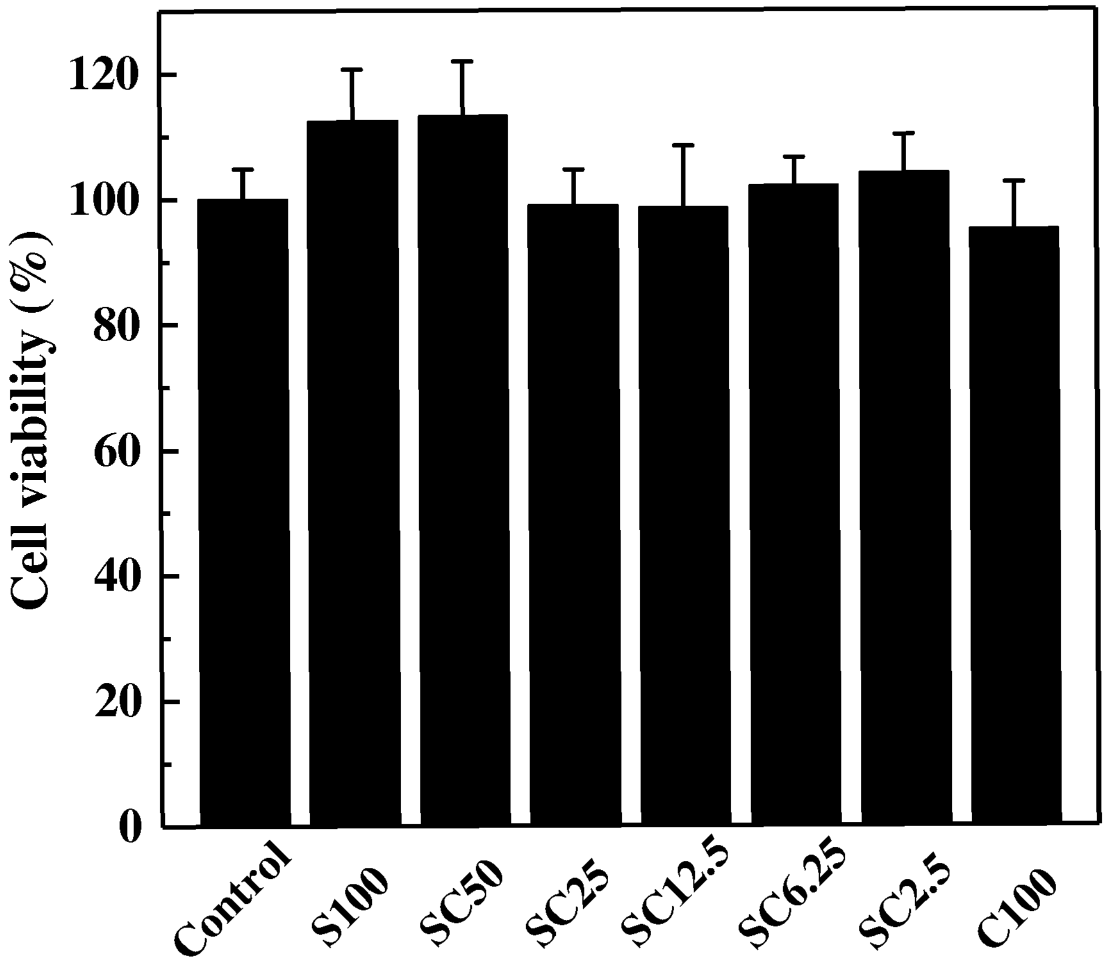
Cell viability (%)
120
100
80
60
40
20
0
Control
S100
SC50
SC25
SC12.5
SC6.25
SC2.5
C100

# Figure 24

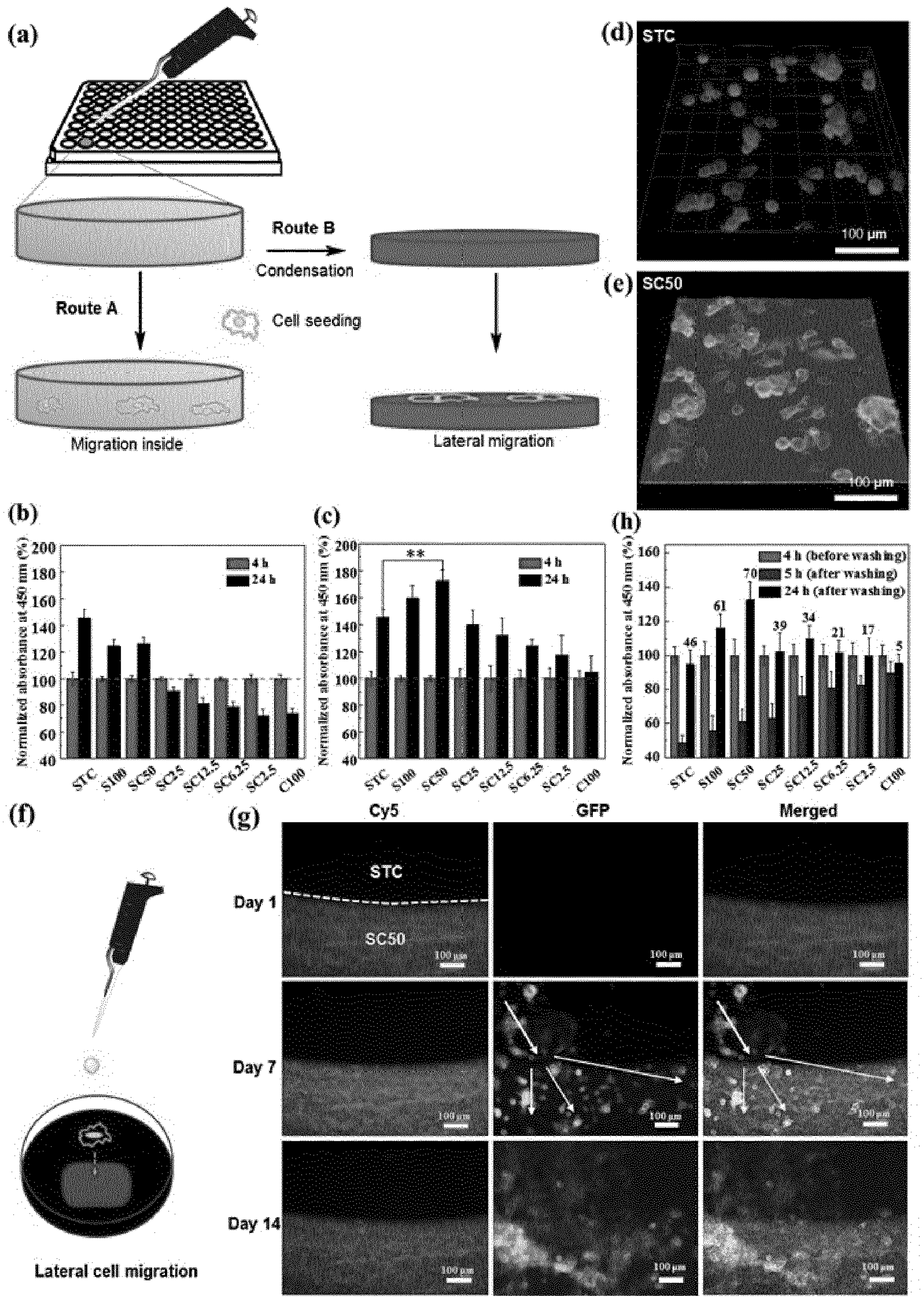
(a)
Route B
Condensation
Route A
Cell seeding
Migration inside
Lateral migration
(d) STC
100 μm
(e) SC50
100 μm
(b)
(c)
(h)
Normalized absorbance at 450 nm (%)
4 h
24 h
**
4 h (before washing)
5 h (after washing)
24 h (after washing)
46
61
70
39
34
21
17
5
STC
S100
SC50
SC25
SC12.5
SC6.25
SC2.5
C100
(f)
Lateral cell migration
(g)
Cy5
GFP
Merged
Day 1
Day 7
Day 14
100 μm

## Figure 25

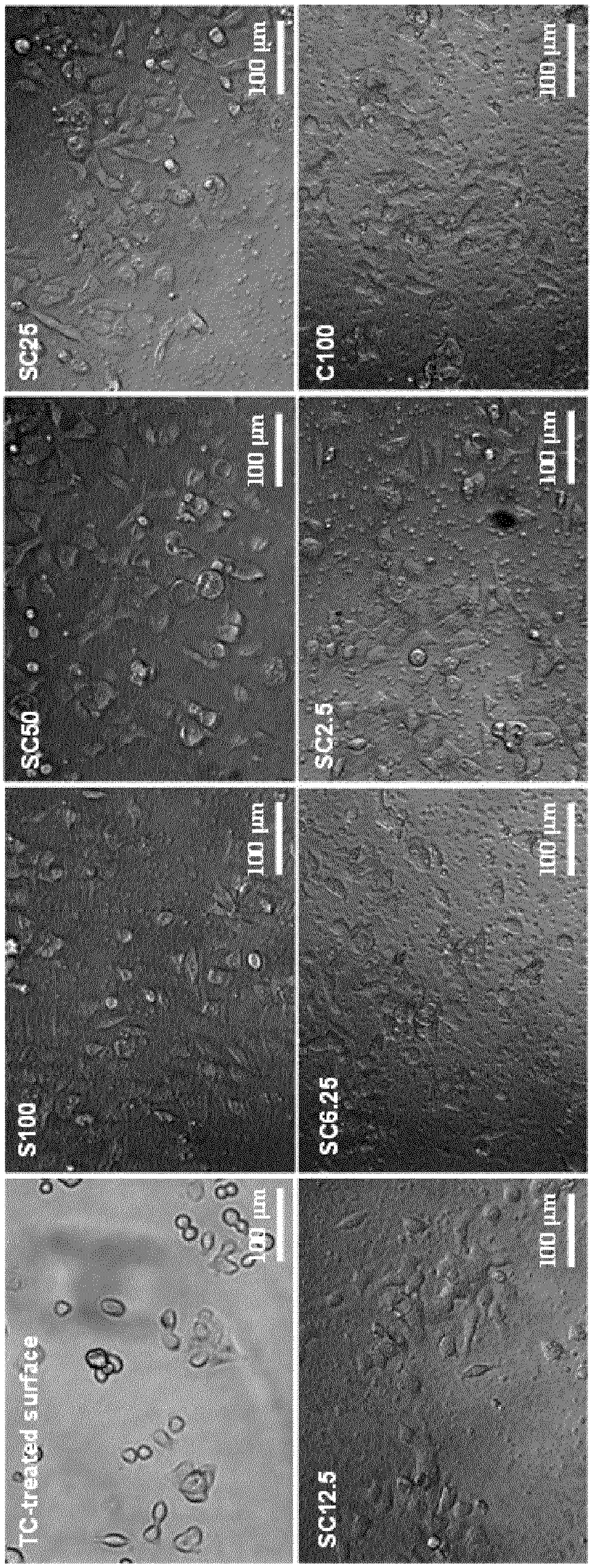
TC-treated surface
S100
SC50
SC25
SC12.5
SC6.25
SC2.5
C100
100 µm

Figure 26

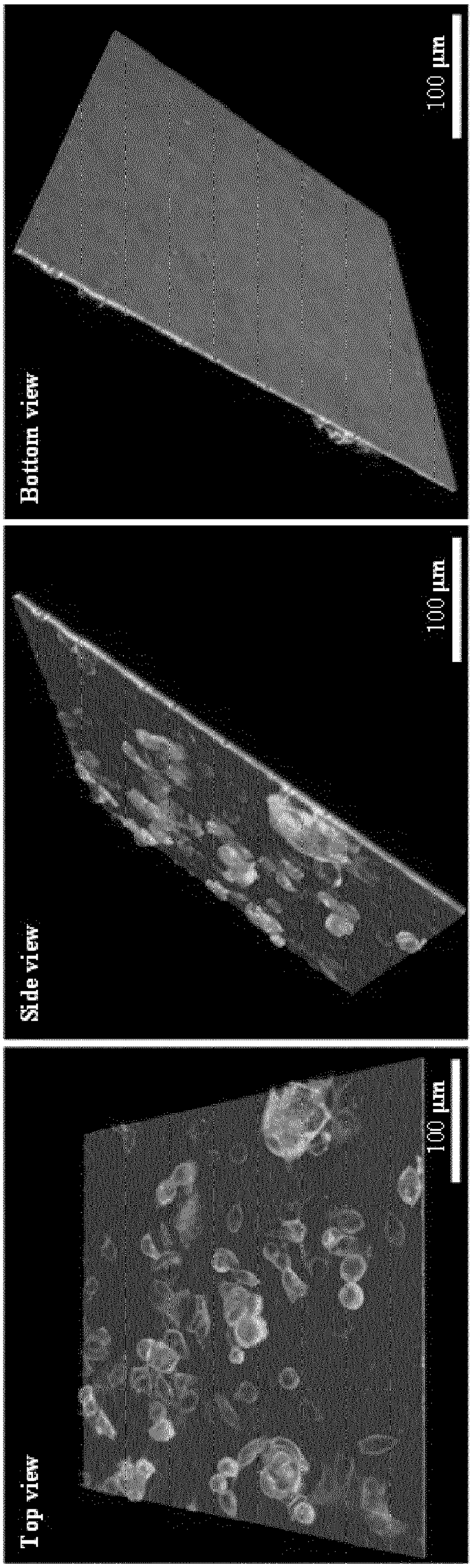

Figure 27

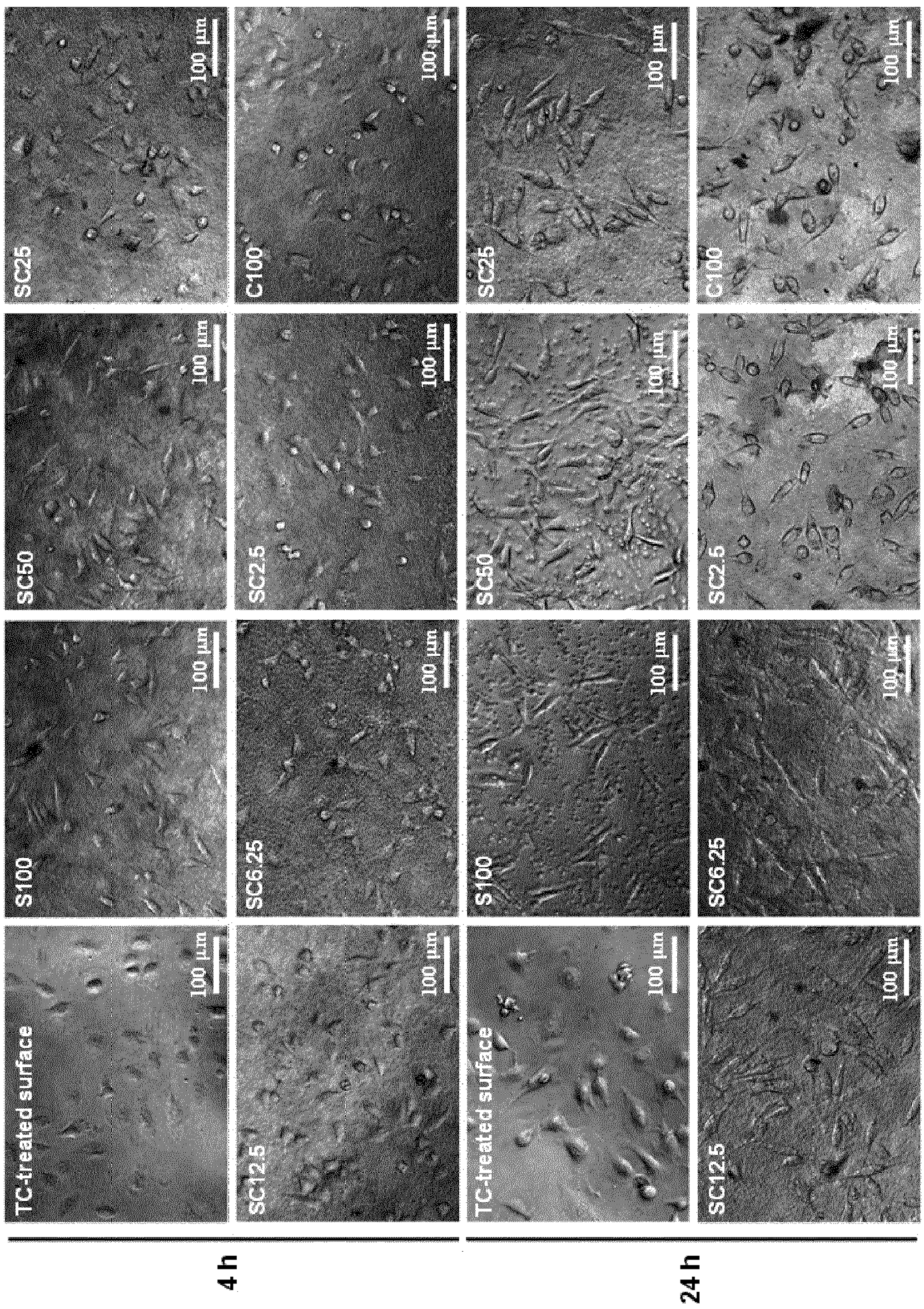
TC-treated surface
S100
SC50
SC25
SC12.5
SC6.25
SC2.5
C100
100 µm
4 h
24 h

Figure 28

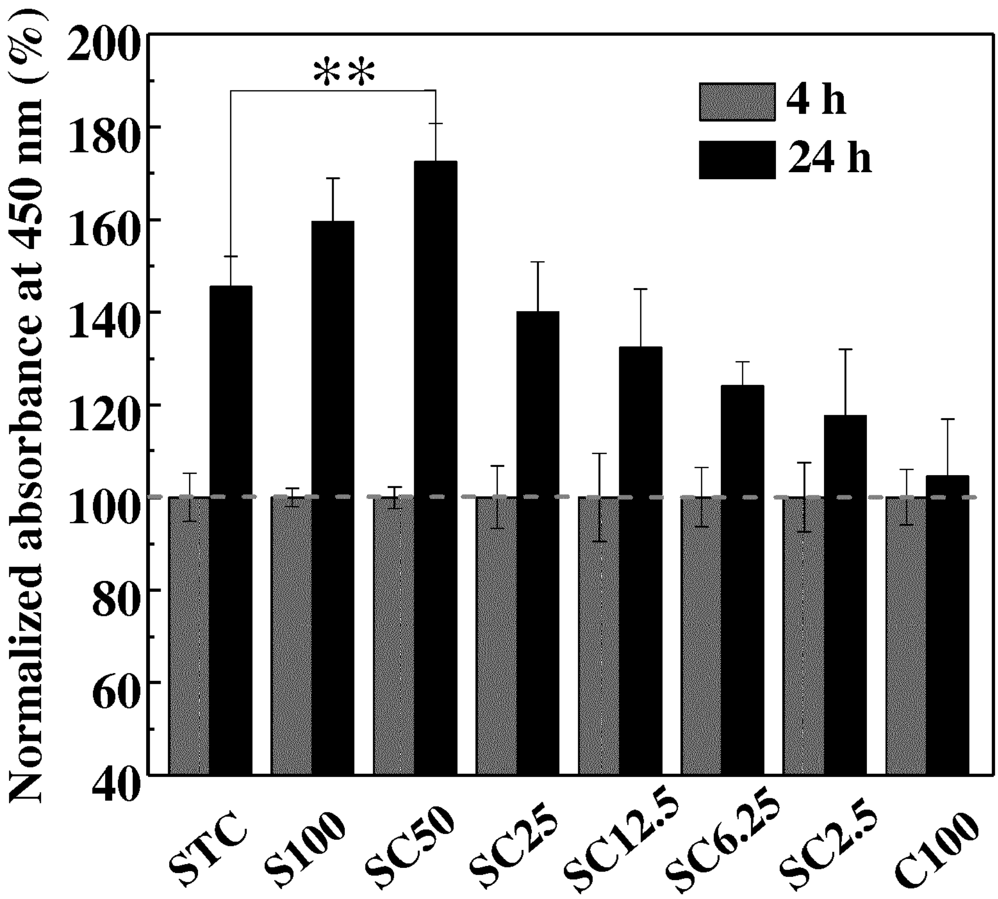


Figure 29

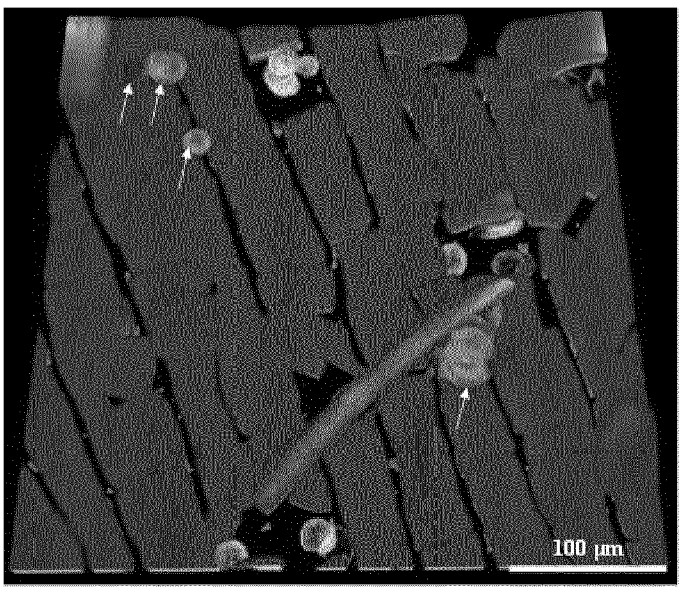

Figure 30

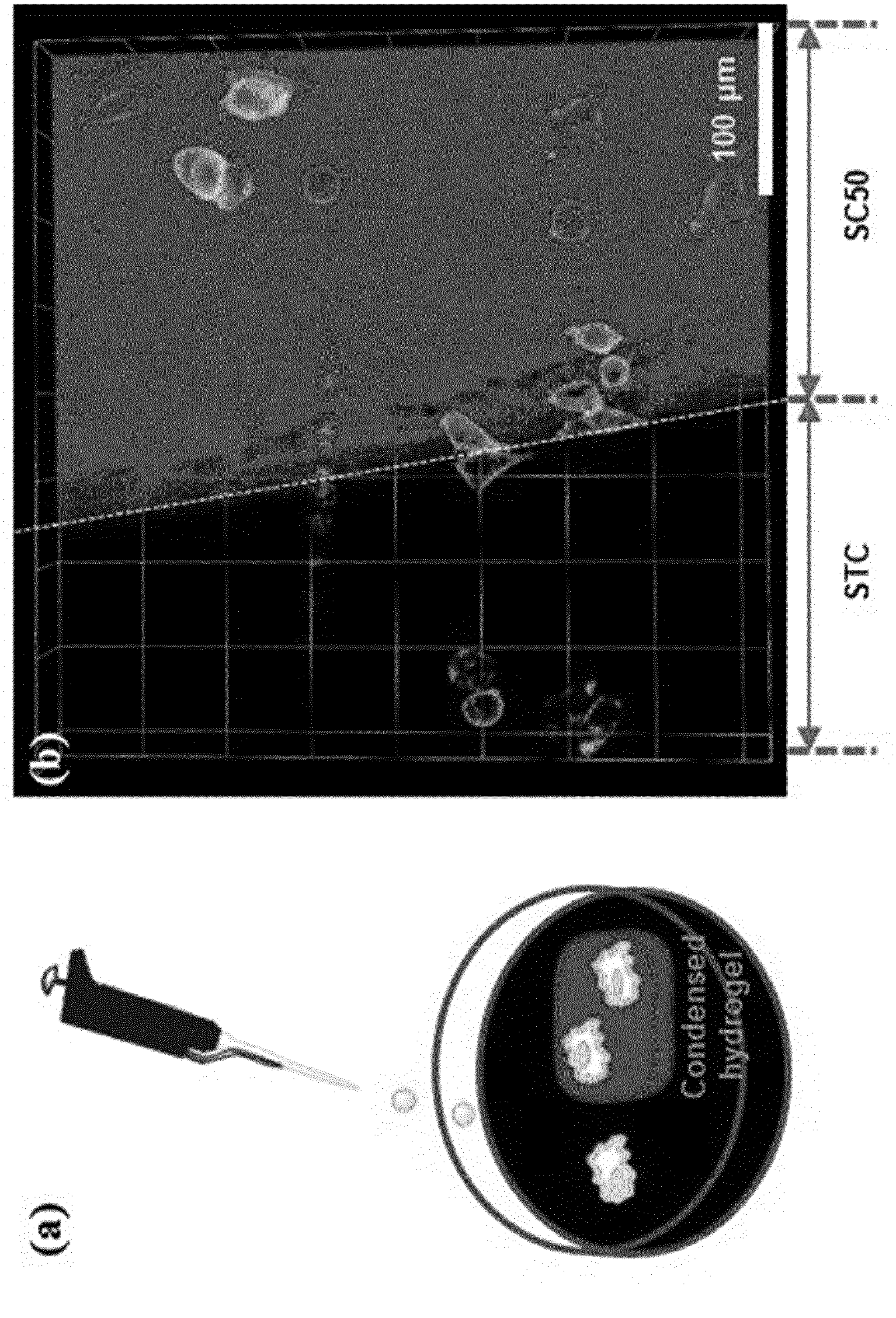

Figure 31

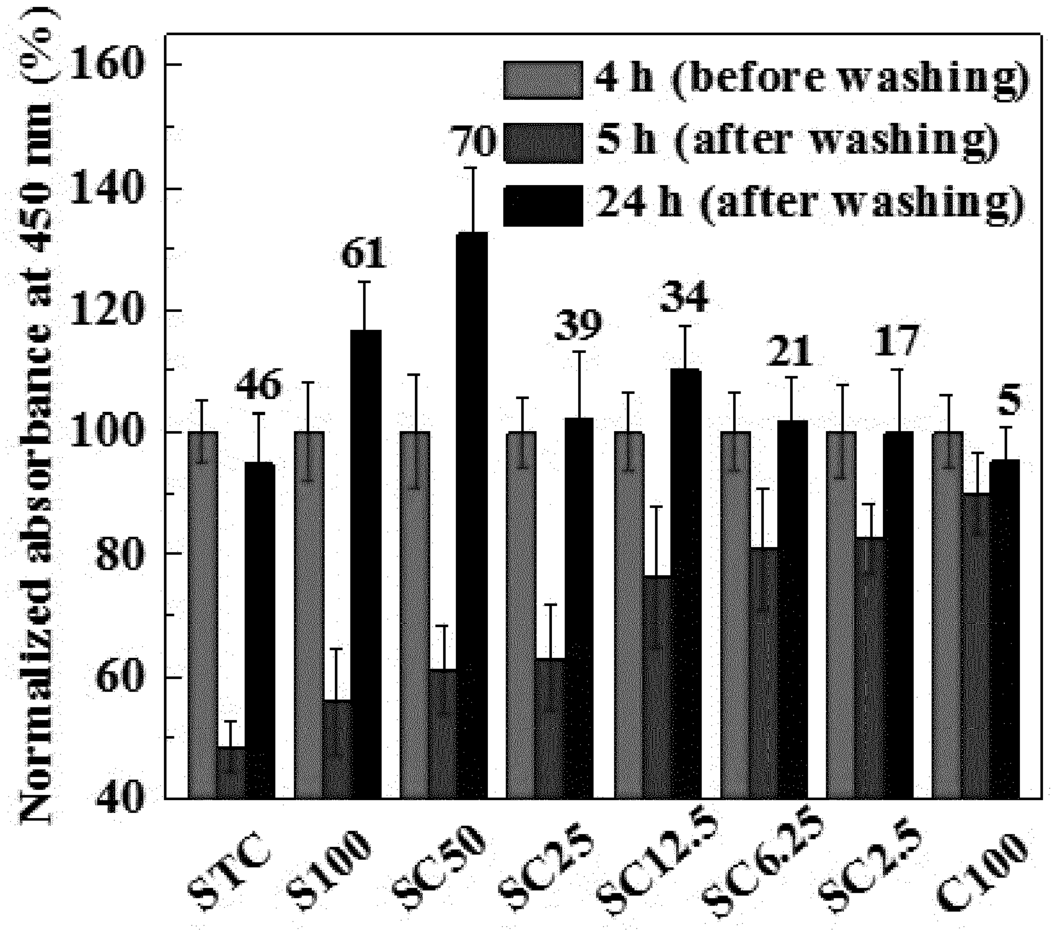
Normalized absorbance at 450 nm (%)
160
140
120
100
80
60
40
4 h (before washing)
5 h (after washing)
24 h (after washing)
46
61
70
39
34
21
17
5
STC
S100
SC50
SC25
SC12.5
SC6.25
SC2.5
C100

# Figure 32

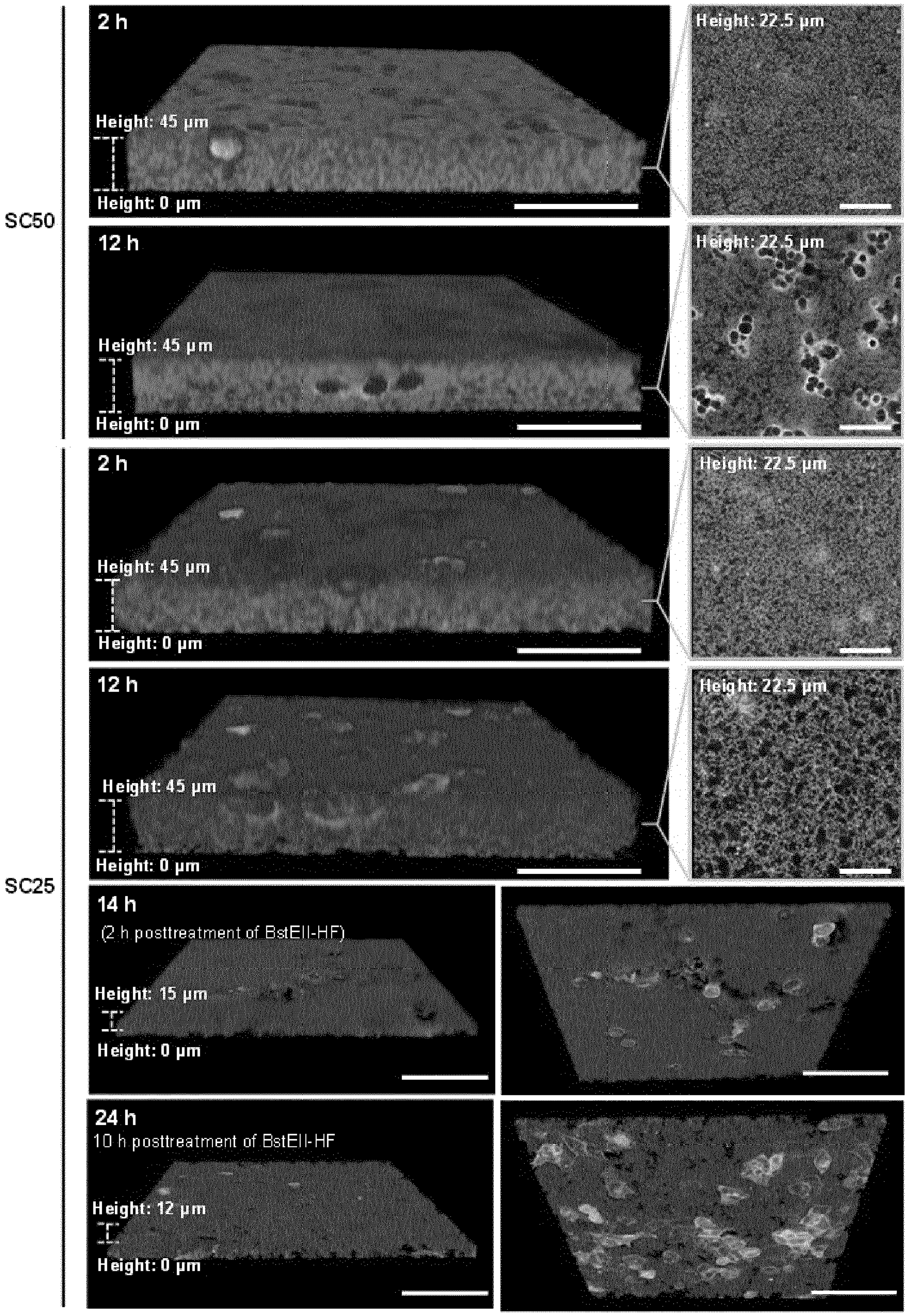
SC50
2 h
Height: 45 µm
Height: 0 µm
Height: 22.5 µm
12 h
Height: 45 µm
Height: 0 µm
Height: 22.5 µm
SC25
2 h
Height: 45 µm
Height: 0 µm
Height: 22.5 µm
12 h
Height: 45 µm
Height: 0 µm
Height: 22.5 µm
14 h
(2 h posttreatment of BstEII-HF)
Height: 15 µm
Height: 0 µm
24 h
10 h posttreatment of BstEII-HF
Height: 12 µm
Height: 0 µm

Figure 33

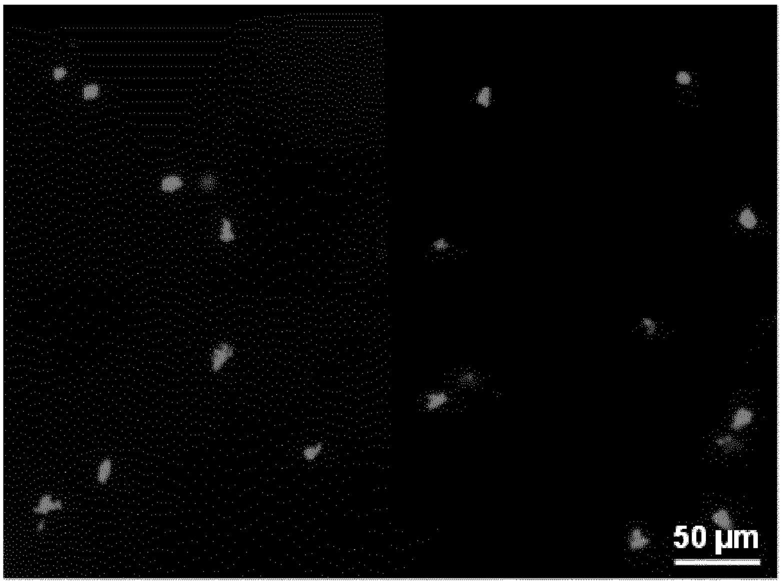


Figure 34

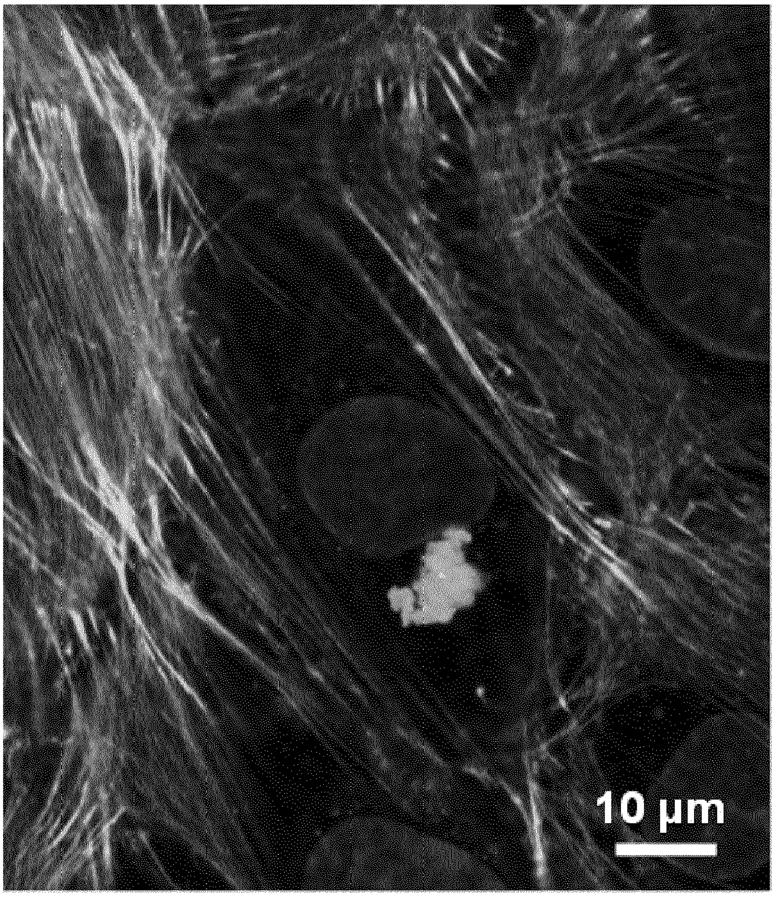

Figure 35

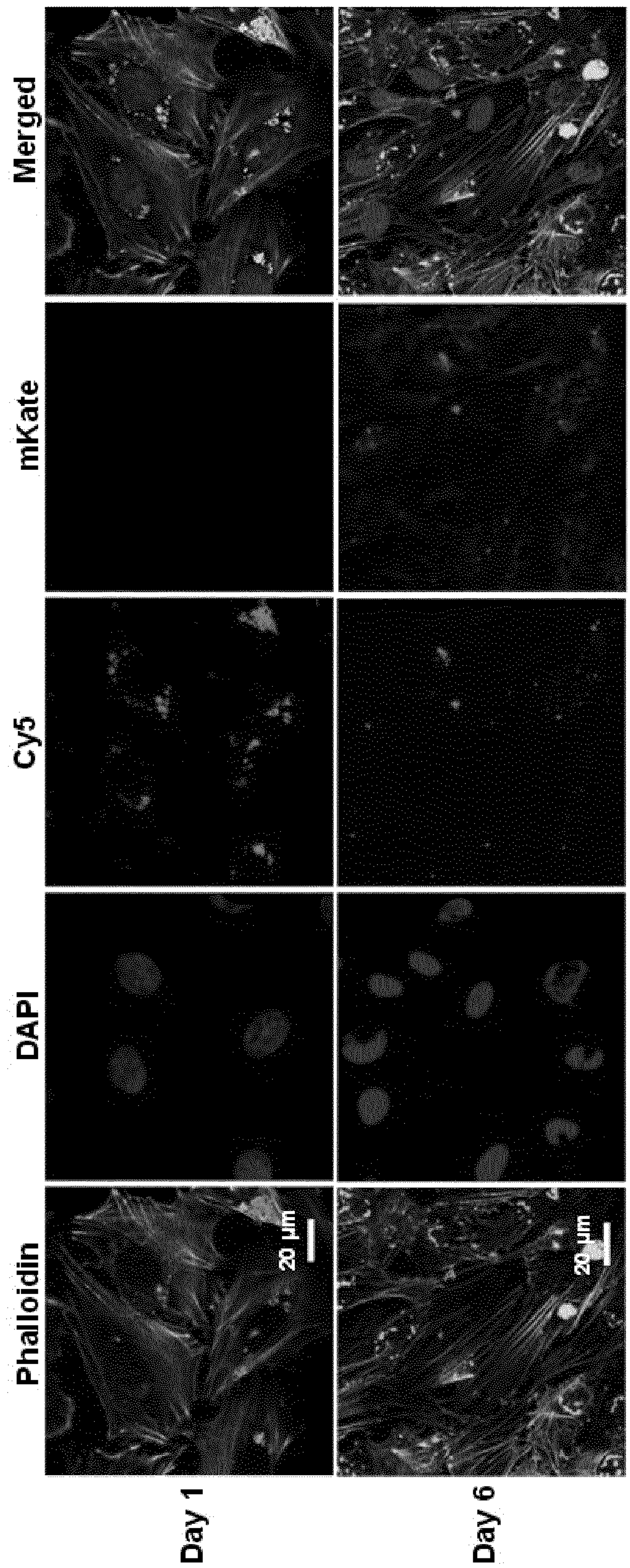

Europäisches Patentamt
European Patent Office
Office européen des brevets

# EUROPEAN SEARCH REPORT

Application Number
EP 18 18 1821

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2005/019369 A1 (LYLES MARK B [US]) 27 January 2005 (2005-01-27)<br>* example 5 *<br>* claims 1-51 *<br>* paragraph [0051] *<br>----- | 1-3,8,9<br><br>1-14 | INV.<br>B82Y5/00<br>A61K47/69 |
| Y | BAOFEN YE ET AL: "Colorimetric logic response based on aptamer functionalized colloidal crystal hydrogels", NANOSCALE, vol. 7, no. 17, 1 January 2015 (2015-01-01), pages 7565-7568, XP055512439, United Kingdom ISSN: 2040-3364, DOI: 10.1039/C5NR00586H<br>* the whole document *<br>* figures 1,3 *<br>----- | 1-14 | |
| Y | LI ZHOU ET AL: "Towards intelligent bioreactor systems: triggering the release and mixing of compounds based on DNA-functionalized hybrid hydrogel", CHEMICAL COMMUNICATIONS, vol. 50, no. 71, 1 January 2014 (2014-01-01), pages 10255-10257, XP055512295, ISSN: 1359-7345, DOI: 10.1039/C4CC04791E<br>* the whole document *<br>* Scheme 1 *<br>-----<br>-/-- | 1-14 | TECHNICAL FIELDS SEARCHED (IPC)<br>B82Y<br>A61K |

The present search report has been drawn up for all claims

1

| Place of search | Date of completion of the search | Examiner |
|---|---|---|
| Munich | 5 October 2018 | Bayer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 18 18 1821

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ANATOLY ZINCHENKO ET AL: "DNA-Assisted Solubilization of Carbon Nanotubes and Construction of DNA-MWCNT Cross-Linked Hybrid Hydrogels", NANOMATERIALS, vol. 5, no. 1, 3 March 2015 (2015-03-03), pages 270-283, XP055512187, DOI: 10.3390/nano5010270 * the whole document * * Experimental Section * ----- | 1-14 | |
| Y | NIKHITA D. MANSUKHANI ET AL: "Optothermally Reversible Carbon Nanotube-DNA Supramolecular Hybrid Hydrogels", MACROMOLECULAR RAPID COMMUNICATIONS, vol. 39, no. 2, 24 October 2017 (2017-10-24), page 1700587, XP055512190, DE ISSN: 1022-1336, DOI: 10.1002/marc.201700587 * the whole document * * Experimental Section * ----- | 1-14 | TECHNICAL FIELDS SEARCHED (IPC) |
| | -/-- | | |

The present search report has been drawn up for all claims

1

| Place of search | Date of completion of the search | Examiner |
|---|---|---|
| Munich | 5 October 2018 | Bayer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 18 18 1821

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ARGHYA PAUL ET AL: "The attenuation of restenosis following arterial gene transfer using carbon nanotube coated stent incorporating TAT/DNAAng1+Vegf nanoparticles", BIOMATERIALS., vol. 33, no. 30, 1 October 2012 (2012-10-01), pages 7655-7664, XP055293796, GB ISSN: 0142-9612, DOI: 10.1016/j.biomaterials.2012.06.096 * the whole document * * Materials and methods * ----- | 1-14 | |
| A | ARNOLD LEIDNER ET AL: "Biopebbles: DNA-Functionalized Core-Shell Silica Nanospheres for Cellular Uptake and Cell Guidance Studies", ADVANCED FUNCTIONAL MATERIALS, vol. 28, no. 18, 1 May 2018 (2018-05-01), page 1707572, XP055511830, DE ISSN: 1616-301X, DOI: 10.1002/adfm.201707572 * the whole document * * figure 1 * ----- | 1-14 | |
| | | | TECHNICAL FIELDS SEARCHED (IPC) |

1

The present search report has been drawn up for all claims

| Place of search | Date of completion of the search | Examiner |
|---|---|---|
| Munich | 5 October 2018 | Bayer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO. EP 18 18 1821

This annex lists the patent family members relating to the patent documents cited in the above-mentioned European search report. The members are as contained in the European Patent Office EDP file on
The European Patent Office is in no way liable for these particulars which are merely given for the purpose of information.

05-10-2018

| Patent document cited in search report | Publication date | Patent family member(s) | Publication date |
|---|---|---|---|
| US 2005019369 A1 | 27-01-2005 | EP 1660630 A2 | 31-05-2006 |
| | | US 2005019369 A1 | 27-01-2005 |
| | | WO 2005010164 A2 | 03-02-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHENG, E. ; XING, Y. ; CHEN, P. ; YANG, Y. ; SUN, Y. ; ZHOU, D. ; XU, L. ; FAN, Q. ; LIU, D. A.** pH-triggered, fast-responding DNA hydrogel. *Angew. Chem., Int. Ed. Engl.,* 2009, vol. 121 (41), 7796-7799
- **XING, Y. ; CHENG, E. ; YANG, Y. ; CHEN, P. ; ZHANG, T. ; SUN, Y. ; YANG, Z. ; LIU, D.** Self-assembled DNA hydrogels with designable thermal and enzymatic responsiveness. *Adv. Mater.,* 2011, vol. 23 (9), 1117-1121
- **NÖLL, T. ; SCHÖNHERR, H. ; WESNER, D. ; SCHOPFERER, M. ; PAULULAT, T. ; NÖLL, G.** Construction of Three-Dimensional DNA Hydrogels from Linear Building Blocks. *Angew. Chem., Int. Ed. Engl.,* 2014, vol. 53 (32), 8328-8332
- **UM, S. H. ; LEE, J. B. ; PARK, N. ; KWON, S. Y. ; UMBACH, C. C. ; LUO, D.** Enzyme-catalysed assembly of DNA hydrogel. *Nat. Mater.,* 2006, vol. 5 (10), 797-801
- **LEE, J. B. ; PENG, S. ; YANG, D. ; ROH, Y. H. ; FUNABASHI, H. ; PARK, N. ; RICE, E. J. ; CHEN, L. ; LONG, R. ; WU, M.** A mechanical metamaterial made from a DNA hydrogel. *Nat. Nanotechnol.,* 2012, vol. 7 (12), 816-820
- **HUANG, Y. ; XU, W. ; LIU, G. ; TIAN, L.** A pure DNA hydrogel with stable catalytic ability produced by one-step rolling circle amplification. *Chem. Commun.,* 2017, vol. 53 (21), 3038-3041
- **HU, R. ; ZHANG, X. ; ZHAO, Z. ; ZHU, G. ; CHEN, T. ; FU, T. ; TAN, W.** DNA nanoflowers for multiplexed cellular imaging and traceable targeted drug delivery. *Angew. Chem., Int. Ed. Engl.,* 2014, vol. 126 (23), 5931-5936
- **ZHU, G. ; HU, R. ; ZHAO, Z. ; CHEN, Z. ; ZHANG, X. ; TAN, W.** Noncanonical self-assembly of multifunctional DNA nanoflowers for biomedical applications. *J. Am. Chem. Soc.,* 2013, vol. 135 (44), 16438-16445
- **LV, Y. ; HU, R. ; ZHU, G. ; ZHANG, X. ; MEI, L. ; LIU, Q. ; QIU, L. ; WU, C. ; TAN, W.** Preparation and biomedical applications of programmable and multifunctional DNA nanoflowers. *Nat. Protoc.,* 2015, vol. 10 (10), 1508-1524
- **SUN, W. ; JIANG, T. ; LU, Y. ; REIFF, M. ; MO, R. ; GU, Z.** Cocoon-like self-degradable DNA nanoclew for anticancer drug delivery. *J. Am. Chem. Soc.,* 2014, vol. 136 (42), 14722-14725
- **KIM, E. ; ZWI-DANTSIS, L. ; REZNIKOV, N. ; HANSEL, C. S. ; AGARWAL, S. ; STEVENS, M. M.** One-Pot Synthesis of Multiple Protein - Encapsulated DNA Flowers and Their Application in Intracellular Protein Delivery. *Adv. Mater.,* 2017, vol. 29, 1701086
- **WANG, J. ; CHAO, J. ; LIU, H. ; SU, S. ; WANG, L. ; HUANG, W. ; WILLNER, I. ; FAN, C.** Clamped Hybridization Chain Reactions for the Self - Assembly of Patterned DNA Hydrogels. *Angew. Chem., Int. Ed. Engl.,* 2017, vol. 56 (8), 2171-2175
- **LEIDNER, A. ; WEIGEL, S. ; BAUER, J. ; REIBER, J. ; ANGELIN, A. ; GRÖSCHE, M. ; SCHARNWEBER, T. ; NIEMEYER CHRISTOF, M.** Biopebbles: DNA-Functionalized Core-Shell Silica Nanospheres for Cellular Uptake and Cell Guidance Studies. *Adv. Funct. Mater.,* 2018
- **BARET, J.-C.** Surfactants in droplet-based microfluidics. *Lab Chip,* 2012, vol. 12 (3), 422-433
- **BAUER, W.-A. C. ; FISCHLECHNER, M. ; ABELL, C. ; HUCK, W. T.** Hydrophilic PDMS microchannels for high-throughput formation of oil-in-water microdroplets and water-in-oil-in-water double emulsions. *Lab Chip,* 2010, vol. 10 (14), 1814-1819
- **J. N. ZADEH ; C. D. STEENBERG ; J. S. BOIS ; B. R. WOLFE ; M. B. PIERCE ; A. R. KHAN ; R. M. DIRKS ; N. A. PIERCE.** *J. Comput. Chem.,* 2011, vol. 32, 170
- **D. SHANGGUAN ; Y. LI ; Z. TANG ; Z. C. CAO ; H. W. CHEN ; P. MALLIKARATCHY ; K. SEFAH ; C. J. YANG ; W. TAN.** *Proc. Natl. Acad. Sci. U. S. A.,* 2006, vol. 103, 11838
- **G. ZHU ; R. HU ; Z. ZHAO ; Z. CHEN ; X. ZHANG ; W. TAN.** *J. Am. Chem. Soc.,* 2013, vol. 135, 16438
- **OELSCHLAEGER, C. ; BOSSLER, F. ; WILLENBACHER, N.** Synthesis, structural and micromechanical properties of 3D hyaluronic acid-based cryogel scaffolds. *Biomacromolecules,* 2016, vol. 17, 580-589
- **LEIDNER, A. et al.** Biopebbles: DNA - Functionalized Core-Shell Silica Nanospheres for Cellular Uptake and Cell Guidance Studies. *Adv. Funct. Mater.,* 2018, vol. 0

- **LILIENTHAL, S. ; SHPILT, Z. ; WANG, F. ; ORBACH, R. ; WILLNER, I.** Programmed DNAzyme-Triggered Dissolution of DNA-Based Hydrogels: Means for Controlled Release of Biocatalysts and for the Activation of Enzyme Cascades. *ACS Appl. Mater. Interfaces,* 2015, vol. 7, 8923-8931
- **SHANGGUAN, D. et al.** Aptamers evolved from live cells as effective molecular probes for cancer study. *Proc. Natl. Acad. Sci. U. S. A.,* 2006, vol. 103, 11838-11843
- **ZHU, G. et al.** Self-Assembled Aptamer - Based Drug Carriers for Bispecific Cytotoxicity to Cancer Cells. *Chem. - Asian J.,* 2012, vol. 7, 1630-1636
- **ANGELIN, A. et al.** Multiscale Origami Structures as Interface for Cells. *Angew Chem Int Ed Engl,* 2015, vol. 54, 15813-15817

SEQUENCE LISTING

<110> KIT

<120> Composite material comprising DNA hydrogel and silica nanoparticles

<130> K 6049EU

<160> 14

<170> PatentIn version 3.5

<210> 1
<211> 58
<212> DNA
<213> Artificial Sequence

<220>
<223> oligonucleotide for preparing DNA hairpins for the linear HCR process

<400> 1
gatcgcgatc ctggctcctg tgattgtgct ctagacatcg ctagagcaca atcacagg 58

<210> 2
<211> 52
<212> DNA
<213> Artificial Sequence

<220>
<223> oligonucleotide for preparing DNA hairpins for the linear HCR process

<400> 2
ctagagcaca atcacaggag ccagttttcc tgtgattgtg ctctagcgat gt 52

<210> 3
<211> 44
<212> DNA
<213> Artificial Sequence

<220>
<223> initiator for HCR process

<220>
<221> amino-modified
<222> (1)..(1)

<400> 3
tttttttttt tttttttttt catctcagtc taggattcgg cgtg 44

<210> 4
<211> 50
<212> DNA
<213> Artificial Sequence

<220>
<223> oligonucleotide for preparing DNA hairpins for the linear HCR process

<400> 4

agtctaggat tcggcgtgac gactttcacg ccgaatccta gactgagatg 50

<210> 5
<211> 76
<212> DNA
<213> Artificial Sequence

<220>
<223> oligonucleotide for preparing DNA hairpins for the linear HCR process

<400> 5
acatcgctag agcacaatca caggttagtc gtcacgccga atcctagact ttcatctcag 60

tctaggattc ggcgtg 76

<210> 6
<211> 24
<212> DNA
<213> Artificial Sequence

<220>
<223> initiator for HCR process

<400> 6
acatcgctag agcacaatca cagg 24

<210> 7
<211> 52
<212> DNA
<213> Artificial Sequence

<220>
<223> primer for SiNP modification

<220>
<221> amino-modified
<222> (1)..(1)

<400> 7
tttttttttt tttctaactg ctgcgccgcc gggaaaatac tgtacggtta ga 52

<210> 8
<211> 80
<212> DNA
<213> Artificial Sequence

<220>
<223> primer for CNT modification

<400> 8
tttttttttt tttttttttt tttttttttt tttttttttt tctaactgct gcgccgccgg 60

gaaaatactg tacggttaga 80

<210> 9
<211> 41
<212> DNA

<213>  Artificial Sequence

<220>
<223>  aptamer which specifically binds to the PTK7 receptor

<400>  9
atctaactgc tgcgccgccg ggaaaatact gtacggttag a                           41

<210>  10
<211>  98
<212>  DNA
<213>  Artificial Sequence

<220>
<223>  template for RCA

<220>
<221>  phosphorylated
<222>  (1)..(1)

<400>  10
ttcccggcgg cgcagcagtt agatgctgct gcagcgatac gcgtatcgct atgggtaacc       60

gtacggttac ccgcagcagc atctaaccgt acagtatt                               98

<210>  11
<211>  98
<212>  DNA
<213>  Artificial Sequence

<220>
<223>  template for simultaneous RCA and HCR

<220>
<221>  phosphorylated
<222>  (1)..(1)

<400>  11
ttcccggcgg cgcagcagtt agatgctgct gcagcgaaca tcgctagagc acaatcacag       60

gtacgatatg ccgcagcagc atctaaccgt acagtatt                               98

<210>  12
<211>  52
<212>  DNA
<213>  Artificial Sequence

<220>
<223>  primer for PCR

<400>  12
cgtacgatat gccatagcga tacgcggcgg tttgcgtatt gggcgctctt cc               52

<210>  13
<211>  20
<212>  DNA
<213>  Artificial Sequence

<220>
<223> primer for PCR

<400> 13
ctctccccgc gcgttggccg 20

<210> 14
<211> 6210
<212> DNA
<213> Artificial Sequence

<220>
<223> plasmid for PCR

<400> 14
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatacggt aaatggcccg cctggcatta 540

tgcccagtac atgaccttat gggactttcc tacttggcag tacatctacg tattagtcat 600

cgctattacc atggtgatgc ggttttggca gtacatcaat gggcgtggat agcggtttga 660

ctcacgggga tttccaagtc tccaccccat tgacgtcaat gggagtttgt tttggcacca 720

aaatcacggg actttccaaa atgtcgtaac aactccgccc cattgacgca aatgggcggt 780

aggcgtgtac ggtgggaggt ctatataagc agagctctct ggctaactag agaacccact 840

gcttactggc ttatcgaaat taatacgact cactataggg agacccaagc tggctagtta 900

agctatcaac aagtttgtac aaaaaagcagg ctctaaggag gatagaacca tggtgtctaa 960

gggcgaagag ctgattaagg agaacatgca catgaagctg tacatggagg gcaccgtgaa 1020

caaccaccac ttcaagtgca catccgaggg cgaaggcaag ccctacgagg gcacccagac 1080

catgagaatc aaggtggtcg agggcggccc tctccccttc gccttcgaca tcctggctac 1140

cagcttcatg tacggcagca aaaccttcat caaccacacc caggcatccc cgacttcttt 1200

aagcagtcct tccctgaggg cttcacatgg gagagagtca ccacatacga agacgggggc 1260

gtgctgaccg ctacccagga caccagcctc caggacggct gcctctctac aacgtcaaga 1320

tcagaggggt gaacttccca tccaacggcc ctgtgatgca gaagaaaaca ctcggctggg 1380

aggcctccac cgagatgctg taccccgctg acggcggcct ggaaggcaga agcgacatgg 1440
ccctgaagct cgtgggcggg ggccacctga tctgcaactt gaagaccaca tacagatcca 1500
agaaacccgc taagaacctc aagatgcccg gcgtctacta tgtggacaga agactggaaa 1560
gaatcaagga ggccgacaaa gagacctacg tcgagcagca cgaggtggct gtggccagat 1620
actgcgacct ccctagcaaa ctggggcaca aacttaatta cccagctttc ttgtacaaag 1680
tggttgatct agagggcccg cggttcgaag gtaagcctat ccctaaccct ctcctcggtc 1740
tcgattctac gcgtaccggt catcatcacc atcaccattg agtttaaacc cgctgatcag 1800
cctcgactgt gccttctagt tgccagccat ctgttgtttg cccctccccc gtgccttcct 1860
tgaccctgga aggtgccact cccactgtcc ttcctaataa aatgaggaaa ttgcatcgca 1920
ttgtctgagt aggtgtcatt ctattctggg gggtggggtg gggcaggaca gcaaggggga 1980
ggattgggaa gacaatagca ggcatgctgg ggatgcggtg ggctctatgg cttctgaggc 2040
ggaaagaacc agctggggct ctagggggta tccccacgcg ccctgtagcg gcgcattaag 2100
cgcggcgggt gtggtggtta cgcgcagcgt gaccgctaca cttgccagcg ccctagcgcc 2160
cgctcctttc gctttcttcc cttcctttct cgccacgttc gccggctttc cccgtcaagc 2220
tctaaatcgg gggctccctt tagggttccg atttagtgct ttacggcacc tcgaccccaa 2280
aaaacttgat tagggtgatg gttcacgtag tgggccatcg ccctgataga cggtttttcg 2340
ccctttgacg ttggagtcca cgttctttaa tagtggactc ttgttccaaa ctggaacaac 2400
actcaaccct atctcggtct attcttttga tttataaggg attttgccga tttcggccta 2460
ttggttaaaa aatgagctga tttaacaaaa atttaacgcg aattaattct gtggaatgtg 2520
tgtcagttag ggtgtggaaa gtccccaggc tccccagcag gcagaagtat gcaaagcatg 2580
catctcaatt agtcagcaac caggtgtgga aagtccccag gctccccagc aggcagaagt 2640
atgcaaagca tgcatctcaa ttagtcagca accatagtcc cgcccctaac tccgcccatc 2700
ccgcccctaa ctccgcccag ttccgcccat tctccgcccc atggctgact aatttttttt 2760
atttatgcag aggccgaggc cgcctctgcc tctgagctat tccagaagta gtgaggaggc 2820
ttttttggag gcctaggctt ttgcaaaaag ctcccgggag cttgtatatc cattttcgga 2880
tctgatcaag agacaggatg aggatcgttt cgcatgattg aacaagatgg attgcacgca 2940
ggttctccgg ccgcttgggt ggagaggcta ttcggctatg actgggcaca acagacaatc 3000
ggctgctctg atgccgccgt gttccggctg tcagcgcagg ggcgcccggt tctttttgtc 3060
aagaccgacc tgtccggtgc cctgaatgaa ctgcaggacg aggcagcgcg gctatcgtgg 3120
ctggccacga cgggcgttcc ttgcgcagct gtgctcgacg ttgtcactga agcgggaagg 3180
gactggctgc tattgggcga agtgccgggg caggatctcc tgtcatctca ccttgctcct 3240
gccgagaaag tatccatcat ggctgatgca atgcggcggc tgcatacgct tgatccggct 3300

acctgcccat tcgaccacca agcgaaacat cgcatcgagc gagcacgtac tcggatggaa 3360

gccggtcttg tcgatcagga tgatctggac gaagagcatc aggggctcgc gccagccgaa 3420

ctgttcgcca ggctcaaggc gcgcatgccc gacggcgagg atctcgtcgt gacccatggc 3480

gatgcctgct tgccgaatat catggtggaa aatggccgct tttctggatt catcgactgt 3540

ggccggctgg gtgtggcgga ccgctatcag gacatagcgt tggctacccg tgatattgct 3600

gaagagcttg gcggcgaatg ggctgaccgc ttcctcgtgc tttacggtat cgccgctccc 3660

gattcgcagc gcatcgcctt ctatcgcctt cttgacgagt tcttctgagc gggctctggg 3720

gttcgcgaaa tgaccgacca agcgacgccc aacctgccat cacgagattt cgattccacc 3780

gccgccttct atgaaaggtt gggcttcgga atcgttttcc gggacgccgg ctggatgatc 3840

ctccagcgcg gggatctcat gctggagttc ttcgcccacc ccaacttgtt tattgcagct 3900

tataatggtt acaaataaag caatagcatc acaaatttca caaataaagc atttttttca 3960

ctgcattcta gttgtggttt gtccaaactc atcaatgtat cttatcatgt ctgtataccg 4020

tcgacctcta gctagagctt ggcgtaatca tggtcatagc tgtttcctgt gtgaaattgt 4080

tatccgctca caattccaca caacatacga gccggaagca taaagtgtaa agcctggggt 4140

gcctaatgag tgagctaact cacattaatt gcgttgcgct cactgcccgc tttccagtcg 4200

ggaaacctgt cgtgccagct gcattaatga atcggccaac gcgcggggag aggcggtttg 4260

cgtattgggc gctcttccgc ttcctcgctc actgactcgc tgcgctcggt cgttcggctg 4320

cggcgagcgg tatcagctca ctcaaaggcg gtaatacggt tatccacaga atcaggggat 4380

aacgcaggaa agaacatgtg agcaaaaggc cagcaaaagg ccaggaaccg taaaaaggcc 4440

gcgttgctgg cgtttttcca taggctccgc ccccctgacg agcatcacaa aaatcgacgc 4500

tcaagtcaga ggtggcgaaa cccgacagga ctataaagat accaggcgtt tccccctgga 4560

agctccctcg tgcgctctcc tgttccgacc ctgccgctta ccggatacct gtccgccttt 4620

ctcccttcgg gaagcgtggc gctttctcat agctcacgct gtaggtatct cagttcggtg 4680

taggtcgttc gctccaagct gggctgtgtg cacgaacccc ccgttcagcc cgaccgctgc 4740

gccttatccg gtaactatcg tcttgagtcc aacccggtaa gacacgactt atcgccactg 4800

gcagcagcca ctggtaacag gattagcaga gcgaggtatg taggcggtgc tacagagttc 4860

ttgaagtggt ggcctaacta cggctacact agaagaacag tatttggtat ctgcgctctg 4920

ctgaagccag ttaccttcgg aaaaagagtt ggtagctctt gatccggcaa acaaaccacc 4980

gctggtagcg gtggtttttt tgtttgcaag cagcagatta cgcgcagaaa aaaaggatct 5040

caagaagatc ctttgatctt ttctacgggg tctgacgctc agtggaacga aaactcacgt 5100

taagggattt tggtcatgag attatcaaaa aggatcttca cctagatcct tttaaattaa 5160

```
aaatgaagtt ttaaatcaat ctaaagtata tatgagtaaa cttggtctga cagttaccaa     5220
tgcttaatca gtgaggcacc tatctcagcg atctgtctat ttcgttcatc catagttgcc     5280
tgactccccg tcgtgtagat aactacgata cgggagggct taccatctgg ccccagtgct     5340
gcaatgatac cgcgagaccc acgctcaccg gctccagatt tatcagcaat aaaccagcca     5400
gccggaaggg ccgagcgcag aagtggtcct gcaactttat ccgcctccat ccagtctatt     5460
aattgttgcc gggaagctag agtaagtagt tcgccagtta atagtttgcg caacgttgtt     5520
gccattgcta caggcatcgt ggtgtcacgc tcgtcgtttg gtatggcttc attcagctcc     5580
ggttcccaac gatcaaggcg agttacatga tcccccatgt tgtgcaaaaa agcggttagc     5640
tccttcggtc ctccgatcgt tgtcagaagt aagttggccg cagtgttatc actcatggtt     5700
atggcagcac tgcataattc tcttactgtc atgccatccg taagatgctt ttctgtgact     5760
ggtgagtact caaccaagtc attctgagaa tagtgtatgc ggcgaccgag ttgctcttgc     5820
ccggcgtcaa tacgggataa taccgcgcca catagcagaa ctttaaaagt gctcatcatt     5880
ggaaaacgtt cttcggggcg aaaactctca aggatcttac cgctgttgag atccagttcg     5940
atgtaaccca ctcgtgcacc caactgatct tcagcatctt ttactttcac cagcgtttct     6000
gggtgagcaa aaacaggaag gcaaaatgcc gcaaaaaagg gaataagggc gacacggaaa     6060
tgttgaatac tcatactctt cctttttcaa tattattgaa gcatttatca gggttattgt     6120
ctcatgagcg gatacatatt tgaatgtatt tagaaaaata aacaaatagg ggttccgcgc     6180
acatttcccc gaaaagtgcc acctgacgtc                                      6210
```